# EUROPEAN PATENT APPLICATION

(11) **EP 4 241 791 A1**
(43) Date of publication of application: **13.09.2023**
(21) Application number: 22382209.9
(22) Date of filing: 07.03.2022
(51) Int. Cl.: A61K 48/00

(54) **COMBINED GENE AND RADIO THERAPY FOR THE TREATMENT OF CANCER**

(71) Applicant: InnaTher Gene Therapy S.à.r.l., 2411 Luxembourg (LU)
(72) Inventor: PRIETO VALTUEÑA, Jesus M., Pamplona (Navarra) (ES); MARCO MARTÍNEZ, Sonia, Pamplona (Navarra) (ES); HONORATO CÍA, Beatriz, Pamplona (Navarra) (ES); TENESACA CAYAMBE, Shirley Mireya, Pamplona (Navarra) (ES); GOULD, David James, London, W13 9TQ (GB)
(74) Representative: FARAGO Patentanwälte GmbH

(57) **Abstract**

The invention relates to an expression system comprising a first and/or a second nucleic acid construct, particularly for use in a combined gene and radiation therapy for the treatment of diseases, especially cancer. The first nucleic acid construct encodes for a target STING, that is responsive to a STING agonist recognizing said target STING but not the human STING and natural variants thereof, wherein said sequence encoding the target STING is operatively linked to elements allowing expression and/or translation of said target STING. The second nucleic acid construct comprise a therapeutic gene sequence which is operatively linked to a STING responsive promoter. Said expression system is administered to a target tissue or organ that received prior low dose radiation and is followed by another round of radiation therapy and/or treatment with a STING agonist.

## Description

### Field of the invention

The invention relates to an expression system comprising a first and/or a second nucleic acid construct, in particular for use in a combined gene and radiation therapy for the treatment of diseases, especially cancer. The first nucleic acid construct encodes for a target STING, that is responsive to a STING agonist recognizing said target STING but not the human STING and natural variants thereof, wherein said sequence encoding the target STING is operatively linked to elements allowing expression and/or translation of said target STING. The second nucleic acid construct comprise a therapeutic gene sequence which is operatively linked to a STING responsive promoter. Said expression system is administered to a target tissue or organ that received prior low dose radiation and is followed by another round of radiation therapy and/or treatment with a STING agonist.

### Background of the invention

Cancer is a group of diseases involving abnormal cell growth and proliferation with around 19 million new cases annually and it is the cause of around 10 million deaths annually (Global Cancer Observatory, 2020). While significant progress in the treatment of cancers has been made in the last decades, the annual mortality rate shows that still a huge number of people die because of cancer.

One severe obstacle for the treatment of cancer is that there are several different types of cancers each with individual challenges for its treatment. For example, some cancers are easily accessible and can therefore be treated locally (i.e. surgical removal, local radiotherapy) while others can only be treated by systemic approaches (i.e. chemotherapy, hormone therapy, immunotherapy).

A plethora of different treatments is available such as radiotherapy, chemotherapy, surgical removal, hormone therapy and immunotherapy. Some of these treatments target tumors by systemically interfering with cell proliferation, hence stopping tumor growth. However, these treatments cause severe side effects for the patient like fatigue, nausea and vomiting, anemia and infection, as also physiologically dividing cells like hematopoietic cells, cells in the mouth, digestive tract, and reproductive system are unwantedly targeted by these systemic treatments. Also, local therapies such as local radiotherapy cause severe side effects depending on the localization of the tumor. In contrast, the concept of immunotherapy is to stimulate the body's immune system to act against cancer cells, thereby aiming to reduce unwanted side effects. Immunotherapies can be categorized as active or passive. Active immunotherapy is characterized by immunization of patients with agents that increase the immune response towards a tumor antigen. Examples include therapeutic cancer vaccines, cell-based immunotherapies, and targeted antibody therapies. In contrast, passive immunotherapy does not directly target tumor cells, but enhances the ability of the immune system to attack cancer cells. Examples include checkpoint inhibitors and cytokines. However, immunotherapies so far show several disadvantages such as i) poor tumor targeting, ii) weak tumor cell killing ability, iii) high effort and thereby high cost, iv) tumor immune escape, v) over-stimulation of the immune system which can cause severe side effects (Tan et al. 2020, Biomed Pharmacother. 124: 109821).

STING (also known as Endoplasmic reticulum interferon stimulator (ERIS), Mediator of IRF3 activation (MITA) or Transmembrane protein 173 (TMEM173) is a four-transmembrane endoplasmic protein present in the endoplasmic reticulum (ER) which acts as a sensor of the presence of double-stranded DNA (dsDNA) in the cytosol. As such, STING is activated by DNA viruses, by DNA-damaging insults like radiotherapy and by micronuclei frequently present in the cytosol in malignant cells (Li et al. Journal of Hematology & Oncology 2019, 12:35). STING activates TANK-binding kinase 1 (TBK1) leading to phosphorylation and activation of interferon regulatory transcription factor 3 (IRF3) and upregulation of type I interferon (IFN) expression. STING also recruits IκB kinase (IKK) and activates the NF-κB pathway which promotes the production of pro-inflammatory molecules (including IL-6 and TNFα) and cooperates with TBK1-IRF3 in the production of type I IFN (Li et al. Journal of Hematology & Oncology 2019, 12:35). Type I IFN stimulates immune-effector cells including dendritic cells (DC) and natural killer (NK) cells and promotes the production of chemokines like CXCL9 and CXCL10 which attract T lymphocytes to the tumor which are critical for anti-tumor immunity (Mowat, C. et al J. Exp. Med. 2021 Vol. 218 No. 9 e20210108). In fact, STING agonists, like cyclic dinucleotides (CDN), exert potent antitumor effects in different tumor models (Sivick KE et al Cell Reports 2018; 25, 3074-3085). The importance of STING in cancer pathology can also be derived from the fact that STING expression is usually suppressed or lost in many cancers.

Unfortunately, significant STING polymorphism exists in the human population, which affects both the molecular responses induced by the protein's activation and the degree of sensitivity to stimulatory ligands. Consequently, this can greatly impact the efficacy and safety of molecular entities pursued for clinical purposes.

Based on these numerous findings, STING is now regarded as a master regulator in the cancer-immunity cycle.

A well described activator of STING is 5,6-Dimethylxanthenone-4-acetic acid (DMXAA) disclosed in WO 2004/039363 A1 but also other STING agonists are known in the art. Clinical trials with DMXAA revealed that it is well tolerated and only displayed minor side effects. However, while antitumor effects of STING were described in the animal model, the clinical trials revealed that DMXAA failed in the trials due to lack of efficacy. Further studies have demonstrated the reason for this therapeutic inefficacy, showing that DMXAA potently activates murine STING but is unable to activate human STING due to amino acid differences of the cyclic-dinucleotide (CDN)-binding site of STING.

Documents US 5,126,132 A and US 2005/214274 A1 disclose cell-based immunotherapies where immune-competent cells from the patient like T-cells are isolated, cultivated and modified *ex vivo* so that a specific type of cancer is recognized by these cells, and then reinfused back into the patient. However, next to the elaborate process to generate these *ex* vivo-modified cells, they only target a specific type of cancer.

Immunotherapies using the cytokine interleukin-2 (IL-2) to stimulate the immune system in targeting cancer cells have been approved as early as 1992 for the use to treat melanoma and renal cancer. However, studies show that only around 7% to 16% of patients respond to this treatment at all and severe side effects are common (Boni et al. Expert Opin Drug Saf. 2017 Dec;16(12):1347-1357).

The use of autologous cancer cells as vaccines to augment anti-tumor immunity has been explored for some time. However, due to the weak immunogenicity of many cancers, downregulation of MHC molecules, the lack of adequate costimulatory molecule expression and secretion of immuno-inhibitory cytokines by cancer cells, the response to such vaccines has not resulted in long term efficacy (see, e.g., Armstrong TD and Jaffee EM, Surg Oncol Clin N Am. 1 1(3):681-96, 2002). Numerous cytokines have been shown to play a role in regulation of the immune response to tumors. For example, U.S. Patent No. 5,098,702 describes using combinations of TNF, IL-2 and IFN-β in synergistically effective amounts to combat existing tumors. U.S. Patent Nos. 5,078,996, 5,637,483 and 5,904,920 describe the use of GM-CSF for treatment of tumors. However, direct administration of cytokines for cancer therapy may not be practical, as they are often systemically toxic (Asher et al., J. Immunol. 146: 3227-3234, 1991).

Document US 2021/038684 A1 provides compositions and methods for the treatment of cancer using IL-2 immunotherapy. The methods of the invention comprise administering to a patient an interleukin-2/interleukin-2-receptor α fusion protein systemically, wherein administration results in a dose dependent increase in circulating NK cells and CD8+ cells in a patient in the absence of a dose dependent increase in circulating immunosuppressive T regulatory (Treg) cells. However, while aiming to overcome tumor immune escape this invention comprises the disadvantages known for cancer immunotherapy like weak tumor cell killing ability with only some patients responding to the treatment at all (Boni et al., Journal of Clinical Oncology 2021, 39:15_suppl, 2513-2513). Moreover, cytokine-based immunotherapies normally need to be performed repeatedly to ensure sufficient immune system stimulation by avoiding over-stimulation thereby causing additional medical costs and burden for the patient.

Documents WO 2020/172553 and WO 2021/127556 A1 disclose anti-cancer therapies combining different types of therapies such as combining a cell-based immunotherapy or a checkpoint inhibitor immunotherapy with radiotherapy. However, these therapies to be combined are not each targeted to the cancer and thus do not achieve synergistic effects through combination of the therapies.

Hence, there is still a need for the efficient and safe treatment of proliferative diseases like cancer, where many different types of cancer are targeted efficiently, these treatments show no or minor unwanted side effects and a wide variety of patients respond to the treatment.

The objective of the present invention thus is to provide an improvement or an alternative to the prior art.

This problem is solved by provision of a recombinant expression system for use in a combined gene and radiation therapy according to claim 1 and a recombinant expression system comprising a nucleic acid construct according to claim 15. Specific embodiments are subject matter of further dependent claims.

### Summary of the invention:

In a first aspect the present invention provides a recombinant expression system for use in a combined gene and radiation therapy of a disease in a recipient mammal, wherein
a. the recombinant expression system comprises a first nucleic acid construct encoding for a target STING, that is responsive to a STING agonist recognizing said target STING but not the human STING and natural variants thereof, said sequence encoding the target STING is operatively linked to elements allowing expression and/or translation of said target STING; and/or a second nucleic acid construct comprising a therapeutic gene sequence which is operatively linked to a STING responsive promoter; and
b. the recombinant expression system is administered to the recipient mammal after irradiation of said recipient mammal with low dose radiation of the tissue or organ to be transfected or transduced with said recombinant expression system (target tissue or organ); and/or
c. after administration of said recombinant expression system in step b) the target tissue or organ of the recipient mammal is subjected to low dose radiation and/or a STING agonist is administered to the recipient mammal.

In a similar aspect, the invention relates to a method for treating a disease in a recipient mammal using a vector comprising a recombinant expression system, the method comprising:
a. providing an recombinant expression system, wherein the recombinant expression system comprises a first nucleic acid construct encoding for a target STING, that is responsive to a STING agonist recognizing said target STING but not the human STING and natural variants thereof, said sequence encoding the target STING is operatively linked to elements allowing expression and/or translation of said target STING; and/or a second nucleic acid construct comprising a therapeutic gene sequence which is operatively linked to a STING responsive promoter; and
b. administering the recombinant expression system to the recipient mammal after irradiation of said recipient mammal with low dose radiation of the tissue or organ to be transfected or transduced with said recombinant expression system (target tissue or organ); and/or
c. after administration of said recombinant expression system in step b), subjecting the target tissue or organ of the recipient mammal to low dose radiation and/or administering a STING agonist to the recipient mammal.

Notably, the embodiments described in the following apply to both, the recombinant expression system of the invention for use in a combined gene and radiation therapy and the method of treating a disease in a recipient mammal using said recombinant expression system.

It is conceivable that the use for a combined gene and radiation therapy or the method of treating a disease in a recipient mammal according to the invention may be combined with other treatments that are anti-cancer therapies. Several anti-cancer therapies are known in the art such as chemotherapy, hormone therapy, radiotherapy, photodynamic therapy, stem cell transplants, surgery, targeted therapy, hyperthermia, or other immunotherapies.

The use of a combined gene and radiation therapy or the method of treating a disease in a recipient mammal according to the invention offers several advantages over prior art cancer therapies.

In the second nucleic acid construct, the therapy-associated expression of the therapeutic gene sequence is ensured by placing the transgene under the control of a STING-responsive promoter. Said promoter is characterized by that it promotes the transcription of the therapeutic gene sequence only when STING is active in the cell. STING may be activated by several therapeutic relevant means allowing the temporal and spatial control of STING activity in a given tissue or organ. Several STING agonists are known in the art which allow specific STING activation. Moreover, STING is known to be activated by encountering DNA-damaging insults like radiotherapy and by micronuclei frequently present in the cytosol in malignant cells. Thus, the expression of the therapeutic gene sequence being controlled by the STING responsive promoter is linked to different cancer-associated stimuli which can be further and selectively stimulated by a STING agonist pharmacotherapy.

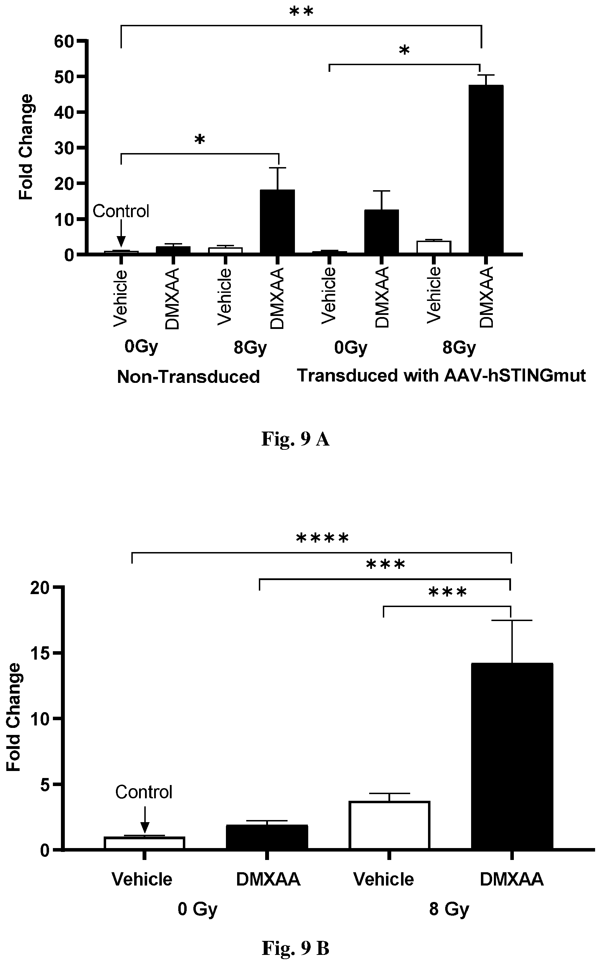

The inventors found that the first nucleic acid of the expression system according to the invention induces the upregulation of STING-associated genes or proteins upon specifically activating the target STING by administering a STING agonist that recognizes said target STING but not the human STING and natural variants thereof. It was further found that the upregulation of STING-associated genes or proteins are also induced by low dose radiotherapy. It could be shown that the enrichment in STING (e.g. by transducing the cells with a target STING) makes the cells more responsive to either STING agonists like DMXAA (canonical pathway) or to low dose radiotherapy (non-canonical pathway) separately and enables the full display of the synergism between the two stimuli (STING agonist and low dose radiotherapy) when they are applied together (see Figure 9 A-C). Accordingly, in one aspect of the invention it is sufficient that the expression system includes only the cassette encoding a target STING that is responsive to a STING agonist that does not activate human STING.

The expression system can also include the first and the second nucleic acid construct representing two expression cassettes a) the first nucleic acid construct encoding a target STING that is responsive to a STING agonist that does not activate human STING and natural variants thereof (in the following also designated as "human-non-reacting STING agonist"), and b) the second nucleic acid construct that encodes a therapeutic gene, being preferably an immunomodulatory gene or protein, under the control of a promoter that is activated upon STING activation.

According to the invention the expression system comprises both cassettes or only one of them. The combined expression of both nucleic acid constructs and hence both cassettes has the advantage that after intratumor injection of the expression systems (being preferably a vector and more preferably a viral vector) containing said expression cassettes, the systemic administration of the agonist of said target STING enables the selective activation of STING within the tumor without affecting the endogenous human STING in the rest of the body, thus avoiding toxicity. The intratumor activation of the target STING induces the production of IFN type I, chemokines and other cytokines within the tumor by induction of endogenous genes or by activation of the promoter of the second expression cassette that encodes an immunomodulatory gene or protein, like IL-12. In this manner the systemic administration of the agonist of said target STING triggers intratumor production of IFN type I, and other immunostimulatory peptides resulting from STING activation, together with the production of the immunomodulatory gene or protein expressed by the second expression cassette. The local intratumor production of this combination of immunomodulatory genes or proteins would promote antitumor immunity without systemic toxicity.

The use of the system composed of the two expression cassettes injected into a freshly irradiated tumor enables local activation of the transgenic target STING by the ionizing radiation (non-canonical pathway) and this activation could be furtherly increased by launching the canonical pathway of STING activation using a human-non reacting STING agonist (like DMXAA). By combining irradiation and DMXAA the inventors found that a synergistic effect is obtained leading to an intense STING response locally causing strong activation of the STING-responsive promoter that directs the expression of the immunomodulatory peptide encoded by the second expression cassette with resulting high intratumor levels of a combination of immunomodulatory molecules.

By injecting the tumor with long-term expression vector(s) (like adeno-associated virus vectors) containing the two expression cassettes, in the same or separate vectors, it is possible to induce the production of the mentioned combination of immunomodulatory genes or proteins within the tumor by repeating the systemic administration of the agonist of the target STING.

As already described above, cancer immunotherapies have several limitations such as i) poor tumor targeting, ii) weak tumor cell killing ability, iii) high effort and thereby high cost, iv) tumor immune escape, v) over-stimulation of the immune system which can cause severe side effects (Tan et al. 2020, Biomed Pharmacother. 124: 109821). The immunotherapy according to the invention is a more efficient and specific immunotherapy as it also incorporates gene therapy. Hence, the immunotherapy according to the invention is not merely an immunotherapy by itself but a combination of immunotherapy and gene therapy, hereinafter termed "immunogene therapy" to effectively treat proliferative disorders like cancer.

The inventors surprisingly found that the inventive combination of an immunogene therapy comprising the viral vector in combination with a radiotherapy does not simply add the effects received by each individual therapeutic approach but rather acts in a synergistic way in that both treatment effects are potentiated when the therapies are combined. Hence, the combination therapy according to the invention significantly reduced tumor progression in an experimental model. In addition, the therapy led to a reduction in tumor size and even to complete remission. The therapy also showed an abscopal effect in that also the tumor size of tumors that were not directly treated were reduced by the therapy as well.

The synergistic effect of the invention manifests in three ways: 1) the radiotherapy prior to treatment with the viral vector enhances the efficiency of gene delivery to the target cell, thus increasing the number of cells producing the therapeutic gene and 2) the radiotherapy/radiotherapies given immediately before vector administration or given after the administration of the viral vector induces the expression of the therapeutic gene sequence by activation of the STING responsive promoter, and 3) the therapeutic gene expressed by the viral vector enhances the immune-stimulatory effects set into motion by radiotherapy. Hence, the effects of the therapies are multiplied rather than merely added as is also shown in the examples of this application.

As the mechanism underlying this synergy it is assumed that the radiotherapy given immediately before vector administration leads to the activation of the DNA damage response (which lasts for several days, (Zhao Z et al., Genomics 2020; 112: 1309-1317]) and to the presence of cytosolic DNA in the irradiated cells. All these changes maintain STING activation for a period of time. For this reason, the activation of the STING-responsive promoter takes also place when radiotherapy is given immediately before vector administration

The system of this invention allows the expression of the therapeutic gene sequence on demand and in a highly localized manner. Thereby disadvantages of systemic immunotherapies known in the art like toxicity are circumvented.

Furthermore, in case that the recombinant expression comprises the first nucleic acid construct encoding for a target STING, it was found that the expression of said construct induces the upregulation of STING-associated genes or proteins upon specifically activating the target STING by administering a STING agonist that recognizes said target STING but not the human STING and natural variants thereof.

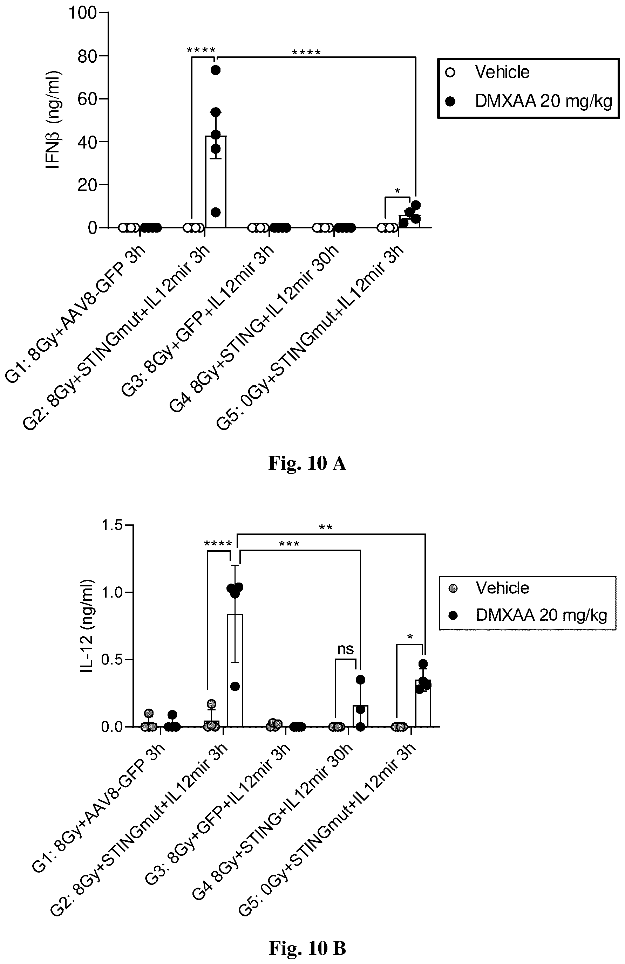

Accordingly, the enrichment in STING (e.g. by transducing the cells with a target STING) makes the cells more responsive to either STING agonists like DMXAA (canonical pathway) or to low dose radiotherapy (non-canonical pathway) separately and enables the full display of the synergism between the two stimuli (STING agonist and low dose radiotherapy) when they are applied together (see Figure 9 A-C and Figure 10 A-D), thereby providing a third synergistic effect according the invention.

Accordingly, in one aspect of the invention it is sufficient that the recombinant expression system only includes only the first nucleic acid construct encoding a target STING that is responsive to a STING agonist which does not activate human STING.

Moreover, the inventors found that the administration of the recombinant expression system, being preferably a viral vector, and expression in combination with the radiotherapy according to the invention is non-toxic and shows minor side effects *in vivo.* Of note, the inventors found that the administration of the recombinant expression system, being preferably a viral vector, and expression in combination with radiotherapy causes the reduction of tumors eventually leading to full remission when administered *in vivo.* Even more, by administering the recombinant expression system of the invention, being preferably a viral vector, locally in the tumor, not only the targeted tumor is reduced in its size, but also non-targeted tumors show significant size reductions, also known as the abscopal effect.

### Detailed description of the invention

The recombinant expression system generates a specific and basal immunological pathway in the transfected cancer cell that makes use of further endogenous pathway components.

Of note the term "recombinant expression system" includes any nucleotides, analogues thereof, and polymers thereof. Hence, any of the recombinant expression system disclosed herein refer to a polymeric form of nucleotides of any length, either ribonucleotides (RNA) or deoxyribonucleotides (DNA). These terms refer to the primary structure of the molecules and, thus, include double- and single-stranded DNA, and double- and single-stranded RNA. A more comprehensive definition is enclosed in the definition section of this application.

The first component of this pathway is the so-called "target STING", which is defined by its responsiveness to a STING agonist that is unable to activate the human STING allowing a selective activation of the target STING. The first component is given by the first nucleic acid construct of the invention and can be regarded as both "co-effector" and "activator" part of the pathway. Notably, the first component can be also given alone, making the cells expressing this first component responsive to STING agonists and low dose radiotherapy, whereby the full display of the synergism between these two stimuli is given (STING agonist and low dose radiotherapy) when they are applied together.

In the nucleic acid construct according to the invention, the target STING is operatively linked to elements allowing expression and/or translation of said target STING. In the event that the recombinant expression system is a DNA-based expression system this element is typically a promoter that allows the transcription of an mRNA encoding the target STING. In the event that the recombinant expression system is an RNA-based expression system this element is typically an RNA element that allows the translation of the mRNA encoding the target STING such as e.g. a CAP, the 5'UTR, the 3'UTR or a poly A site.

The second component of this pathway is an artificial gene construct that includes an immunomodulatory gene or protein, said sequence is operatively linked to a STING-responsive promoter. The immunomodulatory gene or protein activates both innate and adaptive immune responses in order to generate a specific cancer immune response. The second component is given by the second nucleic acid construct of the invention and can be regarded as "co-effector" part of the pathway.

The recombinant expression system according to the invention comprises a recombinant expression system and allows the temporal and spatial expression of a therapeutic gene sequence depending on STING activity and/or the temporal and spatial expression of the target STING depending on the nucleic acid constructs included in the recombinant expression system. In case of the second nucleic acid construct, this is achieved by operatively linking the therapeutic gene sequence to the STING responsive promoter. One of several ways to activate STING is by exposing a cell to ionizing radiation which generates DNA damages that are recognized through the cGAS/STING pathway inducing the expression of STING downstream targets such as type I interferons or inflammatory cytokines.

Ionizing radiation is widely used as a treatment of diseases. The most prominent use being the treatment of cancer which is a proliferative disease characterized by rapid division of cancer cells. The DNA of cells that receive ionizing radiation is damaged thereby inhibiting cell division or in the case of excessive DNA damage killing the cell. Thus, the initial low dose radiation of the tissue or organ as described in b) of the invention works two-fold: i) the low dose radiation damages DNA of the targeted cells thereby working in its original manner, and ii) low dose radiation enhances cell transduction by AAV vectors. This is so, because radiotherapy triggers the DNA damage response (DDR) which, among other effects, promotes the conversion of AAV single stranded DNA into the transcriptionally active double stranded DNA (Peng D. et al J. Hepatol. 2000; 32: 975-85). As a result, there is an increase in the number of the target cells expressing the transgene conveyed by the viral vector.

On the other hand, DNA damaging agents have been shown to increase cytosolic DNA in tumor cells (Li, C et al. Molecular Immunology 2021; 131, 180-190) subsequently leading to STING activation (Deng, L. et al., Immunity 2014; 41, 843-852; Dunphy et al. Molecular Cell, 2018; 71, 745-760). Thus, radiotherapy given previously to intratumor injection of an AAV vector would cause both, an enhancement of tumor transduction by AAV vectors, and also STING activation. The latter effect would promote the production of an immunostimulatory cytokine expressed by the vector under the control of a STING-responsive promoter.

The low dose radiation described in c) of the invention again works two-fold. Firstly, the low dose radiation damages DNA of the targeted cells thereby working in its original manner. Secondly, the DNA damage causes activation of the cGAS/STING pathway thereby inducing the expression of the therapeutic gene sequence delivered by the recombinant expression system, being preferably a viral vector. Thus, the temporal and spatial expression of the therapeutic gene sequence and the target STING may be controlled by localized low dose radiation although also other measures, can be taken to induce STING activity.

Taken together, the low dose radiation and the immunogene therapy work synergistically in that every therapy for itself has an effect, however as the therapies interact with each other as described above the effects are multiplied rather than simply added. This is also shown *in vitro* (see Example 9) and *in vivo* (see Example 10). Thus, the combination of immunogene therapy and radiotherapy according to the invention is an effective treatment for the therapy of a disease in a recipient mammal.

Advantageously, in the context of the invention, it may be provided that the disease is selected from the group consisting of cancer, autoimmune disease, inflammatory disease, or infectious disease.

It may further be possible that the cancer is a solid cancer selected from the group consisting of: carcinoma including that of the bladder (including accelerated and metastatic bladder cancer), breast, colon (including colorectal cancer), kidney, liver, lung (including small and non-small cell lung cancer and lung adenocarcinoma), ovary, prostate, testes, genitourinary tract, lymphatic system, rectum, larynx, pancreas (including exocrine pancreatic carcinoma), esophagus, stomach, gall bladder, cervix, thyroid, and skin (including squamous cell carcinoma); hematopoietic tumors of lymphoid lineage including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, histiocytic lymphoma, and Burkitt's lymphoma; brain cancer, tumors of the central and peripheral nervous system including astrocytoma, neuroblastoma, glioma, and schwannomas; cancer of the thymus, tumors of mesenchymal origin including fibrosarcoma, rhabdomyosarcoma, and osteosarcoma; other tumors including melanoma, xenoderma pigmentosum, keratoactanthoma, seminoma, thyroid follicular cancer, and teratocarcinoma; melanoma, unresectable stage III or IV malignant melanoma, squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatoma, breast cancer, colon carcinoma, colon cancer, esophageal cancer, lung cancer, mesothelioma, multiple myeloma, Merkel cell carcinoma, skin cancer, testicular cancer, uterine cancer, plasma cell tumors, and head and neck cancer, gastric cancer, germ cell tumor, bone cancer, bone tumors, adult malignant fibrous histiocytoma of bone; childhood, malignant fibrous histiocytoma of bone, sarcoma, pediatric sarcoma, sinonasal natural killer, neoplasms, plasma cell; neuroendocrine tumors, cholangiocarcinoma, or carcinoid tumors.

Preferably the solid cancer is selected from the group consisting of bladder cancer, bone cancer, brain cancer, breast cancer, cancer of the thymus, cervical cancer, colon cancer, esophageal cancer, gastric cancer, glioblastoma, head & neck cancers, Hodgkin's lymphoma, liver cancer, lung cancer, melanoma, mesothelioma, multiple myeloma, Merkel cell carcinoma, non-Hodgkin's lymphoma, ovarian cancer, pancreatic cancer, prostate cancer, rectal cancer, renal cancer, sarcoma, skin cancer, testicular cancer, thyroid cancer, uterine cancer, plasma cell tumors, neuroendocrine tumors, cholangiocarcinoma, or carcinoid tumors.

Basically, every disease that can be treated by radiotherapy is a suitable disease to be targeted by the combined therapy according to the invention as the synergistic effect of enhanced gene delivery and the induction of the expression of the therapeutic gene sequence is universally applicable, and furthermore combines with the effects of radiotherapy alone. Hence, also non-cancer diseases like arthrosis, Dupuytren's disease, Ledderhose disease, calcaneal spur, tennis elbow, vertebral body haemangioma, vertebral body haemangioma, chronic synovitis, Pigmented villonodular synovitis (PVNS), chronic joint effusion, rheumatoid arthritis and activated arthrosis, each of which is treatable by radiotherapy, are particularly accessible by the combination therapy according to the invention.

In a further aspect, the invention relates to a recombinant expression system comprising a nucleic acid construct encoding for a therapeutic gene sequence which is operatively linked to a STING responsive promoter. Therewith, the invention relates to a recombinant expression system comprising the second nucleic acid construct as described in the present application.

Notably, according to this aspect, the recombinant expression system comprising the second nucleic acid construct is claimed as such and not only for use in the combination therapy according to the present invention or in the method of treating a disease in a recipient mammal using said expression system according to the invention.

In a preferred embodiment, said expression system is provided as an expression vector, which is more preferably a viral vector; and even more preferably as a viral particle that comprises the viral vector.

Notably, the embodiments described in the following for the expression system comprising the second nucleic acid apply to both, the expression system of the invention as such and also the expression system for use in a combined gene and radiation therapy or the method of treating a disease in a recipient mammal using said the expression system.

It is further conceivable within the scope of the invention that the therapeutic gene sequence of the second nucleic acid construct encodes for a protein or gene selected from the group consisting of cytokines such as chemokines, interferons, interleukins, lymphokines, and tumor necrosis factors; hormones; growth factors; cell surface receptors; enzymes; transcription factors; tumor suppressor proteins; nanobodies; monoclonal antibodies; single chain variable fragments (scFv); peptides blocking the activity of cytokines, nuclear factors or hormones; shRNA against cytokines, nuclear factors or hormones.

A non-limiting list of therapeutic proteins representing immunomodulatory proteins, which can be used in the second nucleic acid construct of the recombinant expression system of the invention, being preferably in form of a viral vector, includes the following proteins: interleukin-1, interleukin-2, interleukin-3, interleukin-4, interleukin-5, interleukin-7, interleukin-8, interleukin-9, interleukin-10, interleukin-11, interleukin-12, interleukin-13, interleukin-14, interleukin-15, interleukin-16, interleukin-17, interleukin-18, interleukin-19, interleukin-20, interleukin-21, interleukin-22, interleukin-23, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, XCL1, XCL2, CX3CL1, granulocyte-macrophage colony-stimulating factor, type I interferons, IFN-γ, TNF-α, FLT3-ligand, a blocking peptide targeting TGF-β, a blocking peptide targeting IL-10, a blocking peptide targeting FoxP3, a monoclonal antibody or single-chain variable fragment (scFv) or nanobody neutralizing PD1, PDL1, CTLA4, CD137, TIM3, LAG3, and a fragment or variant thereof. A non-limiting list of therapeutic genes useful in the invention is: shRNA targeting TGF-β, a shRNA targeting IL-10, or a shRNA targeting FoxP3.

The person skilled in the art recognizes that any gene or protein that retards, halts or reverses disease progression, or cures the disease is a suitable molecule that might be encoded by the therapeutic gene sequence of the second nucleic acid construct of the invention.

It may be provided that the gene or protein encoded by the therapeutic gene sequence of the second nucleic acid construct of the recombinant expression system of the invention is selected from the group consisting of interleukin-1 (IL-1), interleukin-2 (IL-2), interleukin-4 (IL-4), interleukin-5 (IL-5), interleukin-7 (IL-7), interleukin-10, interleukin-12 (IL-12), interleukin-15 (IL-15), interleukin-21 (IL-21), interleukin-23 (IL-23), granulocyte-macrophage colony-stimulating factor (GM-CSF), type I interferons (IFN-α and IFN-β), IFN-γ, TNF-α, FLT3-ligand; immunostimulatory monoclonal antibody or single chain variable fragment (scFV) or nanobody neutralizing PD1, PDL1, CTLA4, CD137, TIM3, LAG3; blocking peptides or shRNA against immunosuppressive factors such as TGF-β or IL-10 or FoxP3.

In one embodiment of the invention, the gene encoded by the therapeutic gene sequence of the second nucleic acid construct of the recombinant expression system of the invention is a shRNA directed against mRNAs of immunosuppressive factors such as cytokines, nuclear factors or hormones. Preferably, the shRNA is directed against the TGF-β-mRNA, IL-10-mRNA or FoxP3 mRNA.

There are several immunomodulatory proteins known in the art that are used in immunotherapies for cancer treatment. For example, IL-2 is a protein which has been used in immunotherapies for more than 20 years in the treatment of cancer. However, only very few patients respond at all to IL-2-related immunotherapies while these therapies are known to cause severe side effects. IL-12 re-programs myeloid-derived suppressor cells (MDSCs) to become efficient antigen-presenting cells (APC). In addition, IL-12 strongly stimulates T cells and NK cells and increases IFN-γ production propitiating a Th1 type of response capable of controlling the growth of the treated tumor but also of distant metastasis (Lasek et al. Cancer Immunol. Immunother. 2014; 63: 419). IL-12 is a heterodimeric cytokine which is encoded by two separate genes. To facilitate its expression in recombinant expression systems it is feasible to use the recombinant single chain IL-12 (scIL-12) transgene that encodes both subunits of IL-12. Thus, in a preferred embodiment the therapeutic gene sequence encodes IL-12.

In one embodiment, the at least one immunomodulatory protein is a complex or fusion protein of IL-15 with the Sushi domain of IL-15 receptor α chain to enhance the agonist activity of IL-15 via trans-representation such as provided by the compound P22339 (Hu, Q., Ye, X., Qu, X. et al., Sci Rep 2018, 7675).

It may optionally be possible that the IL-12 is selected from the group consisting of the α subunit of IL-12, the β subunit of IL-12, single chain IL-12 comprising the α and β subunit of IL-12. In addition, the IL-12 may be a single chain IL-12 comprising or consisting of the amino acid sequence of SEQ ID NO: 9 or 10 (human or murine single chain IL-12), or a polypeptide having at least 70%, 75%, 80%, 85%, 90%, 95%, or 99% sequence identity with respect to SEQ ID NO: 9 or 10, wherein the polypeptide has the biological activity of interlekin-12. The sequence identity of the polypeptide sequence with respect to SEQ ID NO: 9 or 10 can be at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

Immunotherapies that involve systemic availability of an immunomodulatory protein often cause unwanted side effects. However, an advantage of this invention is that the recombinant expression system used herein induces the local expression of a therapeutic gene sequence which may be an immunomodulatory protein thereby inducing a highly local and targeted immune response. For example, an intratumor administration of the recombinant expression system, which is a preferred embodiment of the invention, induces an immune response by producing immune-stimulatory cytokines such as interleukins or chemokines within the tumor mass. Without being wished to be bound by theory, it is thought that the release of the immunomodulatory protein within or in the vicinity of the tumor causes T-cells to recognize the tumor cells that normally escape the recognition of the immune system, thereby enabling the immune system to target and kill these cells. Thus, tumor progression is at least retarded, preferably halted, and more preferably reversed.

In a preferred embodiment the therapeutic gene sequence of the second nucleic acid construct of the recombinant expression system of the invention encodes an immunomodulatory gene or protein, more preferably it encodes an immunostimulatory gene or protein. That is a gene or protein that stimulates the immune system such as by directly stimulating it (e.g. IL-12) or by inhibiting immunosuppressive molecules (e.g. shRNA targeting TGF-β).

According to a further possibility it may be provided for the second nucleic acid construct that the STING-responsive promoter that is operatively linked to the therapeutic gene sequence is selected from the group consisting of IFN-β-responsive promoter (preferably according to SEQ ID NO: 25), IFN-β-minimal promoter (preferably according to SEQ ID NO: 31), CXCL9-responsive promoter (preferably according to SEQ ID NO: 23), CXCL10-responsive promoter (preferably according to SEQ ID NO: 24), STAT-motif promoter (preferably according to SEQ ID NO: 26), IRSF3-motif promoter (preferably according to SEQ ID NO: 27 or to SEQ ID NO: 28), ISRE-motif promoter (preferably according to SEQ ID NO: 11 or to SEQ ID NO: 12 or to SEQ ID NO: 13), NFκB-motif promoter (preferably according to SEQ ID NO: 29 or to SEQ ID NO: 30) and STAT6-motif promoter. In a preferred embodiment, the STING responsive promoter comprises at least one interferon-stimulated response element (ISRE).

By operatively linking the expression of the therapeutic gene sequence to the activity of STING using a STING-responsive promoter according to the invention a targeted and local expression of the therapeutic gene sequence can be achieved thereby avoiding potential side effects by systemic expression of the transgene encoded by the second nucleic acid construct.

It may optionally be possible that the STING-responsive promoter of the second nucleic acid construct of the recombinant expression system of the invention is selected from the list consisting of:
a. a nucleotide sequence comprising one to four copies of the ISRE as depicted in SEQ ID NO: 11, comprising preferably four copies of said ISRE;
b. a nucleotide sequence according to SEQ ID NO: 12 which comprises four copies of the ISRE interspaced with 20 nucleotides;
c. a nucleotide sequence according to SEQ ID NO: 13 which comprises four copies of the ISRE interspaced with 6 nucleotides.

The use of STING-responsive promoters in the context of the invention ensures the controlled and targeted expression of the therapeutic gene sequence. Moreover, the aforementioned STING-responsive promoters have been shown to have additional advantages. As elaborated in the examples of this application the STING-responsive promoters have been shown to be inducible by administering a human-non-reacting STING agonist in the presence of target STING. Moreover, the induction of these promoters has been shown to be specific in that in the absence of the selective STING agonist only limited expression occurs. In particular, promoters carrying four copies of the ISRE as depicted in SEQ ID NO: 12 are highly inducible while also displaying high specificity.

It is known that several viral particles, especially AAV, show a tropism towards liver cells. This means that when viral particles gain access to the blood circulatory system, they preferentially transduce liver cells, eventually causing the expression of the encoded transgene in the liver. Also in the event of intratumor injections of said viral particles, some of these particles will access the blood circulatory system, as the tumor is highly vascularized. To prevent the unwanted expression of the transgene encoded by the viral particle in the liver it is therefore suitable to control its expression. Several methods are known by the person skilled in the art to achieve this goal.

In general, a suitable method to limit transgene expression to a specific tissue or a specific cell type is to use viral particles with a distinct tropism towards said tissues or cell types. A way to achieve this is by altering the serotype of an AAV or by altering the capsid of the AAV. Several AAV serotypes are known in the art each with distinct tropisms. However, as the invention is intended to target several different types of cancers, it would be necessary to optimize the serotype or capsid for each cancer or tumor type. Nonetheless, this may be a suitable way to achieve tissue- or cell type-specific expression of a transgene.

Another possibility is to use RNA interference (RNAi). RNAi is characterized in that an RNA molecule reduces or inhibits expression of a nucleic acid sequence with which the RNA molecule shares substantial or total homology. Non-limiting examples of RNAi are small interfering RNA, small hairpin RNA or microRNA (miR). To reduce or inhibit transgene expression in a specific tissue using RNAi it may be conceivable that for example a shRNA targeting the nucleotide sequence of said transgene may be operatively linked to a tissue specific promoter. When said shRNA is administered it thereby reduces or inhibits transgene expression in the tissue. Although this method would be feasible in the context of the invention it may be disadvantageous in that an additional administration of said shRNA would be necessary to limit transgene expression in the target tissue, e.g. the liver.

An elegant way to avoid this drawback is to operatively link a miRNA target sequence to the nucleotide sequence of the transgene. Thereby, expression of the transgene will be reduced or inhibited in tissues where a miRNA that is associated to said miRNA target sequence is present. Accordingly, a preferred embodiment of the invention comprises that the sequence motif is a target sequence of a microRNA which is highly abundant in the liver. Thereby, a single administration of a nucleic acid construct is sufficient to avoid transgene expression in the liver while transcription in other tissues or organs is not impaired.

According to a further possibility it may be provided that the second nucleic acid construct of the recombinant expression system comprising a therapeutic gene sequence further comprises at least one sequence motif that inhibits transgenic expression of the therapeutic gene sequence in the liver, wherein the sequence motif is preferably a target sequence of a microRNA which is highly abundant in the liver.

It is optionally conceivable that the the microRNA highly abundant in the liver is selected from the group consisting of miR-122, miR-192, miR-199a, miR-101, miR-99a, let7a, let7b, let7c or let7f. Furthermore, it may be advantageous that the microRNA abundant in the liver is miR-122. The advantage of miR-122 is that it is highly expressed in the liver and, importantly, specifically expressed in the liver. Hence, by choosing a miR-122 target sequence a significant reduction of the transgene in the liver is ensured while expression in other tissues is not impaired. Moreover, it has been shown that miR-122 is downregulated in most liver tumors so that the recombinant expression system according to the invention is also suitable to treat liver cancer.

According to a preferred embodiment of the invention, the respective miR-122 target sequence is a miR-122 target sequence as depicted in SEQ ID NO: 14. Preferably, the target sequence comprises 1 to 5 copies of the miR-122 target sequence of SEQ ID NO: 14, and more preferably the target sequence comprises five copies of this miR-122 target sequence. Increasing the copy number of miR-122 target sequences increased the miR-mediated reduction, whereas no significant improvement was observed above the disclosed number of 5 copies.

It is preferred that the nucleotide sequence comprises 5 copies of the miR-122 target sequence as depicted in SEQ ID NO: 15. As elaborated in the examples of this application, the nucleotide sequence as depicted in SEQ ID NO: 15 has been shown to substantially inhibit transgene expression in the liver thus causing the plasma levels of the transgene to be undetectable by the methods described in Example 4.

It may be provided that the low dose radiation is based on X-rays, gamma-rays or particle rays. Each type of ray has its own characteristics and therapeutic application profile. The person skilled in the art chooses the respective ray based on the disease itself, the localization, and the progression of the disease.

It is optionally conceivable that the low dose radiation has a total dose of radiation of 40 Gy or less and preferably of 8 Gy and less. The total dose of radiation can be less than 40 Gy, 38 Gy, 36 Gy, 34 Gy, 32 Gy, 30 Gy, 28 Gy, 26 Gy, 24 Gy, 22Gy, 20 Gy, 19 Gy, 18 Gy, 17 Gy, 16 Gy, 15 Gy, 14 Gy, 13 Gy, 12 Gy, 11 Gy, 10 Gy, 9 Gy, 8 Gy, 7 Gy, 6 Gy, 5 Gy, 4 Gy, 3 Gy, 2.5 Gy, 2.0 Gy, 1.5 Gy, 1 Gy, 0.5 Gy, 0.25 Gy, 0.2 Gy, 0.15 Gy, 0.1 Gy, 0.05 Gy, or 0.01 Gy.

Using low dose radiation has several advantages. Firstly, radiation treatment always comes with side effects. By using low doses of radiation these side effects are significantly reduced. Secondly, there is a limit to the amount of radiation an area of your body can safely receive over the course of your lifetime, also known as the lifetime limiting dose. Using low dose radiation not only is it more likely to stay below this limit but it also allows for a higher number of radiation treatments. In the context of the invention this is particularly useful as it allows the repeated expression of the therapeutic gene sequence under control of the STING-responsive promoter. Thirdly, low dose radiation enhances the susceptibility of cells to gene delivery. AAVs are single-stranded DNA vectors which need to be converted into double-stranded DNA to form transcription templates. Without being wished to be bound by therapy it is thought that the DNA Damage Response elicited by radiotherapy helps to convert single stranded DNA into double stranded DNA. The use of radiotherapy to enhance tumor transduction by AAV vectors is an attractive option as radiation is frequently given to treat solid tumors. Published data relating AAV transduction and radiation refer to *in vitro* studies with different radiation doses and to *in vivo* studies with high radiation doses. However high radiation doses cause vessel damage and mark alteration of tissue architecture. Thus, a relatively low radiation dose preserves tumor vessels thereby sustaining lymphocyte trafficking to the tumor.

According to a further possibility it may be provided that the low dose radiation is administered using stereotactic radiosurgery, preferably performed by linear accelerator (LINAC) machines, gamma Knife machines or proton beam therapy.

An advantage in using the aforementioned administration methods is that they allow high precision targeting of the target tissue or organ. Next to limiting unwanted side effects derived from irradiation also transgene expression is induced in a specific area. In the context of the invention this restricts the expression of the target gene sequence to a well-defined area thereby circumventing systemic availability of the transgene and thus avoiding unwanted side effects. As described in the examples below although a target tumor received radiation therapy combined with immunogene therapy also other non-targeted tumors showed a reduction in size (abscopal effect). Thus, by using high precision radiotherapy not only are side effects reduced but also distant tumors are still affected by the treatment.

Advantageously, in the context of the invention, it may be provided that the time interval between the low dose irradiation of b. and the subsequent administration of the viral vector is between 1 minute and 10 days, preferably between 10 minutes and 2 days and more preferably between 30 minutes and 24 hours. The time interval may be anything between 1 minute and 10 days. The subsequent administration of the viral vector starts at the earliest 1 minute, 2 minutes, 5 minutes, 10 minutes, 15 minutes, 20 minutes, 25 minutes, 30 minutes, 45 minutes, 1 hour, 2 hours, 3 hours, 4 hours, 5 hours, 6 hours, 7 hours, 8 hours, 10 hours, 12 hours, 14, hours, 16 hours, 18 hours, 20 hours, 22 hours, 1 day, 2 days, 3 days, 4 days, 5 days, 6 days, 7 days, 8 days, 9 days after receiving low dose radiation. This ensures the enhanced gene delivery and increased expression of the transgene in the target tissue or organ.

Advantageously, in the context of the invention, it may be provided that the time interval between the administration of the viral vector and the subsequent low dose irradiation of c. is between 1 day and 30 days preferably between 5 days and 20 days and more preferably between 7 days and 15 days. The time interval may be anything between 1 day and 30 days. The subsequent low dose radiation starts at the earliest 1 day, 2 days, 3 days, 4 days, 5 days, 6 days, 7 days, 8 days, 9 days, 10 days, 11, days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days after administration of the viral vector. Subsequently, low-dose radiations may be repeated at intervals between 1 day and 30 days. The subsequent low dose radiations start at the earliest 1 day and 30 days. The subsequent low dose radiation starts at the earliest 1 day, 2 days, 3 days, 4 days, 5 days, 6 days, 7 days, 8 days, 9 days, 10 days, 11, days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days after the previous dose radiation.

According to the invention the recombinant expression system further comprises a first nucleic acid construct comprising a sequence encoding for a target STING, which is responsive to a STING agonist which recognizes said target STING but not the human STING and natural variants thereof, said sequence encoding the target STING is operatively linked to a promoter.

Notably, the target STING differs from the native human STING due to its activation by human-non-reacting agonists such as DMXAA. However, upon activation the target STING displays biological activity of human STING in that the target STING is still able to bind to and activate downstream effector proteins that are also activated by human STING such as iRhom2 and TANK-binding kinase 1 (TBK1). Upon activation, STING suffers a conformational change leading to TBK1 recruitment. TBK1, in turn, phosphorylates the transcription factors interferon regulatory factor 3 (IRF3) and nuclear factor-KB (NF-κB), which translocate to the nucleus and stimulate the production of pro-inflammatory molecules such as type I interferon, IL6, TNF-α or CXCL10. (Ahn, J.et al Exp Mol Med 51, 1-10, 2019)

Using an exogenic target STING has several advances in the context of the invention. Firstly, by using target STING it is possible to induce STING activity selectively of the exogenic target STING but not the endogenous STING. Meaning that STING activity is induced only in targeted cells that have been transfected or transduced with the viral vector and thereby STING activity is limited locally. Thus, systemic STING activation is avoided and with it unwanted side effects resulting from systemic STING activation. Hence, expression of the therapeutic gene sequence is limited locally to the targeted cells. So, not only may STING activity be controlled by local administration of radiotherapy but, in addition, also by using target STING and a respective agonist. Secondly, the cGAS/STING pathway is epigenetically silenced in many human tumors, and tumors with low cGAS/STING expression are associated with poor prognosis, likely because of the escape of the tumor to immunity (Chabanon RM et al., Nature Reviews Cancer 2021; 21:701). Hence, by providing exogenous target STING to tumor cells the availability and functionality of the cGAS/STING is ensured. Moreover, activity of exogenous target STING may be controlled by two different means as described before.

It may be provided that after administration of said recombinant expression system, being preferably a viral vector, comprising the first nucleic acid construct the recipient mammal is administered with a human-non-reacting STING agonist which does not react with human STING. Said human-non-reacting STING agonist can be selected from the group consisting of 5,6-dimethylxanthenone-4-acetic acid (DMXAA), 7-bromo-DMXAA, 7-iodo-DMXAA, 7-hydroxy-DMXAA, 7-formyl-DMXAA, 7-hydroxymethyl-DMXAA, 7-(2-hydroxyethyl)-DMXAA, flavone-8-acetic acid (FAA), 2,7-bis(2-dimethylamino ethoxy)fluoren-9-one (Tilorone) and 10-carboxymethyl-9-acridanone (CMA), and more preferably, wherein the STING agonist is DMXAA. The person skilled in the art recognizes that also analogues of the molecules disclosed herein and any of their stereoisomers may be used. In addition, one or more atoms of the aforementioned molecules, their analogues and/or their stereoisomers may be replaced by their respective isotopes.

The use of a human-non-reacting STING agonist in the context of this invention has some advantages when used in the treatment of humans. By definition, the human-non-reacting STING agonist is unable to bind human wild-type STING or natural variants thereof in a way that causes STING activation. Thereby it can be administered systemically without causing a systemic STING response, thus avoiding unwanted side effects. As the invention intends to express a target STING that is responsive to the human-non-reacting STING agonist it is thereby possible to specifically activate this transgenic STING, thus causing a local STING activation. Hence, in the context of the present invention, the human-non-reacting STING agonist has two roles: It is used to define the STING to be used as target STING in the gene therapy and furthermore it is used in the subsequent pharmacotherapy to activate this target STING, thus enabling a highly selective therapeutic approach.

Preferably, the target STING is responsive to DMXAA. A use of a DMXAA-responsive STING has the advantage that the STING agonist DMXAA is already proven in clinical studies as a molecule with beneficial safety and only minor side effects.

Furthermore, it could be shown that the activation of the target STING by DMXAA shows a dose dependent-induction of downstream STING targets such as IFN-β (see Example 1). This allows to control the immune response by simply adjusting the DMXAA dose during treatment.

Intratumor injections of DMXAA have been shown to effectively prime CD8+ T cell responses and promote elimination of the established tumors in a STING-dependent fashion in mice (Corrales et al. Cell Reports, 2015; 11, 1018-1030). Clinical trials addressing the antitumor properties of DMXAA revealed that it shows only minor side effects. However, these trials revealed that DMXAA fails to activate human wild-type STING or natural variants thereof. Nonetheless, murine STING and rat STING are responsive towards DMXAA in that they show biological activity of STING.

It is further conceivable within the scope of the invention that the target STING is a mutated form of a natural variant of human STING that further comprises at least one, at least two, or at least three amino acid substitutions that renders the human STING variant sensitive to the human-non-reacting STING agonist as determined in an *in vitro-agonist* determination assay.

The *in vitro-agonist* determination assay can be used to identify human-non-reacting STING agonists. There are several possible methods to perform such assays known by the person skilled in the art. In one non-limiting example a vector encoding a reporter (e.g. luciferase) under the control of a STING-responsive promoter can be constructed. Cells may be transformed, transfected or transduced with said vector and in addition with vectors that induce the expression of human STING or natural variants thereof, mutated human STING variants or non-human STING. These cells should then be brought in contact with a STING agonist (being preferably DMXAA) and the luciferase activity should be measured. If luciferase activity in cells transformed, transfected or transduced with vectors that induce the expression of mutated human STING variants or nun-human STING are detectable but the luciferase activity in cells transformed, transfected or transduced with vectors that induce the expression of human STING or natural variants thereof does not significantly differ to mock-treated control groups, the agonist is deemed human-non-reacting. Another example of an *in vitro-agonist* determination assay would be to directly measure the IFN-β levels when cells that are transformed, transfected or transduced with vectors that induce the expression of mutated human STING variants, nun-human STING, or human STING or natural variants thereof are contacted with a STING agonist.

It is further conceivable within the scope of the invention that the target STING is a non-human STING, that is sensitive to the human-non-reacting STING agonist as determined in an *in vitro-agonist* determination assay whereby the human-non-reacting STING agonist is preferably DMXAA. Non-limiting examples of non-human STINGs that are sensitive to human-non-reacting STING agonists as determined in an *in vitro-agonist* determination assay are murine STING or natural variants thereof, rat STING or natural variants thereof or a hybrid STING. In a preferred embodiment the non-human STING is a murine STING according to SEQ ID NO: 7, which encodes the protein of SEQ ID NO: 8.

In a preferred embodiment of the invention, the target STING is a DMXAA-sensitive human STING as determined in an *in νitro*-DMXAA determination assay. As described above the use of DMXAA for treatment in a human has the advantages that DMXAA has been shown to have beneficial safety, showing only minor side effects and due to its inability to activate human STING it can be administered systemically without causing systemic STING activation.

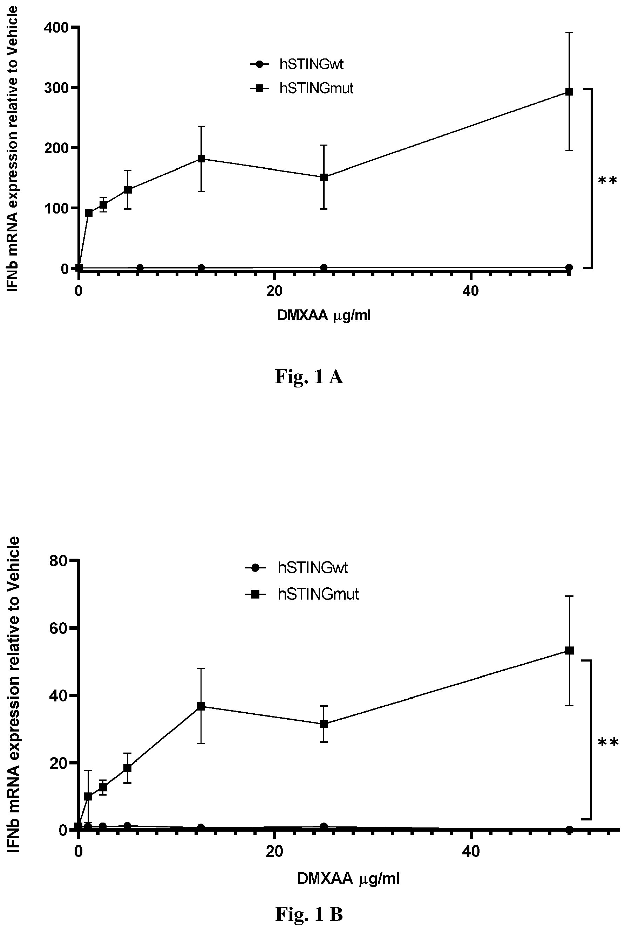

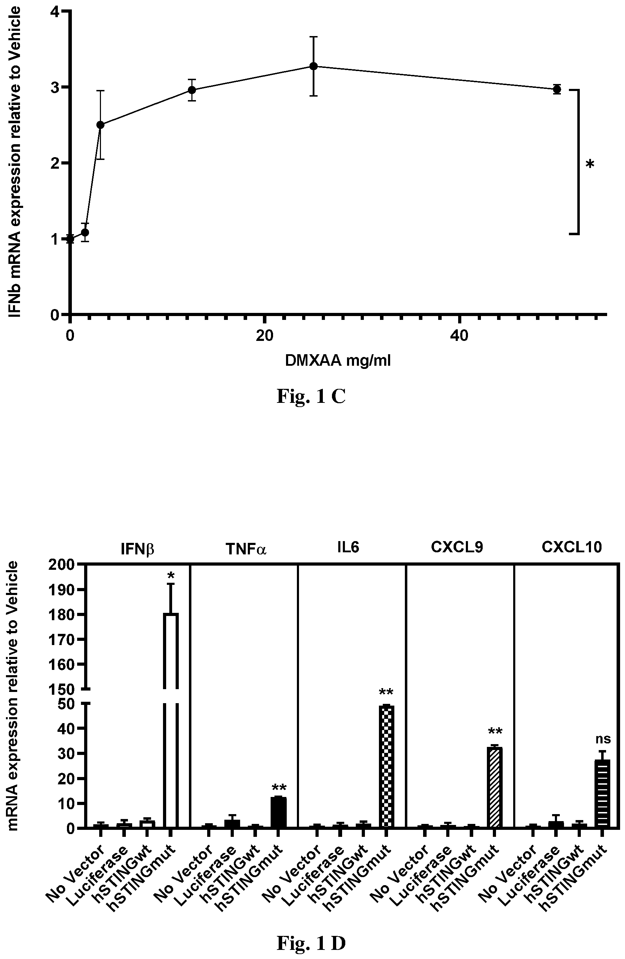

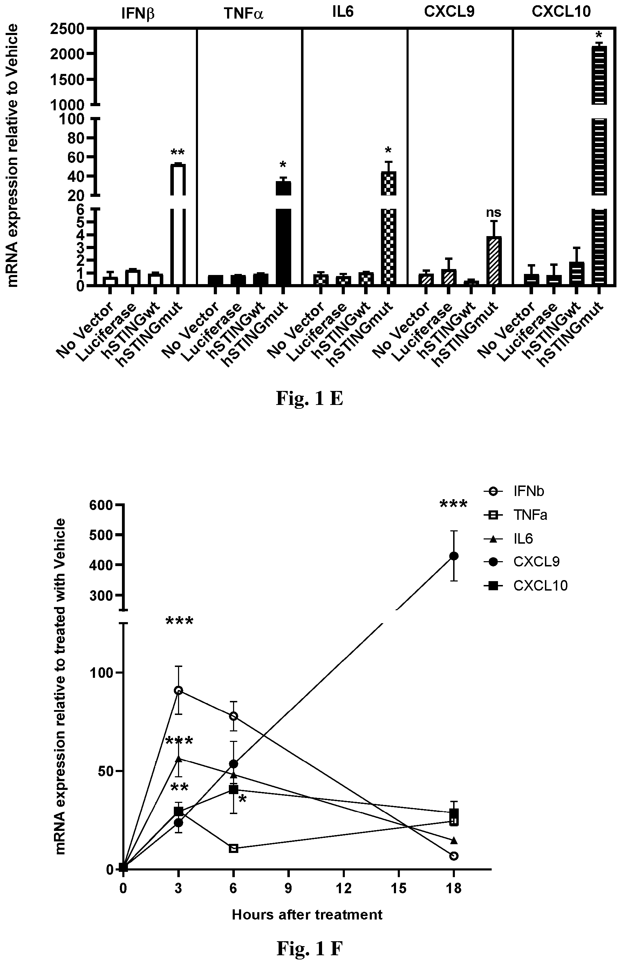

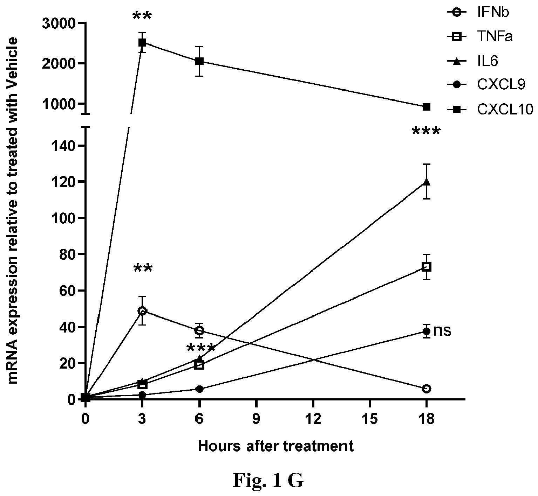

The *in* vitro-DMXAA determination assay can be used to identify STINGs that are responsive to DMXAA. Cells may be transduced with vectors that induce expression of any STING (e.g. human wild-type STING, murine STING). If upon addition of DMXAA, the cells respond by showing, in triplicated determinations, statistically significant elevations in mRNA levels for IFN-β and other cytokines determined by quantitative PCR (qPCR) compared to a mock-treated control, the expressed STING is deemed DMXAA-sensitive (see for Example Fig 1). An example of DMXAA-irresponsive STING is human wild-type STING or natural variants thereof. Another example of an *in vitro*-DMXAA determination assay would be to directly measure the IFN-β levels of cells that were transformed, transfected or transduced with a vector encoding STING (e.g. human wild-type STING, murine STING) after being contacted by DMXAA.

Advantageously, in the context of the invention, it may be provided that the target STING is a DMXAA-sensitive human STING variant which is selected from the group consisting of:
a. a human STING[R] variant further comprising at least 1, at least 2, or at least 3 amino acid substitutions selected from the group consisting of S 162A, G230I, and Q266I;
b. a human STING[AQ] variant further comprising at least 1, at least 2, or at least 3 amino acid substitutions selected from the group consisting of S 162A, A230I, E260I and E260V;
c. a human STING[H] variant further comprising at least 1, at least 2, or at least 3 amino acid substitutions selected from the group consisting of S 162A, G230I, and Q266I; and
d. a truncated human STING[H] variant further comprising at least 1, or at least 2 amino acid substitutions selected from the group consisting of S162A and G230I.

Several natural variants of human STING have been identified, e.g. as a result of several single polymorphisms (SNPs) and SNPs haplotypes which produce several allelic polymorphic forms of the protein. The amino acid sequence of the most common natural variant or allele of human STING is SEQ ID NO: 1, which is referred as the wild-type human STING. The most common natural allelic variants of human STING include: hSTING[R] as depicted in SEQ ID NO: 1, commonly referred as hSTING R232, i.e. amino acid at position 232 is arginine, this STING is also considered the wild-type isoform; hSTING[H] as depicted in SEQ ID NO: 2, commonly referred as hSTING H232, i.e. amino acid at position 232 is histidine; hSTING[AQ] as depicted in SEQ ID NO: 3, whose sequence differs from hSTING[R] by incorporation of amino acid substitutions G230A, and R293Q. In addition, a truncated form of hSTING[H] as depicted in SEQ ID NO: 4 is characterized. A further natural variant is STING[HAQ] which possess the amino acid substitutions R71H, G230A and R293Q.

In a further possibility it may be provided that the DMXAA-sensitive human STING variant encoded by the sequence of the first nucleic acid construct is selected from the group consisting of hSTING[R]-S 162A/G230I/Q266I, hSTING[R]-S 162A/Q266I, hSTING[R]-G230I, hSTING[R]-S162A, hSTING[R]-Q266I, hSTING[R]-S162A/G230I, hSTING[AQ]-S162A/A230I/Q266I, hSTING[AQ]-S162A, hSTING[AQ]-S162A/R169A, hSTING[AQ]-S162A/T267A, hSTING[AQ]-E260I, hSTING[AQ]-E260V, hSTING[H]-S162A/G230I/Q266I, hSTING[H]-S162A/Q266I, hSTING[H]-G230I, hSTING[H]-S162A, hSTING[H]-S162A/G230I and hSTING[H]-Q266I and preferably is hSTING[R]-S162A/G230I/Q266I.

Experiments that have been conducted show that the mutation S162A is crucial in allowing DMXAA to bind and activate human STING and that the mutations Q266I and G230I or A230I increase the sensitivity towards DMXAA (Gao et al., 2014, Cell Reports 8, 1668-1676).

According to a further advantage it can be provided that the DMXAA-sensitive human STING variant is selected from the group consisting of SEQ ID NO: 5 which is the sequence of hSTING[R]-S162A/G230I/Q266I or a polynucleotide sequence having at least 75%, at least 80%, at least 85%, at least 90% or at least 95% sequence identity with respect to SEQ ID NO: 5. The sequence identity of the polynucleotide sequence with respect to SEQ ID NO: 5 can be at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. Notably, the polypeptide encoded by any of the aforementioned polynucleotides has the biological activity of being responsive to the agonist DMXAA as determined in an *in vitro*-DMXAA determination assay.

According to a further advantage it can be provided that the DMXAA-sensitive target STING is selected from the group consisting of a polynucleotide sequence encoding a polypeptide comprising SEQ ID NO: 6 which is the sequence of hSTING[R]-S162A/G230I/Q266I or a polynucleotide sequence encoding a polypeptide having at least 75%, at least 80%, at least 85%, at least 90% or at least 95% sequence identity with respect to SEQ ID NO: 6. The sequence identity of the polypeptide encoded by the polynucleotide sequence with respect to SEQ ID NO: 6 can be at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. Notably, the polypeptide encoded by any of the aforementioned polynucleotides has the biological activity of being responsive to a human-non-reacting STING agonist as determined in an *in vitro-agonist* determination assay.

Both, hSTING[R]-S162A/G230I/Q266I and murine STING, have extensively been researched and are known to be highly activated by DMXAA and CMA. In addition, murine STING may also be activated by FAA and Tilorone. Using a variant of human STING however may be advantageous over the use of non-human STING in the treatment of cancer as the former might show better interaction with signal-transducing molecules in human cells. In addition, the use of non-human STING might cause unwanted immunologic responses.

Notably, the polynucleotide sequences from the first nucleic acid construct comprising a sequence encoding for a target STING as described hereinbefore may comprise at least one modification of the polypeptide that is an amino acid substitution, amino acid insertion, amino acid deletion, C-terminal truncation or N-terminal truncation. In addition, also polynucleotide sequences that hybridize under stringent conditions to the complement of the aforementioned polynucleotide sequences are included according to the invention. Furthermore, fragments of at least 50 amino acid residues of the DMXAA-sensitive human STING variant are comprised according to the invention. Notably, the polypeptide encoded by any of the aforementioned polynucleotides has the biological activity of being responsive to a human-non-reacting STING agonist as determined in an *in vitro-agonist* determination assay.

Preferably, it may be provided that the promoter of the first nucleic acid construct comprising a sequence encoding for a target STING is a constitutive promoter such as SV40, CMV0, UBC, EF1a, PGK, CAG, EFS, CBh, CBA, human β actin, Ac5, Polyhedrin, TEF1, GDS, CaMV35S, Ubi, H1 or U6. Preferably, the promoter is selected from the list consisting of SV40, CMV0, UBC, EF1a, PGK, CAG, EFS, CBh and CBA. Using a constitutive promoter in the context of the invention has the advantage that expression of the target STING is ensured. In general, constitutive promoters show higher expression levels compared to inducible or tissue-specific promoters. Alternatively, the promoter may be an inducible promoter, preferably selected from the list consisting of telomerase-responsive promoter, IL-6 responsive promoter, TGF-β-inducible promoter, VEGFR-inducible promoter, Epidermal Growth factor receptor-inducible promoter, HER2/Neu-inducible promoter, MUC1 promoter, BRCA1 promoter, BRCA2 promoter, LALBA promoter, PSA promoter, ERBB2 promoter, CEA promoter, uPAR promoter, Thyroid transcription factor1 (TTF1 ). However, the person skilled in the art recognizes that also cancer-specific promoters are feasible promoters of the nucleotide sequence in the context of the invention.

According to a further possibility it may be provided that the second nucleic acid construct comprising the therapeutic gene sequence further comprises a transgene encoding a thymidine kinase, preferably a thymidine kinase according to SEQ ID NO: 16. The nucleotide sequence encoding the thymidine kinase is linked to the nucleotide sequence of the therapeutic gene sequence and may be interspaced by a 2A self-cleaving peptides (2A peptide). 2A peptides constitute a class of 18-22 amino acid-long peptides, which can induce ribosomal skipping during translation of a protein in a cell. These peptides share a core sequence motif of DXEXNPGP and are found in a wide range of viral families. They help generating polyproteins by causing the ribosome to fail at making a peptide bond. The person skilled in the art recognizes that the use of a 2A peptide allows the co-expression of the therapeutic gene sequence and the thymidine kinase. The co-expression of the thymidine kinase is advantageous in that it allows to assess tumor transduction by PET imaging and, in case of potential IL-12-mediated toxicity, to eliminate the cells expressing the cytokine by administering a thymidine kinase substrate such as for example acyclovir, penciclovir, famciclovir or ganciclovir.

The first and/or the second nucleic acid construct of the recombinant expression system of the invention, being preferably a viral vector, may further comprise a woodchuck hepatitis virus post-transcriptional regulatory element (WPRE). The WPRE is known to increase transgene expression, whereby it is most effective when placed downstream of the transgene (i.e. the target STING or the therapeutic gene sequence), proximal to the polyadenylation signal.

Expediently, the first and/or the second nucleic acid construct of the recombinant expression system of the invention, being preferably a viral vector, further comprises a polyadenylation signal located at the 3'position with respect to the target STING or the therapeutic gene sequence.

In a special embodiment, the inventive recombinant expression system consists of a second nucleic acid construct as given in the following table, wherein the different elements are shown in the 5'to 3'direction as promoter, first open reading frame (ORF1), first translation regulatory element (TRE1), second open reading frame (ORF2), second translation regulatory element (TRE2) and target sequence for a microRNA (microRNA element).

**Table 1: Embodiments of the second nucleic acid construct of the expression system**

| **Promoter** | **ORF1** | **TRE1** | **ORF2** | **TRE2** | **microRNA element** |
|---|---|---|---|---|---|
| ISREx4 ^{A} | scIL12 ^{B} | | | | |
| ISREx4 ^{A} | scIL12 ^{C} | | | | |
| IFN-β minimal | scIL12 ^{B} | | | | |
| IFN-β minimal | scIL12 ^{C} | | | | |
| ISREx4 ^{A} | scIL12 ^{B} | | | | miR122 target site ^{E} |
| ISREx4 ^{A} | scIL12 ^{C} | | | | miR122 target site ^{E} |
| IFN-β minimal | scIL12 ^{B} | | | | miR122 target site ^{E} |
| IFN-β minimal | scIL12 ^{C} | | | | miR122 target site ^{E} |
| ISREx4 ^{A} | scIL12 ^{B} | | | WPRE | miR122 target site ^{E} |
| ISREx4 ^{A} | scIL12 ^{C} | | | WPRE | miR122 target site ^{E} |
| IFN-β minimal | scIL12 ^{B} | | | WPRE | miR122 target site ^{E} |
| IFN-β minimal | scIL12 ^{C} | | | WPRE | miR122 target site ^{E} |
| ISREx4 ^{A} | HSV-TK ^{D} | IRES | scIL12 ^{B} | WPRE | miR122 target site ^{E} |
| ISREx4 ^{A} | HSV-TK ^{D} | IRES | scIL12 ^{C} | WPRE | miR122 target site ^{E} |
| ISREx4 ^{A} | HSV-TK ^{D} | p2A | scIL12 ^{B} | WPRE | miR122 target site ^{E} |
| ISREx4 ^{A} | HSV-TK ^{D} | p2A | scIL12 ^{C} | WPRE | miR122 target site ^{E} |
| ISREx4 ^{A} | scIL12 ^{B} | IRES | HSV-TK ^{D} | WPRE | miR122 target site ^{E} |
| ISREx4 ^{A} | scIL12 ^{C} | IRES | HSV-TK ^{D} | WPRE | miR122 target site ^{E} |
| ISREx4 ^{A} | scIL12 ^{B} | p2A | HSV-TK ^{D} | WPRE | miR122 target site ^{E} |
| ISREx4 ^{A} | scIL12 ^{C} | p2A | HSV-TK ^{D} | WPRE | miR122 target site ^{E} |

| | | | | | |
|---|---|---|---|---|---|
| A - 4 copies of ISRE, preferably given by SEQ ID NO: 12 B - human single chain IL-12 as depicted in SEQ ID NO: 9 C - murine single-chain IL-12 as depicted in SEQ ID NO: 10 D - HSV thymidine kinase as depicted in SEQ ID NO: 16 E - 5 copies of miR-122 target sequence as depicted in SEQ ID NO: 15 | | | | | |

Another aspect of the invention is an expression vector encoding the first and the second nucleic acid construct according to the invention.

Using an expression vector comprising the first and/or second nucleic acid construct has certain advantages. Firstly, expression vectors are specifically designed to allow the introduction of the encoded nucleotide sequences into a host cell and commandeer their expression. Secondly, expression vectors are plasmids that are very stable and thereby protected from degradation. Thirdly, expression vectors are conceivable for gene delivery using different methods. A non-limiting example of an expression vector and a preferred embodiment of the invention is a viral vector, more preferably a viral vector selected from the list consisting of adeno-associated virus (AAV) vector, adenoviral vector, lentiviral vector, vaccine virus vector, or herpes simplex virus vector. Viral vectors in particular combine several advantages of expression vectors such as general safety, low toxicity and stability.

It is conceivable that the first and second nucleic acid construct of the expression system according to the invention are contained within a single expression vector. This has the advantage that one administration of said expression vector is sufficient to express the expression system according to the invention in the patient.

An alternative aspect of the invention is a combination of a first and a second expression vector, the first vector encodes the first nucleic acid construct, and the second vector encodes the second nucleic acid construct according to the invention. This has the advantage that the size of the respective nucleotide sequence is not as limited as it would be if both nucleic acid constructs would be combined in one vector. Size limitation is a frequently occurring problem with viral vectors. For example, adeno-associated viruses (AAV) are limited to a size of around 5 kb, whereas it has to be taken into consideration that the ITRs, the promoter and other elements are not included in the 5 kb.

Thus, in an alternative aspect, the recombinant expression system of the invention provides a combination of a first expression vector encoding the first nucleic acid construct and a second expression vector encoding the second nucleic acid construct.

It is conceivable that the first and second nucleic acid construct according to the invention are contained within a single viral vector. This has the advantage that one administration of said viral vector is sufficient to express the expression system according to the invention in the patient.

It is also advantageous that the first and/or the second nucleic acid construct of the viral vector(s) further comprise a 5'ITR and a 3'ITR sequence, being preferably AAV ITR as depicted in SEQ ID NO: 17. It may be advantageous that the viral vector(s) is (are) an AAV vector, preferably an AAV8, AAV1, AAV3, AAV6, AAV9, AAV2, AAV5, AAVrh.10, or any gain-of-function mutant of AAVrh.10. AAVs are commonly used in biotechnology and therefore well characterized. Advantages of using AAV vectors is that they are generally regarded as safe, display low toxicity and are stable. Moreover, AAVs are already approved by several governmental organizations around the world for use in human.

It is also advantageous that the first and/or the second nucleic acid construct of the viral vector(s) further comprise a 5'ITR, a ψ packaging signal and a 3'ITR sequence, being preferably the 5'ITR, the ψ packaging signal and the 3'ITR of an adenovirus (Ad). It may be advantageous that the viral vector(s) is (are) an Ad vector, preferably a high-capacity Ad vector. Adenoviruses are commonly used in biotechnology and therefore well characterized. An advantage of Ad vectors is that they have a higher size limitation compared to AAV vectors. Moreover, Adenoviruses are already approved by several governmental organizations around the world for use in human.

It is also advantageous that the viral vector(s) is (are) a lentiviral vector, a vaccine virus vector or a herpes simplex virus vector. These vectors are commonly used in biotechnology and therefore well characterized.

It is also conceivable that nanoparticles may be used to deliver the first and/or second nucleic acid construct of the invention. Nanoparticles as gene delivery systems are known in the art and have advantages like tunable size, shape and/or surface thereby allowing to regulate their biological behaviors.

It is also possible that viral particle(s) will be used to deliver the viral vector(s) that contain the first and/or the second nucleic acid construct of the expression system of the invention.

Another aspect of the invention is a viral particle comprising the aforementioned viral vector which comprises the first and second nucleic acid construct of the invention. This has the advantage that one administration of said viral particle is sufficient to express the expression system according to the invention in the patient.

Yet another aspect of the invention is a combination of a first and a second viral particle, the first viral particle comprises a viral vector encoding the first nucleic acid construct and the second viral particle comprises a viral vector encoding the second nucleic acid construct according to the invention. This has the advantage that the size of the respective nucleic acid construct is not as limited as it would be if both nucleic acid constructs would be combined in one viral vector of a single viral particle. Size limitation is a frequently occurring problem with viral vectors. For example, adeno-associated viruses (AAV) are limited to a size of around 5 kb, whereas it has to be taken into consideration that the ITRs, the promoter and other elements are not included in the 5 kb.

The aforementioned viral particle(s) is (are) preferably (an) AAV or (an) Ad viral particle(s); more preferably (an) AAV viral particle(s). As described hereinbefore, Ad and AAV display several advantages over other viral particles such as that they are well characterized, frequently used and approved for treatment in humans. These viruses display further advantages that render them particular useful in gene therapy such as that they are capable of transducing dividing as well as non-dividing cells and that they do not integrate into the host genome.

In an alternative embodiment the combination of the first expression vector and the second expression vector, or the combination of the first viral particle and the second viral particle are provided as a Kit-of-parts.

Another aspect of the invention is a pharmaceutical composition that comprises a pharmaceutically acceptable excipient together with the viral vector(s) or the viral particle(s) according to the invention. Pharmaceutical compositions have the advantage that they are "ready-to-use" in that they may be administered directly to the patient for treatment. In addition, pharmaceutic compositions are tailored to optimize stability and availability of the ingredients according to the therapeutic need while avoiding unwanted side effects for the patient.

It should also be expressly noted that, in the context of the present patent application, indefinite articles and numerical indications such as "a", "an", "one", "two", etc. are generally to be understood as "at least"-indications, i.e. as "at least one...", "at least two...", etc., unless it expressly follows from the respective context or it is obvious or technically imperative to a person skilled in the art that only "exactly one...", "exactly two...", etc. can be meant there.

Further advantages, features and details of the invention will be apparent from the following description, in which embodiments of the invention are described in detail with reference to the figures. In this connection, the features mentioned in the claims and in the description may each be essential to the invention individually or in any combination.

The foregoing explanation of the embodiments describes the present invention exclusively in the context of examples. Of course, individual features of the embodiments can be freely combined with each other, provided that this is technically reasonable, without leaving the scope of the present invention.

### Definitions

Substantially: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and/or chemical phenomena.

Approximately: The term "approximately" as used herein may be applied to modify any quantitative comparison, value, measurement, or other representation that could permissibly vary without resulting in a change in the basic function to which it is related.

A/an: As used herein the specification, "a" or "an" may mean one or more. As used herein in the claim(s), when used in conjunction with the word "comprising," the words "a" or "an" may mean one or more than one.

Or/and/or: The use of the term "or" in the claims is used to mean "and/or" unless explicitly indicated to refer to alternatives only or the alternatives are mutually exclusive, although the disclosure supports a definition that refers to only alternatives and "and/or."

The term "about" means, in general, within a standard deviation of the stated value as determined using a standard analytical technique for measuring the stated value. The terms can also be used by referring to plus or minus 5% of the stated value.

Essentially: The term "essentially" is to be understood that methods or compositions include only the specified steps or materials and those that do not materially affect the basic and novel characteristics of those methods and compositions.

Stereoisomers: In addition, the compounds used in the present invention may contain one or more asymmetric carbon atoms and therefore exists in two or more stereoisomeric forms. Where a compound contains an alkenyl or alkenylene group, cis (E) and trans (Z) isomerism may also occur. The present invention includes use of the individual stereoisomers of the compound and, where appropriate, the individual tautomeric forms thereof, together with mixtures thereof.

Analogues: As used herein the term "analogue" refers to a chemical compound that is structurally similar to another but differs slightly in composition, such as in the replacement of one atom by an atom of a different element or by a functional group.

Isotopes in compounds: The present invention also includes use of all suitable isotopic variations of the compounds described herein or a pharmaceutically acceptable salt thereof. An isotopic variation of a compound of the present invention or a pharmaceutically acceptable salt thereof is defined as one in which at least one atom is replaced by an atom having the same atomic number but an atomic mass different from the atomic mass usually found in nature. Examples of isotopes that can be incorporated into the compounds and pharmaceutically acceptable salts thereof include isotopes of hydrogen, carbon, nitrogen, oxygen, phosphorus, sulphur, fluorine and chlorine such as 2H, 3H, 13C, 14C, 15N, 170, 180, 31P, 32P, 35S, 18F and 36Cl, respectively. Certain isotopic variations of the compound and pharmaceutically acceptable salts thereof, for example, those in which a radioactive isotope such as 3H or 14C is incorporated, are useful in drug and/or substrate tissue distribution studies. Tritiated, i.e., 3H, and carbon-14, i.e., 14C, isotopes are particularly preferred for their ease of preparation and detectability. Further substitution with isotopes such as deuterium, i.e., 2H, may afford certain therapeutic advantages resulting from greater metabolic stability, for example, increased *in vivo* half-life or reduced dosage requirements and hence may be preferred in some circumstances. Isotopic variations of the compounds of the present invention and pharmaceutically acceptable salts thereof can generally be prepared by conventional procedures using appropriate isotopic variations of suitable reagents.

Cytokine: The term "cytokine" refers to the general class of biological molecules which effect/affect cells of the immune system. The definition is meant to include, but is not limited to, those biological molecules that act locally or may circulate in the blood, and which, when used in the compositions or methods of the present invention serve to regulate or modulate an individual's immune response to cancer. Cytokines may be a number of different substances such as chemokines, interferons, interleukins and growth factors. Exemplary cytokines for use in practicing the invention include but are not limited to interferons (e.g. IFN-α, IFN-β, and IFN-γ), interleukins (e.g., IL-1 to IL-29, in particular, IL-2, IL-7, IL-12, IL-15 and IL-18), tumor necrosis factors (e.g., TNF-α and TNF-β), chemokines (e.g. CXCL9, CXCL10), granulocyte-macrophage colony-stimulating factor (GM-CSF), TGF-β, FLT3-ligand, and fragments or variants thereof.

Immunomodulatory: As used herein, the term "immunomodulatory" refers to the property of initiating or modifying (e.g., increasing or decreasing) an activity of a cell involved in an immune response. An immunomodulatory composition or method may increase an activity of a cell involved in an immune response, e.g., by increasing pro- or anti-inflammatory markers, and/or may decrease an activity of a cell involved in an immune response, e.g., by decreasing pro- or anti-inflammatory markers.

Agonist/human-non-reacting STING agonist: As used herein, the term "agonist" refers to a ligand that when bound to a protein stimulates its activity. The term "human-non-reacting STING agonist" refers to an agonist that substantially does not bind to human wild-type STING or natural variants thereof but binds to mutated human STING or STING from other species such as STING derived from mouse, rat or monkey. Optionally, the term "human-non-reacting STING agonist" refers to an agonist that binds human wild-type STING or natural variants thereof without inducing STING activity, whereas STING activity of mutated human STING or STING from other species such as STING derived from mouse, rat or monkey is induced upon binding of said agonist.

Protein/polypeptide: The terms "protein," "peptide," and "polypeptide," are used interchangeably herein, and refer to a polymer of amino acid residues linked together by peptide (amide) bonds. The terms refer to a protein, peptide, or polypeptide of any size, structure, or function. Typically, a protein, peptide, or polypeptide will be at least three amino acids long. A protein, peptide, or polypeptide may refer to an individual protein or a collection of proteins. One or more of the amino acids in a protein, peptide, or polypeptide may be modified, for example, by the addition of a chemical entity such as a carbohydrate group, a hydroxyl group, a phosphate group, a farnesyl group, an isofarnesyl group, a fatty acid group, a linker for conjugation, functionalization, or other modification, etc. A protein, peptide, or polypeptide may also be a single molecule or may be a multi-molecular complex. A protein, peptide, or polypeptide may be just a fragment of a naturally occurring protein or peptide. A protein, peptide, or polypeptide may be naturally occurring, recombinant, or synthetic, or any combination thereof.

Antibody/monoclonal antibody: As used herein, the term "antibody" collectively refers to immunoglobulins or immunoglobulin-like molecules including by way of example and without limitation, IgA, IgD, IgE, IgG and IgM, combinations thereof, and similar molecules produced during an immune response in any vertebrate, for example, in mammals such as humans, goats, rabbits, llama and mice, as well as non-mammalian species, such as shark immunoglobulins. As used herein, the term "monoclonal antibody" refers to an antibody produced by a single clone of B-lymphocytes or by a cell into which the light and heavy chain genes of a single antibody have been transfected. Monoclonal antibodies are produced by methods known to those of skill in the art, for instance by making hybrid antibody-forming cells from a fusion of myeloma cells with immune spleen cells. Monoclonal antibodies include humanized monoclonal antibodies.

Single-chain variable fragment/scFv: The terms "single-chain variable fragment" and "scFv" refer to a fusion protein comprising at least one antibody fragment comprising a variable region of a light chain and at least one antibody fragment comprising a variable region of a heavy chain, wherein the light and heavy chain variable regions are contiguously linked via a short flexible polypeptide linker, and capable of being expressed as a single polypeptide chain, and wherein the scFv retains the specificity of the intact antibody from which it is derived.

The term "nanobody", as used herein, refers to the smallest antigen binding fragment or single variable domain (VHH) derived from naturally-occurring heavy chain antibody and is known to the person skilled in the art.

The term "blocking peptide," as used herein, refers to peptides that can interfere with the molecule that they are targeting in a way that said molecule is impaired in its interaction with its targets. For example, a blocking peptide targeting TGF-β impairs its immunosuppressive function.

Nucleic acid: The term "nucleic acid" includes any nucleotides, analogues thereof, and polymers thereof. The terms "polynucleotide" or "nucleotide sequence" as used herein refer to a polymeric form of nucleotides of any length, either ribonucleotides (RNA) or deoxyribonucleotides (DNA). These terms refer to the primary structure of the molecules and, thus, include double- and single-stranded DNA, and double- and single-stranded RNA. These terms include, as equivalents, analogues of either RNA or DNA made from nucleotide analogues and modified polynucleotides such as, though not limited to, methylated, protected and/or capped nucleotides or polynucleotides. The terms encompass poly- or oligo-ribonucleotides (RNA) and poly- or oligo deoxyribonucleotides (DNA); RNA or DNA derived from N-glycosides or C-glycosides of nucleobases and/or modified nucleobases; nucleic acids derived from sugars and/or modified sugars; and nucleic acids derived from phosphate bridges and/or modified phosphorus-atom bridges. The term encompasses nucleic acids containing any combinations of nucleobases, modified nucleobases, sugars, modified sugars, phosphate bridges or modified phosphorus atom bridges. Examples include, and are not limited to, nucleic acids containing ribose moieties, the nucleic acids containing deoxyribose moieties, nucleic acids containing both ribose and deoxyribose moieties, nucleic acids containing ribose and modified ribose moieties. In some embodiments, the prefix polyrefers to a nucleic acid containing 2 to about 10,000, 2 to about 50,000, or 2 to about 100,000 nucleotide monomer units. In some embodiments, the "oligonucleotide" refers to a nucleic acid containing 2 to about 200 nucleotide monomer units.

Gene: As used herein, the term "gene" means a segment of DNA that contains all the information for the regulated biosynthesis of an RNA product, including promoters, exons, introns, and other untranslated regions that control expression. Also shRNA are included by this definition.

Therapeutic gene sequence: As used herein, the term "therapeutic gene sequence" means any nucleotide sequence that encodes a therapeutic molecule useful in the treatment of a disease. Non-limiting examples of such molecules are proteins, shRNA, hormones or blocking peptides.

Immunomodulatory protein: As used herein, the term "immunomodulatory protein" refers to a protein that modulates ( e.g. by stimulating an immunostimulatory protein or by inhibiting an immunoinhibitory protein) the immune system by inducing activation and/or increasing activity of immune system components.

Hybridize: The terms "hybridizing" or "hybridizes" as used herein is to be understood as two nucleic acid strands (e.g. an oligonucleotide and a target nucleic acid) forming hydrogen bonds between base pairs on opposite strands thereby forming a duplex.

Promoter: The term "promoter" is used herein in its ordinary sense to refer to a nucleotide region comprising a DNA regulatory sequence, wherein the regulatory sequence is derived from a gene and is capable of binding RNA polymerase and initiating transcription of a downstream (3' direction) coding sequence. It may contain genetic elements at which regulatory proteins and molecules may bind, such as RNA polymerase and other transcription factors, to initiate the specific transcription of a nucleic acid sequence. The phrases "operatively positioned", "operatively linked", "under control", and "under transcriptional control" mean that a promoter is in a correct functional location and/or orientation in relation to a nucleic acid sequence to control transcriptional initiation and/or expression of that sequence.

Constitutive promoter: The term "constitutive promoter" refers to a nucleotide sequence which, when operably linked with a polynucleotide which encodes or specifies a gene product, causes the gene product to be produced in a cell under most or all physiological conditions of the cell.

Inducible promoter: The term "inducible promoter" refers to a nucleotide sequence which, when operably linked with a polynucleotide which encodes or specifies a gene product, causes the gene product to be produced in a cell substantially only when an inducer which corresponds to the promoter is present in the cell.

STING-responsive promoter: As used herein, the term "STING-responsive promoter" refers to a nucleotide sequence which, when operably linked with a polynucleotide which encodes or specifies a gene product, causes the gene product to be produced in a cell substantially only when a polypeptide that is regulated by STING activity is present in the cell. Non-limiting examples of such polypeptides are cytokines, especially interferons such as IFN-β, chemokines such as CXCL9 or CXCL10, transcription factors such as NFkB or STATs, interleukins such as IL-6, and TNF-α.

Mutation: The term "mutation," as used herein, refers to a substitution of a residue within a sequence, e.g., a nucleic acid or amino acid sequence, with another residue, or a deletion or insertion of one or more residues within a sequence. Mutations are typically described herein by identifying the original residue followed by the position of the residue within the sequence and by the identity of the newly substituted residue. Various methods for making the amino acid substitutions (mutations) provided herein are well known in the art, and are provided by, for example, Green and Sambrook, Molecular Cloning: A Laboratory Manual (4th ed., Cold Spring Harbor Laboratory Press, Cold Spring Harbor, N.Y. (2012)).

Identity: As used herein, the term "identity" refers to the overall relatedness between polymeric molecules, e.g., between polypeptide molecules and/or between nucleic acid molecules (e.g., DNA molecules and/or RNA molecules). In some embodiments, polymeric molecules are considered to be "substantially identical" to one another if their sequences are at least 25%, 30%, 35%, 40%, 45%, 50%, 55%, 60%, 65%, 70%, 75%, 80%, 85%, 90%, 95%, or 99% identical. Calculation of the percent identity of two nucleic acid or polypeptide sequences, for example, can be performed by aligning the two sequences for optimal comparison purposes (e.g., gaps can be introduced in one or both of a first and a second sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of a reference sequence. The nucleotides or amino acids at corresponding positions are then compared. When a position in the first sequence is occupied by the same residue (e.g., nucleotide or amino acid) as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4: 11-17), which has been incorporated into the ALIGN program (version 2.0). In some exemplary embodiments, nucleic acid sequence comparisons made with the ALIGN program use a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

Hybridization under stringent conditions: In the context of the present invention, a hybridization under stringent conditions refers to hybridization conditions, in which only long sequences with nearly perfect complementary matching will secure anneal. This term is well-known in the art of genetic engineering and has been used in the patent practice for numerous years as a quasi-structural feature for defining DNA claims. Notably, the respective claims define their subject-matter not only by the capability of hybridizing under stringent conditions, but also by a further functional feature, namely that the polypeptide encoded by the claimed DNA sequence has at least the functional property of STING.

Linked: As used herein, the term "linked", when used with respect to two or more moieties, means that the moieties are physically associated or connected with one another to form a molecular structure that is sufficiently stable so that the moieties remain associated under the conditions in which the linkage is formed and, preferably, under the conditions in which the new molecular structure is used, e.g., physiological conditions. In certain preferred embodiments of the invention the linkage is a covalent linkage. In other embodiments the linkage is noncovalent. Moieties may be linked either directly or indirectly. When two moieties are directly linked, they are either covalently bonded to one another or are in sufficiently close proximity such that intermolecular forces between the two moieties maintain their association. When two moieties are indirectly linked, they are each linked either covalently or noncovalently to a third moiety, which maintains the association between the two moieties. In general, when two moieties are referred to as being linked by a "linker" or "linking moiety" or "linking portion", the linkage between the two linked moieties is indirect, and typically each of the linked moieties is covalently bonded to the linker. The linker can be any suitable moiety that reacts with the two moieties to be linked within a reasonable period of time, under conditions consistent with stability of the moieties (which may be protected as appropriate, depending upon the conditions), and in sufficient amount, to produce a reasonable yield.

Operably linked/operatively linked: As used herein, the terms "operably linked" or "operatively linked" refer to a juxtaposition wherein the components described are in a relationship permitting them to function in their intended manner. A control element such as a promoter "operably linked" to a functional element is associated in such a way that expression and/or activity of the functional element is achieved under conditions compatible with the control element. In some embodiments, "operably linked" control elements are contiguous (e.g., covalently linked) with the coding elements of interest; in some embodiments, control elements act in trans to the functional element of interest.

RNA interference/micro RNA/shRNA: As used herein, the term "RNA interference" refers generally to a process in which a RNA molecule reduces or inhibits expression of a nucleic acid sequence with which the RNA molecule shares substantial or total homology. Without wishing to be bound by any theory, it is believed that, in nature, the RNA interference pathway is initiated by a Type III endonuclease known as Dicer, which cleaves long double-stranded RNA (dsRNA) into double-stranded fragments typically of 21-23 base pairs with 2-base 3' overhangs (although variations in length and overhangs are also contemplated), referred to as "short interfering RNAs" ("siRNAs"). Such siRNAs comprise two single-stranded RNAs (ssRNAs), with an "antisense strand" or "guide strand" that includes a region that is substantially complementary to a target sequence, and a "sense strand" or "passenger strand" that includes a region that is substantially complementary to a region of the antisense strand. Those of ordinary skill in the art will appreciate that a guide strand may be perfectly complementary to a target region of a target RNA or may have less than perfect complementarity to a target region of a target RNA. As used herein, the terms "micro RNA" or "miRNA" or "miR" refer to a specific type of siRNA that are produced physiologically in the organism rather than produced synthetically miRNA bind to respective miRNA target sequences and reduce or inhibit expression of a nucleic acid sequence with which the miRNA shares a substantial or total homology. As used herein, the term "shRNA" or "short hairpin RNAs" refers to individual transcripts that adopt stem-loop structures which are processed into siRNA by RNAi machinery. Typical shRNA molecules comprise two inverted repeats containing the sense and antisense target sequence separated by a loop sequence. The base-paired segment may be any suitable length that allows inactivation of a target gene *in vivo,* wherein one strand of the base-paired stem is complementary to the mRNA of said target gene. The loop of the shRNA stem-loop structure may be any suitable length that allows inactivation of the target gene *in vivo.* The base paired stem may be perfectly base paired or may have 1 or 2 mismatched base pairs.

Target gene/RNA: A "target gene", as used herein, refers to a gene whose expression is to be modulated, e.g., upregulated or inhibited. As used herein, the term "target RNA" refers to an RNA to be degraded or translationally repressed or otherwise inhibited using one or more agents, e.g., one or more miRNAs or siRNAs. A target RNA may also be referred to as a target sequence or target transcript. The RNA may be a primary RNA transcript transcribed from the target gene (e.g., a pre-mRNA) or a processed transcript, e.g., mRNA encoding a polypeptide. As used herein, the term "target portion" or "target region" refers to a contiguous portion of the nucleotide sequence of a target RNA. In some embodiments, a target portion of an mRNA is at least long enough to serve as a substrate for RNA interference (RNAi)-mediated cleavage within that portion in the presence of a suitable miRNA or siRNA. A target portion may be from about 8-36 nucleotides in length, e.g., about 10-20 or about 15-30 nucleotides in length. A target portion length may have specific value or subrange within the afore-mentioned ranges. For example, in certain embodiments a target portion may be between about 15-29, 15-28, 15-27, 15- 26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19- 28, 19-27, 19-26, 19-25, 19- 24, 19-23, 19-22, 19-21, 19-20, 20-30, 20-29, 20-28, 20-27, 20- 26, 20-25, 20-24, 20-23, 20-22, 20-21, 21-30, 21-29, 21-28, 21-27, 21-26, 21-25, 21-24, 21-23, or 21-22 nucleotides in length.

Recombinant: As used herein, the term "recombinant" is intended to refer to nucleic acids or polypeptides that are designed, engineered, prepared, expressed, created, manufactured, and/or or isolated by recombinant means, such as polypeptides expressed using a recombinant expression vector transfected into a host cell; polypeptides isolated from a recombinant, combinatorial human polypeptide library; polypeptides isolated from an animal (e.g., a mouse, rabbit, sheep, fish, etc.) that is transgenic for or otherwise has been manipulated to express a gene or genes, or gene components that encode and/or direct expression of the polypeptide or one or more component(s), portion(s), element(s), or domain(s) thereof; and/or polypeptides prepared, expressed, created or isolated by any other means that involves splicing or ligating selected nucleic acid sequence elements to one another, chemically synthesizing selected sequence elements, and/or otherwise generating a nucleic acid that encodes and/or directs expression of the polypeptide or one or more component(s), portion(s), element(s), or domain(s) thereof. In some embodiments, one or more of such selected sequence elements is found in nature. In some embodiments, one or more of such selected sequence elements is designed in silico. In some embodiments, one or more such selected sequence elements results from mutagenesis (e.g., *in vivo* or *in vitro)* of a known sequence element, e.g., from a natural or synthetic source such as, for example, in the germline of a source organism of interest (e.g., of a human, a mouse, etc.).

Natural variant: The term "natural variant" refers to variants of genes or transcripts which originate from the same genetic loci as the target nucleic acid, but may differ for example, by virtue of degeneracy of the genetic code causing a multiplicity of codons encoding the same amino acid, or due to alternative splicing of pre-mRNA, or the presence of polymorphisms, such as single nucleotide polymorphisms (SNPs), and allelic variants. Said natural variants occur in nature and are not artificially generated. In contrast, when the term "variant" is used without the word "natural" being associated to it, the variants may comprise artificially generated variants such as mutations. In the case of human STING some known natural variants are STING[R], STING[AQ], STING[H], STING[HAQ] and a truncated form of STING[H].

Wild-type: As used herein "wild-type" refers to the naturally occurring sequence of a nucleic acid at a genetic locus in the genome of an organism, and sequences transcribed or translated from such a nucleic acid. Thus, the term "wild-type" also may refer to the amino acid sequence encoded by the nucleic acid. As a genetic locus may have more than one sequence or alleles in a population of individuals, the term "wild-type" encompasses all such naturally occurring alleles. As used herein the term "polymorphic" means that variation exists (i.e., two or more alleles exist) at a genetic locus in the individuals of a population. As used herein, "mutant" refers to a change in the sequence of a nucleic acid or its encoded protein, polypeptide, or peptide that is the result of recombinant DNA technology.

Non-human: As used herein the term "non-human" refers to a gene or protein that is not substantially derived from wild-type human genes or proteins. In the context of this invention targeted mutations of a human protein still render it human, whereas hybridizing parts of a human protein with parts of a homolog protein of another species render it non-human.

Hybrid STING: As used herein the term "hybrid STING" refers to STING where a part of the protein sequence is derived from human STING and another part is derived from a homolog STING of another species.

Target STING: As used herein, a target STING is a STING, which is responsive to a STING agonist which recognizes said target STING but not the human STING and natural variants thereof. Hereby, the responsiveness to the STING agonist is determined in an *"in vitro-*DMXAA determination assay". Non-limiting examples for a target STING is murine STING or the mutated human STING hSTING[R]-S162A/G230I/Q266I.

STING-responsive promoter: As used herein, the term "STING-responsive promoter" refers to a promoter that is activated by transcription factors of the STING signalling pathway such as interferon regulator factor 3 (IRF3) or nuclear factor kappa-B (NPκB). Examples for STING-promoters are the interferon-stimulated response element (ISRE) or the κB DNA response elements.

Exogenous/exogenic: The terms "exogenous" or "exogenic" when used in relation to a protein, gene, nucleic acid, or polynucleotide in a cell or organism refers to a protein, gene, nucleic acid, or polynucleotide that has been introduced into the cell or organism by artificial or natural means; or in relation to a cell, the term refers to a cell that was isolated and subsequently introduced to other cells or to an organism by artificial or natural means. An exogenous nucleic acid may be from a different organism or cell, or it may be one or more additional copies of a nucleic acid that occurs naturally within the organism or cell. An exogenous cell may be from a different organism, or it may be from the same organism. By way of a non-limiting example, an exogenous nucleic acid is one that is in a chromosomal location different from where it would be in natural cells, or is otherwise flanked by a different nucleic acid sequence than that found in nature.

Plasmid: A "plasmid," a common type of a vector, is an extra-chromosomal DNA molecule separate from the chromosomal DNA that is capable of replicating independently of the chromosomal DNA. In certain cases, it is circular and double-stranded.

Vector: As used herein, the term "vector" refers to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA or RNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "expression vectors." One of ordinary skill in the art understands that a "viral vector", as described herein, includes viral components in addition to a transgene described herein, e.g., capsid proteins.

Expression system: By "expression system" or "expression construct" is meant one or more nucleic acid molecules that is/are capable of directing transcription. An expression system includes, at a minimum, one or more transcriptional control elements (such as promoters, enhancers or a structure functionally equivalent thereof) that direct gene expression in one or more desired cell types, tissues or organs. Additional elements, such as a transcription termination signal, may also be included. The term "expression system" and the term "recombinant expression system" therefore encompass DNA or RNA nucleic acid molecules that is/are capable of directing transcription. In the context if the present invention, the respective DNA or RNA nucleic acid molecules are also designated as "nucleic acid construct".

AAV: The term "AAV" refers to adeno-associated virus and may be used to refer to the naturally occurring wild-type virus itself or derivatives thereof. The term covers all subtypes, serotypes and pseudotypes, and both naturally occurring and recombinant forms, except where required otherwise. The AAV genome is built of single stranded DNA and comprises inverted terminal repeats (ITRs) at both ends of the DNA strand, and two open reading frames: rep and cap, encoding replication and capsid proteins, respectively. A foreign polynucleotide can replace the native rep and cap genes. AAVs can be made with a variety of different serotype capsids which have varying transduction profiles or, as used herein, "tropism" for different tissue types. As used herein, the term "serotype" refers to an AAV which is identified by and distinguished from other AAVs based on capsid protein reactivity with defined antisera, e.g., AAV1, AAV2, AAV3, AAV4, AAV5, AAV6, AAV7, AAV8, AAV9, and AAVrh10. For example, serotype AAV2 is used to refer to an AAV which contains capsid proteins encoded from the cap gene of AAV2 and a genome containing 5' and 3' ITR sequences from the same AAV2 serotype. Pseudotyped AAV refers to an AAV that contains capsid proteins from one serotype and a viral genome including 5'-3' ITRs of a second serotype. Pseudotyped rAAV would be expected to have cell surface binding properties of the capsid serotype and genetic properties consistent with the ITR serotype. Pseudotyped rAAV are produced using standard techniques described in the art.

Adenovirus: The term "adenovirus" refers to any virus in the genus Adenoviridiae including, but not limited to, human, bovine, ovine, equine, canine, porcine, murine, and simian adenovirus subgenera. Typically, an adenoviral vector is generated by introducing one or more mutations (e.g., a deletion, insertion, or substitution) into the adenoviral genome of the adenovirus so as to accommodate the insertion of a non-native nucleic acid sequence, for example, for gene transfer, into the adenovirus.

Lentivirus: As used herein, the term "lentivirus" refers to a group (or genus) of complex retroviruses. Illustrative lentiviruses include but are not limited to: HIV (human immunodeficiency virus; including HIV type 1, and HIV type 2); visna-maedi virus (VMV) virus; the caprine arthritis-encephalitis virus (CAEV); equine infectious anemia virus (EIAV); feline immunodeficiency virus (FIV); bovine immune deficiency virus (BIV); and simian immunodeficiency virus (SIV).

Viral particle: As used herein, the term "viral particle" refers to all or part of a virion. For example, the viral particle comprises a recombinant genome and may further comprise a capsid. The viral particle may be a gene therapy vector. Herein, the terms "viral particle" and "vector" are used interchangeably. For the purpose of the present application, a "gene therapy" vector is a viral particle that can be used in gene therapy, i.e. a viral particle that comprises all the required functional elements to express a transgene, such as a CFI nucleotide sequence, in a host cell after administration. Suitable viral particles of the invention include a parvovirus, a retrovirus, a lentivirus or a herpes simplex virus. The parvovirus may be an adeno-associated virus (AAV). Optionally, the viral particle is an AAV or adenoviral particle.

Host cell: As used herein, the term "host cell" refers to a cell into which exogenous DNA (recombinant or otherwise) has been introduced. Persons of skill upon reading this disclosure will understand that such terms refer not only to the particular subject cell, but also to the progeny of such a cell. Because certain modifications may occur in succeeding generations due to either mutation or environmental influences, such progeny may not, in fact, be identical to the parent cell, but are still included within the scope of the term "host cell" as used herein. In some embodiments, the cell is a human, monkey, ape, hamster, rat, or mouse cell. In some embodiments, the cell is eukaryotic and is selected from the following cells: CHO (e.g., CHO K1, DXB-11 CHO, Veggie-CHO), COS (e.g., COS-7), retinal cell, Vero, CV1, kidney (e.g., HEK293, 293 EBNA, MSR 293, MDCK, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3 A cell, HT1080 cell, MC38 cell, myeloma cell, tumor cell, and a cell line derived from an aforementioned cell. In some embodiments, the cell comprises one or more viral genes.

Animal: As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (e.g., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, and/or worms. In some embodiments, an animal may be a transgenic animal, a genetically engineered animal, and/or a clone.

Treatment/treating: "Treatment" or " treating" includes (1) inhibiting a disease, disorder or condition in a subject or patient experiencing or displaying the pathology or symptomatology of the disease (e.g., arresting further development of the pathology and/or symptomatology), (2) ameliorating a disease, disorder or condition in a subject or patient that is experiencing or displaying the pathology or symptomatology of the disease (e.g., reversing the pathology and/or symptomatology), and/or (3) effecting any measurable decrease in a disease, disorder or condition in a subject or patient that is experiencing or displaying the pathology or symptomatology of the disease.

Prophylactically treating: "Prophylactically treating" includes: (1) reducing or mitigating the risk of developing the disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

Local administration/local delivery: As used herein, the term "local administration" or "local delivery", in reference to delivery of a recombinant expression system, an expression vector, a viral particle or a pharmaceutical composition described herein, refers to delivery that does not rely upon transport of said products to its intended target tissue or site via the vascular system. Said products described herein may be delivered directly to its intended target tissue or site, or in the vicinity thereof, e.g., in close proximity to the intended target tissue or site. For example, said products may be delivered by injection or implantation of the composition or compound or by injection or implantation of a device containing the composition or compound. Following local administration into a target tissue or in the vicinity of a target tissue or site, said products described herein, or one or more components thereof, may diffuse to the intended target tissue or site. It will be understood that once having been locally delivered a fraction of said products described herein (typically only a minor fraction of the administered dose) may enter the vascular system and be transported to another location, including back to its intended target tissue or site. As used herein, the term "local administration" or "local delivery", in reference to delivery of a viral particle or a pharmaceutical composition described herein, refers to delivery that can rely upon transport of the viral vector or the pharmaceutical composition to its intended target tissue or site via the vascular system.

Concurrent administration: As used herein, the term "Concurrent administration" with respect to two or more compounds, e.g., therapeutic agents, is administration performed using doses and time intervals such that the administered compounds are present together within the body, e.g., at one or more sites of action in the body, over a time interval in non-negligible quantities. The time interval can be minutes (e.g., at least 1 minute, 1-30 minutes, 30-60 minutes), hours (e.g., at least 1 hour, 1-2 hours, 2-6 hours, 6-12 hours, 12-24 hours), days (e.g., at least 1 day, 1-2 days, 2-4 days, 4-7 days, etc.), weeks (e.g., at least 1, 2, or 3 weeks, etc.). Accordingly, the compounds may, but need not be, administered together as part of a single composition. In addition, the compounds may, but need not be, administered essentially simultaneously (e.g., within less than 5 minutes, or within less than 1 minute apart) or within a short time of one another (e.g., less than 1 hour, less than 30 minutes, less than 10 minutes, approximately 5 minutes apart). According to various embodiments of the disclosure, compounds administered within such time intervals may be considered to be administered at substantially the same time. In certain embodiments of the disclosure, concurrently administered compounds are present at effective concentrations within the body (e.g., in the blood and/or at a site of local complement activation) over the time interval. When administered concurrently, the effective concentration of each of the compounds needed to elicit a particular biological response may be less than the effective concentration of each compound when administered alone, thereby allowing a reduction in the dose of one or more of the compounds relative to the dose that would be needed if the compound was administered as a single compound. The effects of multiple compounds may, but need not be, additive or synergistic. The compounds may be administered multiple times. The non-negligible concentration of a compound may be, for example, less than approximately 5% of the concentration that would be required to elicit a particular biological response, e.g., a desired biological response.

Sequential administration: As used herein, the term "sequential administration" of two or more compounds refers to administration of two or more compounds to a subject such that the compounds are not present together in the subject's body, or at a relevant site of activity in the body, at greater than non-negligible concentrations. Administration of the compounds may, but need not, alternate. Each compound may be administered multiple times.

Systemic administration: As used herein, the term "systemic administration" and like terms are used herein consistently with their usage in the art to refer to administration of a compound (e.g., a therapeutic agent, expression vector, viral particle, pharmaceutical composition, and/or formulation) such that the compound becomes widely distributed in the body in significant amounts and has a biological effect, e.g., its desired effect, in the blood and/or reaches its desired site of action via the vascular system. Typical systemic routes of administration include administration by (i) introducing the compound directly into the vascular system or (ii) subcutaneous, oral, pulmonary, or intramuscular administration wherein the compound is absorbed, enters the vascular system, and is carried to one or more desired site(s) of action via the blood.

Intratumor administration: The term "intratumor administration" refers to the administration of a compound (e.g., a therapeutic agent, expression vector, viral particle, pharmaceutical composition, and/or formulation) such that the compound is present within the tumor after administration. Those of ordinary skill in the art will appreciate that the administration thereby can occur directly within the tumor or in close proximity to the tumor.

Subject: As used herein, the terms "subject", "test subject" or "patient" refer to any organism to which a provided compound or composition is administered in accordance with the present invention e.g., for experimental, diagnostic, prophylactic, and/or therapeutic purposes. Typical subjects include animals (e.g., mammals such as mice, rats, rabbits, non-human primates, and humans; insects; worms; etc.) and plants. In some embodiments, a subject may be suffering from, and/or susceptible to a disease, disorder, and/or condition.

Effective/therapeutically effective amount: As used herein, the term "effective" or "therapeutically effective amount" means at least the minimum amount of a compound (e.g., a therapeutic agent, expression vector, viral particle, pharmaceutical composition, and/or formulation) that elicits a desired biological response when administered as part of a therapeutic regimen. In some embodiments, a therapeutically effective amount of a substance is an amount that is sufficient, when administered to a subject suffering from or susceptible to a disease, disorder, and/or condition, to treat, diagnose, prevent, and/or delay the onset of the disease, disorder, and/or condition. As will be appreciated by those of ordinary skill in this art, the effective amount of a substance may vary depending on such factors as the desired biological endpoint, the substance to be delivered, the target cell or tissue, etc. For example, the effective amount of compound in a formulation to treat a disease, disorder, and/or condition is the amount that alleviates, ameliorates, relieves, inhibits, prevents, delays onset of, reduces severity of and/or reduces incidence of one or more symptoms or signs of the disease, disorder, and/or condition. In some embodiments, a therapeutically effective amount is administered in a single dose; in some embodiments, multiple unit doses are required to deliver a therapeutically effective amount. A therapeutically effective amount is also one in which any toxic or detrimental effects of the treatment are outweighed by the therapeutically beneficial effects. In the case of cancer or tumor, an effective amount of a compound may have the effect in reducing the number of cancer cells; reducing the tumor size; inhibiting (i.e., slow to some extent or desirably stop) cancer cell infiltration into peripheral organs; inhibit (i.e. , slow to some extent and desirably stop) tumor metastasis; inhibiting to some extent tumor growth; and/or relieving to some extent one or more of the symptoms associated with the disorder.

Combination of expression vectors/combination of viral particles: By the phrase "combination of expression vectors" or combination of viral particles" is meant herein that the first expression vector or the first viral particle is administered to the patient before, during (including concurrently with - preferably co-formulated with) and/or after treatment of an individual with the second expression vector or second viral particle, respectively. The combined expression vectors or viral particles may be used for simultaneous, sequential, or separate administration. In all cases, it is preferred that any of the herein-mentioned expression vectors and viral particles are administered in pharmaceutically effective amounts, i.e. an administration involving a total amount of each component of the combination that is sufficient to show a meaningful patient benefit. The formulations may conveniently be presented in unit dosage form by methods known to those skilled in the art.

Kit of parts: A "kit of parts" as used herein provides a combination of the first and the second expression vector or a combination of the first and the second viral particle for administration in combination. It is preferred that the kit may for example contain the vectors/particles in dosage forms for administration. A dosage form contains a sufficient amount of one or more of the vectors/particles such that a desirable effect can be obtained when administered to a subject. Thus, it is preferred that the medical packaging comprises an amount of dosage units corresponding to the relevant dosage regimen. Preferably, the kit-of-parts contains instructions indicating the use of the dosage form to achieve a desirable affect and the amount of dosage form to be taken over a specified time period.

Combination therapy: The term "combination therapy", as used herein, refers to those situations in which two or more different compounds (e.g., a therapeutic agent, expression vector, viral particle, pharmaceutical composition, and/or formulation) are administered in overlapping regimens so that the subject is simultaneously exposed to both compounds. When used in combination therapy, two or more different compounds may be administered simultaneously or separately. This administration in combination can include simultaneous administration of the two or more compounds in the same dosage form, simultaneous administration in separate dosage forms, and separate administration. That is, two or more compounds can be formulated together in the same dosage form and administered simultaneously. Alternatively, two or more compounds can be simultaneously administered, wherein the compounds are present in separate formulations. In another alternative, a first compound can be administered followed by one or more additional compounds. In the separate administration protocol, two or more compounds may be administered a few minutes apart, or a few hours apart, a few days apart, or a few weeks apart. In some embodiments, two or more compounds may be administered 1-2 weeks apart.

Radiotherapy: The term "radiotherapy" refers hereinafter to the medical use of ionizing radiation, generally as part of cancer treatment to control or kill malignant cells. The ionizing radiation might be based on X-rays, gamma-rays or particle rays. It may also be used as part of adjuvant therapy, to prevent tumor recurrence after surgery that removes a primary malignant tumor. Radiation therapy may be synergistic with other anti-cancer therapies, and may be used before, during, and after other anti-cancer therapies in susceptible cancers. The term "low-dose radiotherapy" refers hereinafter to radiotherapies with an ionizing radiation dose of less than 40 Gy, preferably less than 8 Gy. Low-dose radiotherapy can be any radiotherapy with an ionizing radiation dose of less than 40 Gy, 38 Gy, 36 Gy, 34 Gy, 32 Gy, 30 Gy, 28 Gy, 26 Gy, 24 Gy, 22Gy, 20 Gy, 19 Gy, 18 Gy, 17 Gy, 16 Gy, 15 Gy, 14 Gy, 13 Gy, 12 Gy, 11 Gy, 10 Gy, 9 Gy, 8 Gy, 7 Gy, 6 Gy, 5 Gy, 4 Gy, 3 Gy, 2.5 Gy, 2.0 Gy, 1.5 Gy, 1 Gy, 0.5 Gy, 0.25 Gy, 0.2 Gy, 0.15 Gy, 0.1 Gy, 0.05 Gy, or 0.01 Gy.

Proliferative disease: As used herein, the term "proliferative disease" refers to conditions in which unregulated or abnormal growth, or both, of cells can lead to the development of an unwanted condition or disease, which may or may not be cancerous. Exemplary cell proliferative disorders of the disclosure encompass a variety of conditions wherein cell division is deregulated. Exemplary cell proliferative disorder include but are not limited to, neoplasms, benign tumors, malignant tumors, pre-cancerous conditions, in situ tumors, encapsulated tumors, metastatic tumors, liquid tumors, solid tumors, immunological tumors, hematological tumors, cancers, carcinomas, leukemias, lymphomas, sarcomas, and rapidly dividing cells

Cancer/tumor: The terms "cancer" or "tumor" refer to the presence of cells possessing characteristics typical of cancer-causing cells, such as uncontrolled proliferation, immortality, invasive or metastatic potential, rapid growth, and certain characteristic morphological features. In some embodiments, such cells exhibit such characteristics in part or in full due to the expression and activity of immune checkpoint proteins, such as PD-1, PD-L1, PD-L2, and/or CTLA-4. Cancer cells are often in the form of a tumor, but such cells may exist alone within an animal, or may be a non-tumorigenic cancer cell, such as a leukemia cell. As used herein, the term "cancer" includes premalignant as well as malignant cancers. Cancers include, but are not limited to, a variety of cancers, carcinoma including that of the bladder (including accelerated and metastatic bladder cancer), breast, colon (including colorectal cancer), kidney, liver, lung (including small and non-small cell lung cancer and lung adenocarcinoma), ovary, prostate, testes, genitourinary tract, lymphatic system, rectum, larynx, pancreas (including exocrine pancreatic carcinoma), esophagus, stomach, gall bladder, cervix, thyroid, and skin (including squamous cell carcinoma); hematopoietic tumors of lymphoid lineage including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, histiocytic lymphoma, and Burkitt's lymphoma; hematopoietic tumors of myeloid lineage including acute and chronic myelogenous leukemias, myelodysplastic syndrome, myeloid leukemia, and promyelocytic leukemia; tumors of the central and peripheral nervous system including astrocytoma, neuroblastoma, glioma, and schwannomas; tumors of mesenchymal origin including fibrosarcoma, rhabdomyosarcoma, and osteosarcoma; other tumors including melanoma, xenoderma pigmentosum, keratoactanthoma, seminoma, thyroid follicular cancer, and teratocarcinoma; melanoma, unresectable stage III or IV malignant melanoma, squamous cell carcinoma, small -cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatoma, breast cancer, colon carcinoma, and head and neck cancer, gastric cancer, germ cell tumor, bone cancer, bone tumors, adult malignant fibrous histiocytoma of bone; childhood, malignant fibrous histiocytoma of bone, sarcoma, pediatric sarcoma, sinonasal natural killer, neoplasms, plasma cell neoplasm; myelodysplastic syndromes; neuroblastoma; testicular germ cell tumor, intraocular melanoma, myelodysplastic syndromes; myelodysplastic/myeloproliferative diseases, synovial sarcoma, chronic myeloid leukemia, acute lymphoblastic leukemia, Philadelphia chromosome positive acute lymphoblastic leukemia (Ph+ ALL), multiple myeloma, acute myelogenous leukemia, chronic lymphocytic leukemia, mastocytosis and any symptom associated with mastocytosis, and any metastasis thereof. In addition, disorders include urticaria pigmentosa, mastocytosises such as diffuse cutaneous mastocytosis, solitary mastocytoma in human, and some rare subtypes like bullous, erythrodermic and teleangiectatic mastocytosis, mastocytosis with an associated hematological disorder, such as a myeloproliferative or myelodysplastic syndrome, or acute leukemia, myeloproliferative disorder associated with mastocytosis, mast cell leukemia, in addition to other cancers. Other cancers are also included within the scope of disorders including, but are not limited to, the following: carcinoma, including that of the bladder, urothelial carcinoma, breast, colon, kidney, liver, lung, ovary, pancreas, stomach, cervix, thyroid, testis, particularly testicular seminomas, and skin; including squamous cell carcinoma; gastrointestinal stromal tumors ("GIST"); hematopoietic tumors of lymphoid lineage, including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma and Burkitt's lymphoma; hematopoietic tumors of myeloid lineage, including acute and chronic myelogenous leukemias and promyelocytic leukemia; tumors of mesenchymal origin, including fibrosarcoma and rhabdomyosarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyosarcoma, and osteosarcoma; and other tumors, including melanoma, xenoderma pigmentosum, keratoactanthoma, seminoma, thyroid follicular cancer, teratocarcinoma, chemotherapy refractory non-seminomatous germ-cell tumors, and Kaposi's sarcoma, and any metastasis thereof. Other non-limiting examples of types of cancers applicable to the methods encompassed by the present invention include human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, bone cancer, brain tumor, lung carcinoma (including lung adenocarcinoma), small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments, cancers are epithelial in nature and include but are not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer.

Anti-cancer: An "anti-cancer" agent is capable of negatively affecting a cancer cell/tumor in a subject, for example, by promoting killing of cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer.

Pharmaceutical composition: The term "pharmaceutical composition" refers to a preparation which is in such form as to permit the biological activity of the active ingredient to be effective, and which contains no additional components which are unacceptably toxic to a subject to which the formulation would be administered. Such formulations are sterile. "Pharmaceutically acceptable" excipients (vehicles, additives) are those which can reasonably be administered to a subject mammal to provide an effective dose of the active ingredient employed.

Synergism/synergistic effect: The terms "synergism" or "synergistic effect" refer to the combined effect of two or more compounds that is greater than the sum of the separate effects of the cancer compounds/therapies alone.

Abscopal effect/direct treatment: As used herein, the term "direct treatment" refers to the administration of a compound, an expression system, an expression vector, a viral vector, a viral particle, a pharmaceutical composition, or any therapy directly to a targeted location. For example, direct treatment of a tumor according to the invention refers to an administration that targets said tumor but no other tumors or metastases. As used herein, the term "abscopal effect" describes the size reduction of a tumor by a direct treatment of another tumor. More explicit, the direct treatment of a tumor may cause the organism to also target another tumor that has not been contacted with a compound, an expression system, an expression vector, a viral vector, a viral particle, a pharmaceutical composition, or any therapy.

*in vitro*-agonist determination assay/*in vitro*-DMXAA determination assay: The term "*in vitro*-agonist determination assay" refers to assays that allow the identification of human-non-reacting STING agonists in that said agonists activate variants of human STING or non-human STING but not human wild-type STING or natural variants thereof. The term *"in vitro*-DMXAA determination assay" refers to assay that allow to identify STINGs that are responsive to DMXAA.

Standard techniques may be used for recombinant DNA, oligonucleotide synthesis, and tissue culture and transformation (e.g., electroporation, lipofection). Enzymatic reactions and purification techniques may be performed according to manufacturer's specifications or as commonly accomplished in the art or as described herein. The foregoing techniques and procedures may be generally performed according to conventional methods well known in the art and as described in various general and more specific references that are cited and discussed throughout the present specification. See e.g., Sambrook et al., Molecular Cloning: A Laboratory Manual (2d ed., Cold Spring Harbor Laboratory Press, Cold Spring Harbor, N.Y. (1989)), which is incorporated herein by reference for any purpose.

### Brief description of the drawings

**Figure 1****,** comprising Figures A - G, illustrate A) IFN-β expression of MC38 cells upon lentiviral transduction with either hSTINGwt or hSTINGmut and subsequent DMXAA treatment relative to vehicle treated cells; data shown are mean ± SEM (n= 3, ^{∗∗} P< 0.01, two-way ANOVA followed by Sidak's multiple comparison test); B) IFN-β expression of Hep2G cells upon lentiviral transduction with either hSTINGwt or hSTINGmut and subsequent DMXAA treatment relative to vehicle treated cells; data shown are mean ± SEM (n= 3, ^{∗∗} P< 0.01, two-way ANOVA followed by Sidak's multiple comparison test); C) IFN-β expression of Hep2G cells upon lentiviral transduction with mSTINGwt and subsequent DMXAA treatment relative to vehicle treated cells; data shown are mean ± SEM (n= 9, ^{∗∗} P< 0.01, two-way ANOVA followed by Sidak's multiple comparison test); D) STING-derived cytokine expression of MC38 cells upon lentiviral transduction with either luciferase, hSTINGwt or hSTINGmut, or no transduction, and subsequent DMXAA treatment relative to vehicle treated cells; data shown are mean ± SEM (n= 3, ^{∗} P< 0,05, ^{∗∗} P< 0.01, one sample at a time by one sample T test); E) STING-derived cytokine expression of Hep2G cells upon lentiviral transduction with either luciferase, hSTINGwt or hSTINGmut, or no transduction, and subsequent DMXAA treatment relative to vehicle treated cells; data shown are mean ± SEM (n= 3, ^{∗} P< 0,05, ^{∗∗} P< 0.01, one sample at a time by one sample T test); F) time-course of STING-derived cytokine expression of MC38 cells upon lentiviral transduction with hSTINGmut and subsequent DMXAA treatment relative to vehicle treated cells; data shown are mean ± SEM (n= 3, ^{∗} P< 0,05, ^{∗∗} P< 0.01, ^{∗∗∗} P<0,001, two-way ANOVA followed by Fisher LSD test); and G) time-course of STING-derived cytokine expression of MC38 cells upon lentiviral transduction with hSTINGmut and subsequent DMXAA treatment relative to vehicle treated cells; data shown are mean ± SEM (n= 3, ^{∗} P< 0,05, ^{∗∗} P< 0.01, ^{∗∗∗} P<0,001, two-way ANOVA followed by Fisher LSD test).

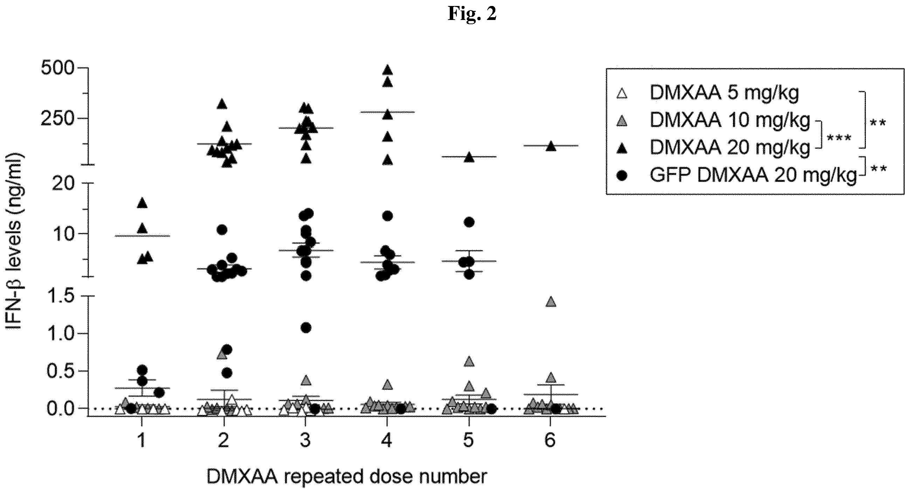

**Figure 2** illustrates IFN-β serum levels of mice injected intravenously with an AAV8 vector encoding either mSTING or GFP and repeatedly treated with DMXAA at different doses (5, 10 or 20 mg/kg bodyweight) twice a week. Data shown are mean ± SEM (n= 4-12 mice per group) ^{∗∗} P< 0.01, ^{∗∗∗} P< 0.001, One-way ANOVA followed by Dunnett's multiple comparisons test.

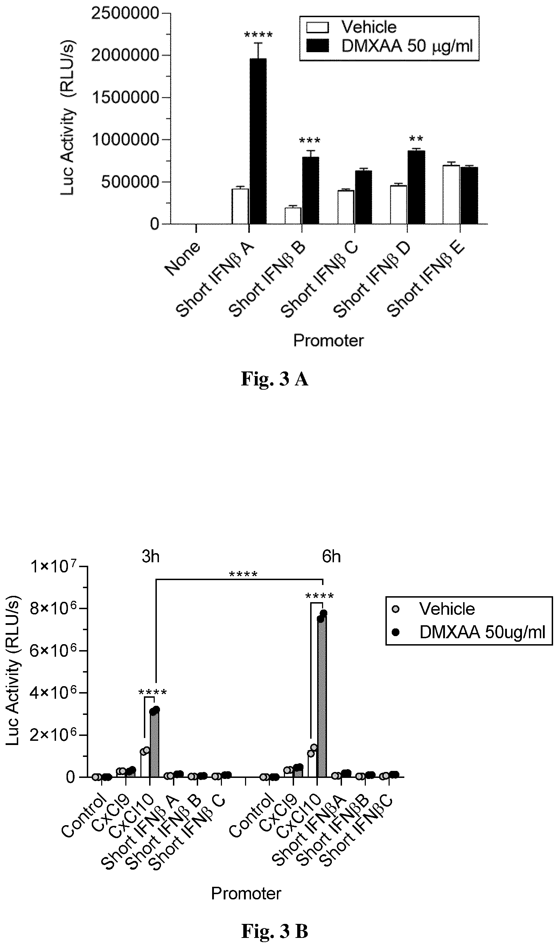

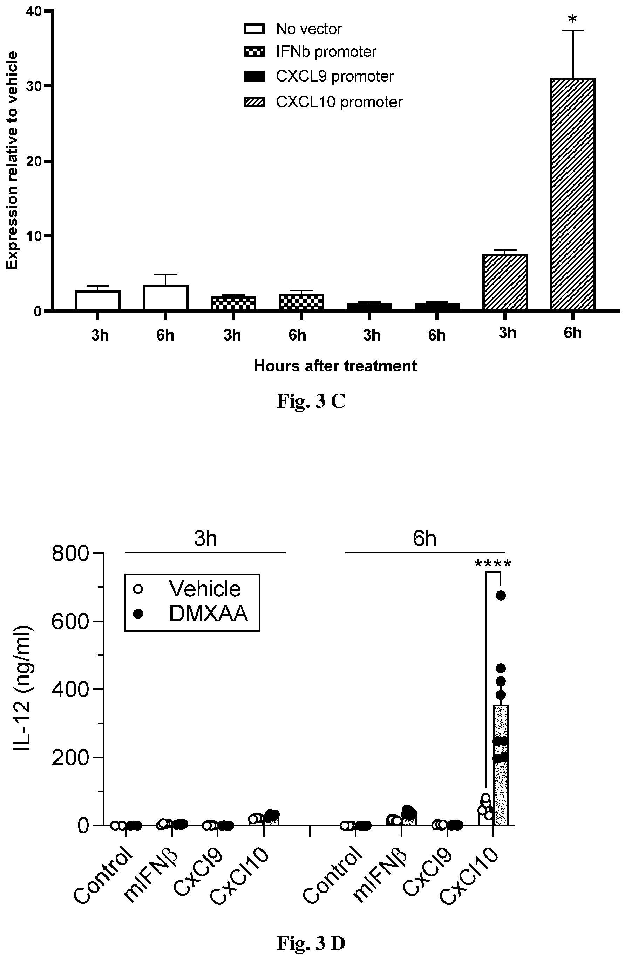

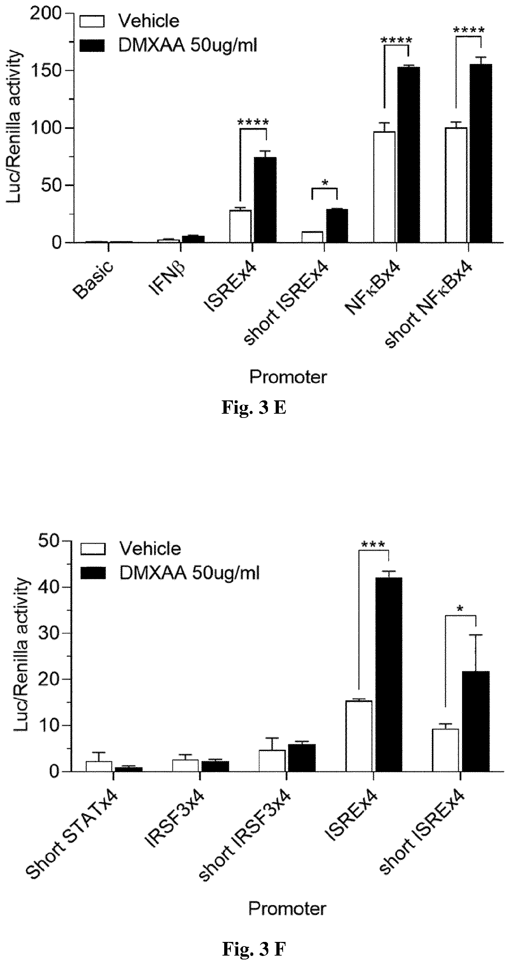

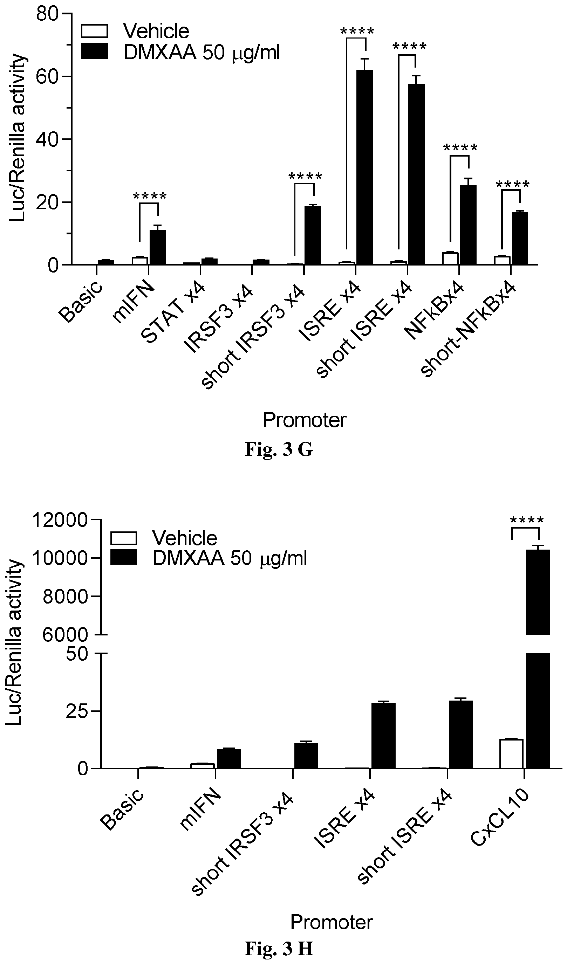

**Figure 3**, comprising Figures A - H, illustrate A) luciferase activity of MC38 cells that were lentivirally transduced with vectors encoding luciferase under the control of different modified IPN-β-promoters in the absence or presence of DMXAA; data are mean ± SEM (n=2 wells per condition) ^{∗∗} p< 0.01, ^{∗∗∗} P< 0.001, ^{∗∗∗∗} P< 0.0001, Two-way ANOVA followed by Sidak's multiple comparison test (Vehicle - DMXAA); B) luciferase activity of MC38 cells that were lentivirally transduced with vectors encoding luciferase under the control of different chemokine-promoters in the absence or presence of DMXAA; data are mean ± SEM (n=2 wells per condition) ^{∗∗∗∗} P< 0.0001, Two-way ANOVA followed by Tukey's multiple comparison test; C) IL-12 mRNA expression in MC38 cells that were lentivirally transduced with vectors encoding IL-12 under the control of either a IFN-β, a CXCL9- or a CXCL10-promoter at different timepoints after DMXAA treatment; data shown are mean ± SEM (n=3, ^{∗} P< 0.05, two-way ANOVA followed by Kruskal-Wallis test; D) IL-12 protein levels in the supernatant of MC38 cells that were lentivirally transduced with vectors encoding IL-12 under the control of either a IFN-β, a CXCL9- or a CXCL10-promoter at different timepoints after DMXAA treatment; data are mean ± SEM (n= 2 - 8 wells/ condition), ^{∗∗∗∗} P< 0.0001, Two-way ANOVA followed by Sidak's multiple comparison test; E) and F) luciferase activity of MC38 cells that were transfected with plasmids encoding luciferase under the control of different synthetic promoters in the absence or presence of DMXAA; data are mean ± SEM (n=2 wells per condition), ^{∗} P< 0.05, ^{∗∗∗} P< 0.001, ^{∗∗∗∗} P< 0.0001, Two-way ANOVA followed by Sidak's multiple comparisons test; and G) and H) luciferase activity of HEK293T cells that were transfected with plasmids encoding luciferase under the control of different synthetic promoters in the absence or presence of DMXAA; data are mean ± SEM (n=2 wells per condition), ^{∗∗∗∗} P< 0.0001, Two-way ANOVA followed by Sidak's multiple comparisons test.

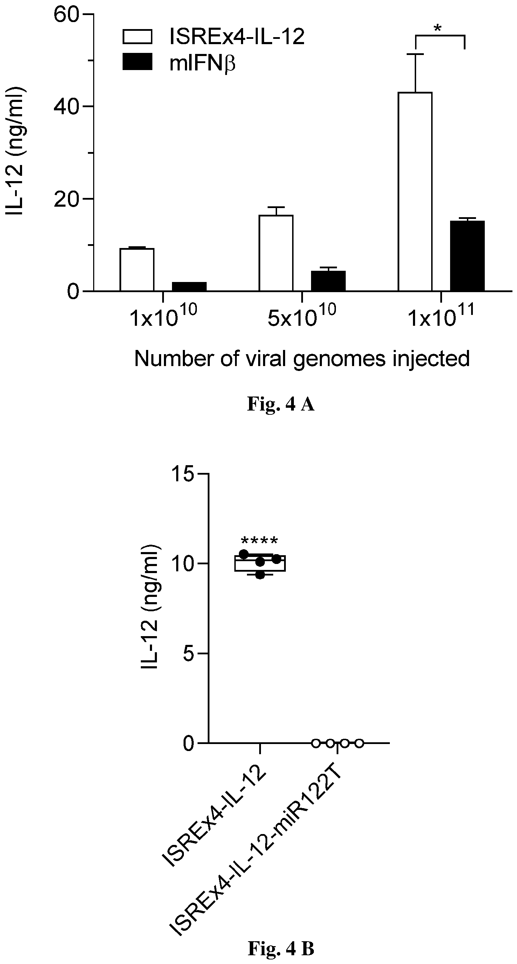

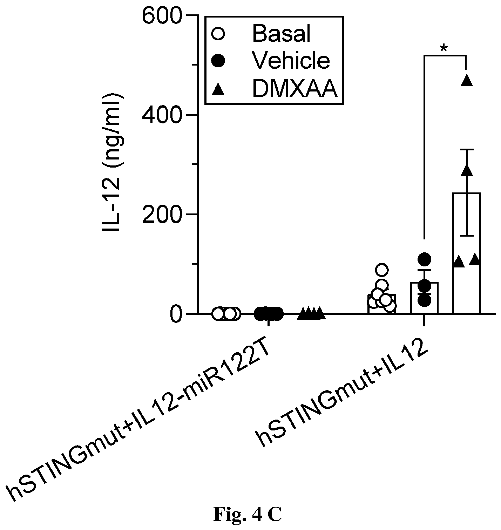

**Figure 4****,** comprising Figures A - C, illustrate A) IL-12 protein levels in the serum of mice upon intravenous injection with an AAV8 encoding IL-12 under the control of either a IFN-β-promoter or a ISRE-promoter in the absence of DMXAA; data are mean ± SEM (n=2-3 animals per condition), ^{∗} P< 0.05 Two-way ANOVA followed by Tukey's multiple comparisons test; B) reduced IL-12 protein levels in the serum of mice upon intravenous injection with an AAV8 encoding single chain (sc)IL-12 operably linked to miR122 binding sites under the control of a ISRE-promoter compared to the said AAV8 vector lacking miR122 binding sites, in the absence of DMXAA; data are mean ± SEM (n=4 animals per condition), ^{∗∗∗∗} P<0.0001, Student t test; and C) reduced IL-12 protein levels in the serum of mice upon intravenous injection with an AAV8 encoding hSTINGmut under a constitutive active promoter and an AAV8 encoding scIL-12 operably linked to miR122 binding sites under the control of a ISRE-promoter or said AAV8 without the miR122 binding sites in the presence or absence of DMXAA; data are mean ± SEM (n=4-8 animals per condition), ^{∗} P< 0.05 Two-way ANOVA followed by Tukey's multiple comparisons test.

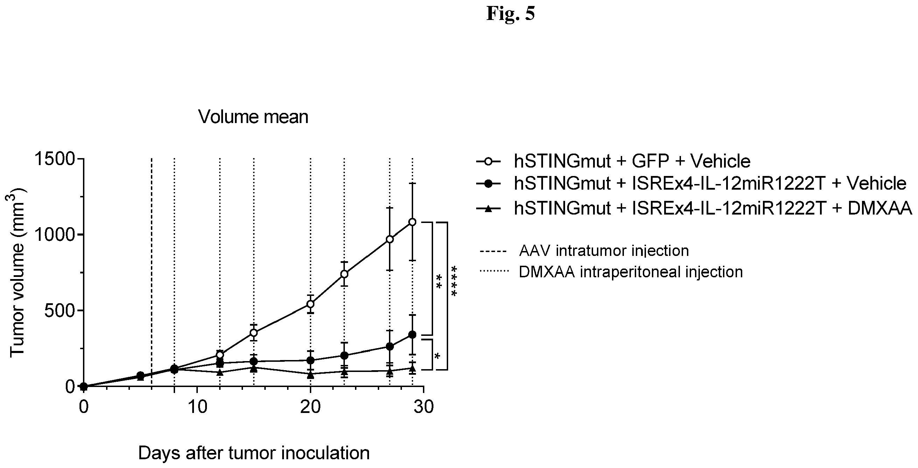

**Figure 5** illustrates the volume of subcutaneous MC38 tumors implanted in STING KO mice which were treated by intratumor co-injection of an AAV8 vector encoding hSTINGmut under the control of a constitutive promoter (AAV8-hSTINGmut) at the dose of 1×10¹¹ vg/mouse plus an AAV8 vector encoding scIL-12 operatively linked to miR122 binding sites and placed under the control of a ISRE promoter (AAV8-ISREx4IL12miR122T) at the dose of 0.5×10¹¹ vg/mouse. These animals received at the indicated time intervals either DMXAA (solid triangles) or vehicle (solid circles). In an additional control group, the tumor was injected with AAV8-hSTINGmut (1×10¹¹ vg/mouse) plus an AAV8 vector encoding GFP (0.5×10¹¹ vg/mouse) followed by vehicle (empty circles). Data are mean ± SEM (n=4-6 animals per condition) ^{∗}P <0.05, ^{∗∗} P< 0.01, ^{∗∗∗∗} P<0.0001 Mixed-effects analysis followed by Tukey's multiple comparisons test.

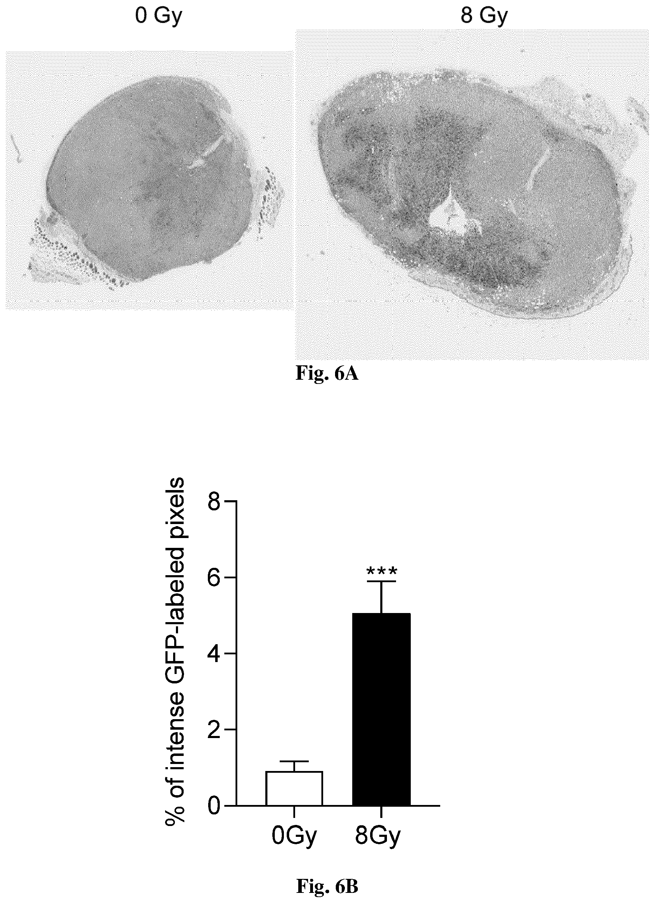

**Figure 6****,** comprising Figures 6A and 6B, illustrate A) sections of subcutaneous MC38 tumor immunohistochemically stained with an anti-GFP antibody 3 days after AAV8-GFP intra-tumor injections. The transduction efficiency of AAV8-GFP injected tumors without radiation pre-treatment (left) is markedly reduced compared to tumors with 8 Gy radiation pre-treatment (right).B) The graph shows percentage of strongly GFP-labelled pixels related to the tumor area. Data are mean ± SEM (n=3-6 animals per condition) ^{∗∗∗} P< 0.001 Unpaired Student's t test

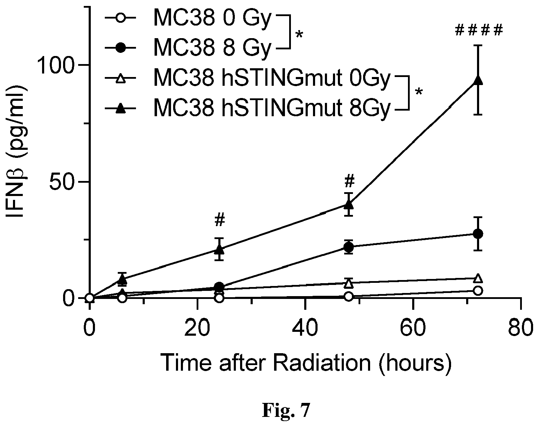

**Figure 7** illustrates IFN-β protein levels in the supernatant of MC38 cells which either received radiation and/or transduction with a hSTINGmut encoding vector quantified at indicated time points after irradiation; data are mean ± SEM (n=2-6 samples per condition), ^{∗} P< 0.05 Mixed-effects followed by Tukey's multiple comparisons test (simple effect). # P<0.05, #### P<0.0001 vs MC38 8Gy, Two way ANOVA followed by Tukey's multiple comparisons test (comparing values in a given time point).

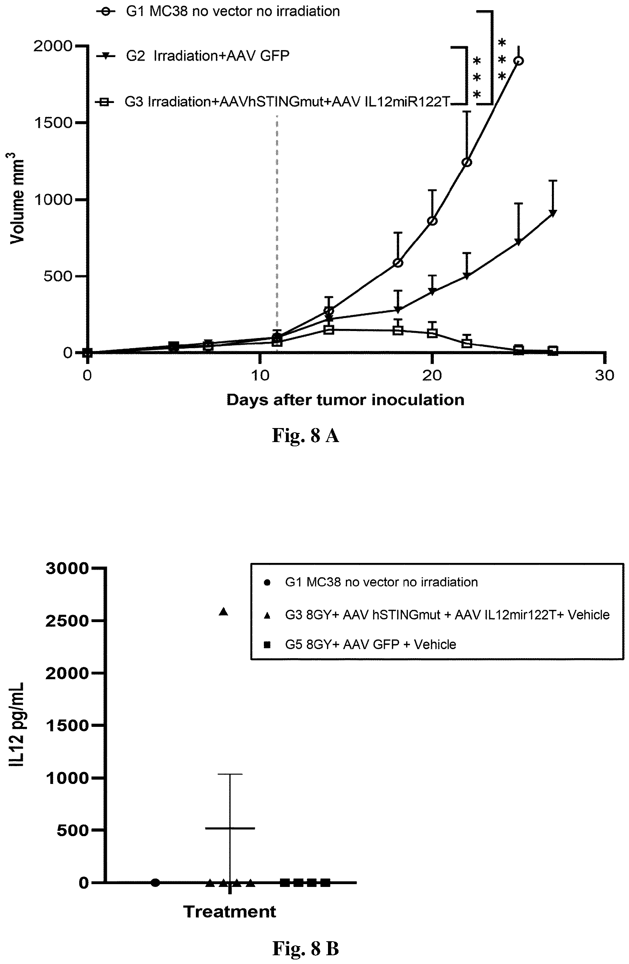

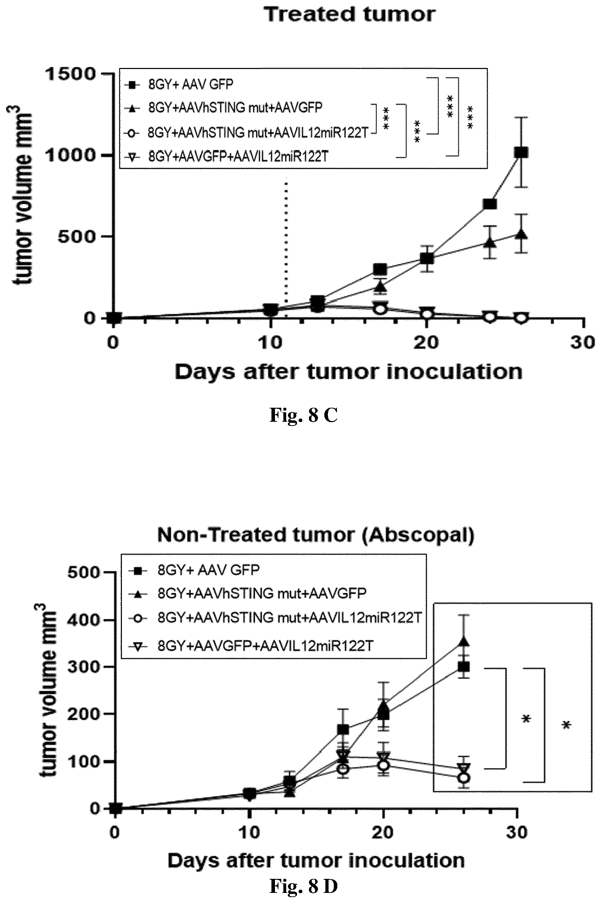

**Figure 8****,** comprising Figures A - D, illustrate A) the time course of tumor volume progression in mm³ of MC38 subcutaneous tumors in C57BL/6 wild-type mice after either radiation or no radiation followed by intratumor injection of either AAV8-GFP, AAV8-hSTINGmut and/or AAV8-ISREx4-IL12miR122T as indicated. Data are mean ± SEM (n=4-5 mice per group), A two-way analysis of variance (ANOVA) test with Tukey's post-test analysis were used to assess significance. Simple main effects analysis showed that treatment has a statistically significant effect on tumor growth (^{∗∗∗}p<0.001). B) The serum levels of IL-12 measured by ELISA on day 17 after treatment of the animals in A). Dots show individual values of each mouse, the middle horizontal line represents the median, and the error bars indicate the standard error of the mean (SEM). There were no statistically significant differences between groups as determined by one-way ANOVA (F (2, 7) =0.4375, p = 0 .6622). C) and D) refer to C57BL/6 wild-type mice with two subcutaneous MC38 tumors, one on the right flank and the other on the left flank; the former received treatment while the contralateral tumor was left untreated in order to analyse for abscopal effects. Right flank tumor was treated by local irradiation with 8 Gy followed by intratumor injection of either AAV8-GFP, AAV8-hSTINGmut and/or AAV8-ISREx4-IL12miR122T as indicated. C) shows the growth of the treated tumor and D) shows the growth of untreated tumor. Data are mean ± SEM (n=6 mice per group). A two-way analysis of variance (ANOVA) test with Tukey's post-test analysis was used to assess significance. Simple main effects analysis showed that treatment has a statistically significant effect on the growth of the treated tumor (^{∗∗∗}p<0.001) accompanied by a statistically significant antitumor effect in the contralateral tumor at day 15 post-treatment (^{∗}p<0.01).

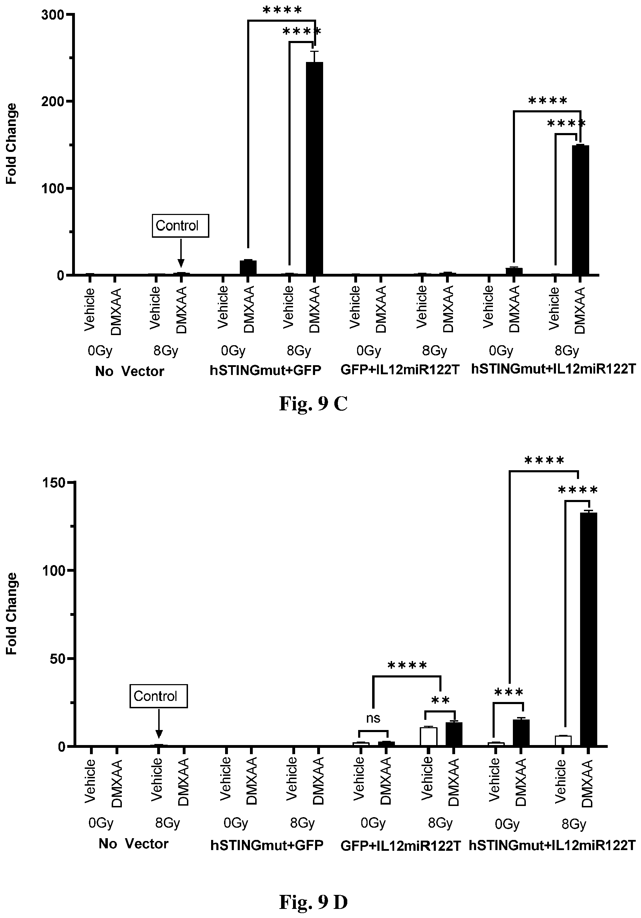

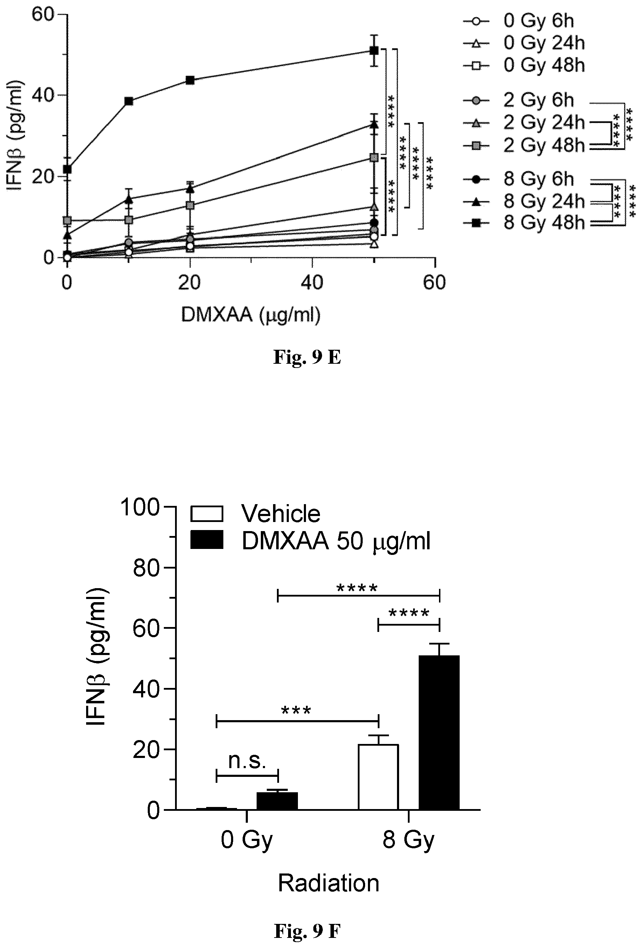

**Figure 9****,** comprising Figures A - F, illustrate A) IFN-β mRNA levels in MC38 cells upon stimulation with DMXAA or vehicle in non-transduced cells or cells transduced with AAV8-hSTINGmut after radiation with 8 Gy or no radiation (0 Gy); data shown are mean ± SEM (n= 3, ^{∗} P< 0,05, ^{∗∗} P< 0.01, one-way ANOVA followed by Kruskal-Wallis test); B) IL-12 mRNA levels in MC38 cells upon stimulation with DMXAA or vehicle in cells transduced with AAV8-hSTINGmut plus AAV8-ISREx4-IL12miR122T after radiation with 8 Gy or no radiation (0 Gy); data shown are mean ± SEM (n= 3, ^{∗∗∗} P< 0,001, one-way ANOVA followed by Kruskal-Wallis test); C) and D) IFN-β mRNA levels (in C) or IL-12 mRNA levels (in D) in HEK293 cells upon stimulation with DMXAA or vehicle in non-transduced cells, cells transduced with AAV8-hSTINGmut plus AAV8-GFP, cells transduced with AAV8-GFP plus AAV-ISREx4-IL12miR122T or cells transduced with AAV8-hSTINGmut plus AAV-ISREx4-IL12miR122T after radiation with 8 Gy or no radiation (0 Gy); data shown are mean ± SEM (n= 3, ^{∗∗}P<0.01, ^{∗∗∗}P<0,001, ^{∗∗∗∗} P< 0.0001, two-way ANOVA followed by Tukey multiple comparison test); E) IFN-β protein levels (pg/ml) obtained by ELISA in supernatants of cultured non-transduced MC38 cells treated with different doses of DMXAA (0, 5, 10, 20 and 50 µg/mL) 24 hours after radiation (0, 2 or 8 Gy) and collected 6, 24 or 48h after DMXAA administration; data are mean ± SEM (n=2-5 samples per condition), ^{∗∗∗∗} P< 0.0001, Two-way ANOVA followed by Tukey's multiple comparisons test; and F) IFN-β protein levels (pg/ml) obtained by ELISA in supernatants of cultured non-transduced MC38 cells treated with DMXAA (50 µg/mL) or vehicle 24 hours after radiation (8 Gy) or no radiation (0 Gy) and collected 24 h after DMXAA administration; data are mean ± SEM (n=3-4 samples per condition), ^{∗∗∗} P<0.001, ^{∗∗∗∗} P< 0.0001, Two-way ANOVA followed by Tukey's multiple comparisons test.

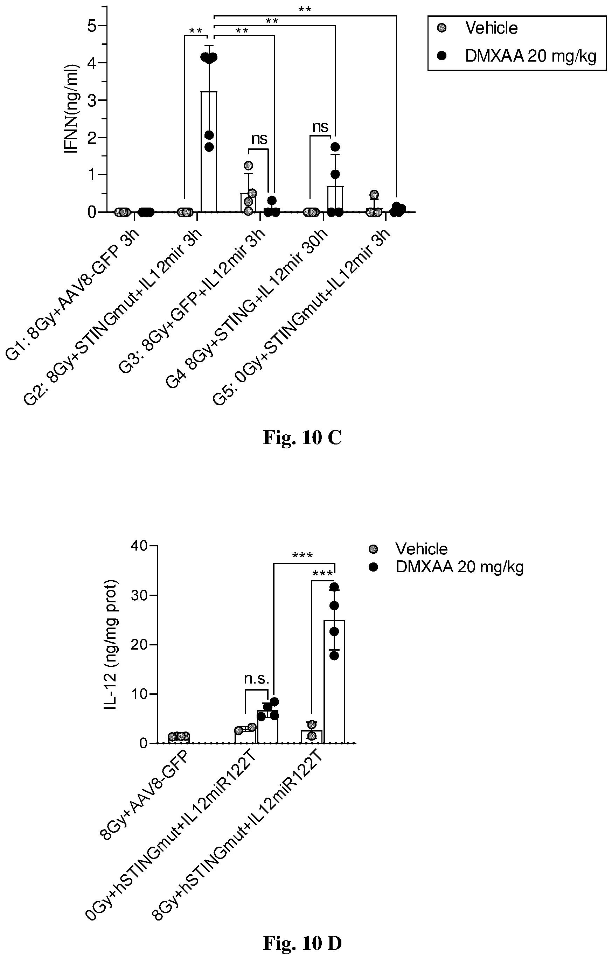

**Figure 10****,** comprising Figure A - D, show results obtained in STING KO mice with subcutaneous MC38 tumors treated with 0 or 8 Gy irradiation followed by intratumor injection with AAV vectors encoding GFP (control) or hSTINGmut and/or IL12miR122T as indicated. Two days later they received intraperitoneal DMXAA or vehicle. The figure illustrates serum levels of A) IFN-β, B) IL-12, C) IFN-γ at the indicated time points. Figure D represents IL-12 levels in tumor extracts obtained 6h after DMXAA or vehicle treatment Values were obtained by ELISA. Data are mean ± SEM (n=4-5 mice per group), ^{∗} P<0.05, ^{∗∗}P<0.01, ^{∗∗∗} P<0.001, ^{∗∗∗∗} P<0.0001, Two-way ANOVA followed by Tukey's multiple comparisons test.

### Example 1: STING response towards DMXAA in vitro

In a first set of three experiments the response of different STINGs towards DMXAA was investigated.

### Experiment 1: Dose response of DMXAA on different STINGs

The objective of this experiment was to examine which STINGs are sensitive towards the STING agonist DMXAA and whether there is a dose-dependency towards DMXAA. As a readout, the expression of the downstream target IFN-β was measured by quantitative real-time PCR (qPCR).

### Material and methods

To this end, either cells from the murine cell line MC38 or cells from the human cell line Hep2G were transduced *in vitro* by lentiviruses encoding for either wildtype murine STING (mSTINGwt), wildtype human STING (hSTINGwt) or human triple-mutant STINGS162A/G230I/Q266I (hSTINGmut). Transduced cells were sorted by flow cytometry based on expression of reporter proteins thus obtaining stable cell lines. Respective stable cells were seeded in 24-well plates at a density of 750 cells/mm² in growth medium. 24 h after seeding, the cells were incubated with different doses of DMXAA for another 3 h (MC38 cells) or 6 h (Hep2G cells). Cells were harvested and homogenized thereafter, and the mRNA extracted using the Maxwell^{®} RSC simplyRNA Tissue Kit protocol (Promega, Madrid, Spain). RNA was reverse transcribed into cDNA using M-MLV reverse transcriptase (Promega, Madrid, Spain) according to the manufacturer's instructions. A quantitative real-time PCR was performed using CFX-Connect Real-Time PCR Detection System (BioRad, Hercules, Calif) to quantify expression levels. Every analysis was performed in 10 µL total volume containing 5 µL of 1x IQ SYBR Green Supermix (Bio-Rad Life Science, Hercules, Calif.), 0.2 µL of each primer (15µM, 300nM final concentration), 2.6 µL of distilled water, and 2 µL of template cDNA. Primers annealing with each gene in the study are detailed in table 1. The expression of IFN-β was measured for either experimental group and expression levels relative to vehicle treated cells were quantified.

### Results and discussion

As shown in Fig. 1 A - C, IFN-β expression upon DMXAA treatment was elevated dose-dependently in either human or murine cells transduced with the DMXAA-sensitive STINGs hSTINGmut or mSTINGwt. In contrast no significant alteration in IFN-β expression was observed in the DMXAA-insensitive hSTINGwt. As IFN-β expression is upregulated upon STING activation this experiment shows that i) DMXAA is able to activate DMXAA-sensitive STINGs and these STINGs have biological activity of STING, and ii) the dose of DMXAA correlates with STING activity. Hence, DMXAA activates DMXAA-sensitive STINGs, but not hSTINGwt.

### Experiment 2: DMXAA mediated expression of cytokines

The objective of this experiment was to examine whether different STINGs show biological activity of STING upon DMXAA treatment. As a readout, the expression of STING-derived cytokines was measured by qPCR.

### Material and methods

To this end, MC38 and Hep2G cells have been transduced with lentiviruses encoding luciferase, hSTINGwt, hSTINGmut to obtain stable cell lines as described above or were not transduced with any lentivirus (no vector). Respective cells were seeded in 24-well plates at a density of 750 cells/mm² in growth medium. 24 h after seeding, the cells were incubated with 50 µg/mL DMXAA for another 3 h (MC38 cells) or 6 h (Hep2G cells). Cells were harvested and prepared for qPCR measurements as described above. The expression of the cytokines IFN-β, TNF-α, IL-6, CXCL9 and CXCL10 were measured for either experimental group and expression levels relative to controls (no vector) were quantified.

### Results and discussion

As shown in Fig. 1 D - E, expression of the aforementioned cytokines was elevated only in hSTINGmut-transduced cells whereas no significant alteration of expression levels was observed in any of the other groups. This furthermore corroborates the findings of the first experiment, that i) DMXAA is able to activate DMXAA-sensitive STINGs and these STINGs have biological activity of STING. In addition, this experiment shows that hSTINGmut displays the biological activity of STING in that it causes an upregulation of known STING downstream proteins (Liu et al., 2020 PNAS 117 (24)).

### Experiment 3: Time-course expression of STING-derived cytokines upon DMXAA treatment

The objective of this experiment was to examine the time-course of STING-derived cytokine expression upon DMXAA treatment. As a readout, the expression of STING-derived cytokines was measured by qPCR.

### Material and methods

To this end, stable cell lines of MC38 and Hep2G cells encoding hSTINGmut have been obtained as described above. 24 h after seeding, the cells were incubated with 50 µg/mL DMXAA for the indicated time points. Cells were harvested and prepared for qPCR measurements as described above. The expression of the cytokines IFN-β, TNF-α, IL-6, CXCL9 and CXCL10 were measured for either experimental group and expression levels relative to controls (0 h after DMXAA treatment) were quantified.

### Results and discussion

As shown in Fig. 1 F - G, while the temporal expression profiles for the different cytokines differed for each cytokine and between MC38 cells and Hep2G cells, overall, an increase in expression levels was observed upon DMXAA-treatment for any given timepoint. Thus, the expression of the investigated cytokines was upregulated DMXAA-dependently, and hence dependent on STING activity, whereas the different temporal expression patterns show that this expression is also dependent on the cell line used and the respective cytokine investigated.

### Example 2: STING response towards DMXAA in vivo

As the general sensitivity of hSTINGmut towards DMXAA was shown *in vitro,* the objective of this experiment was to examine the response of hSTINGmut towards DMXAA *in vivo.*

### Materials and methods

C57 wild type mice, aged 6 to 10 weeks, were injected intravenously with an AAV8 vector encoding either hSTINGmut (AAV8-hSTINGmut, 1×10¹¹ viral genomes) or as a control GFP (AAV8-GFP, 1.5×10¹¹ viral genomes). Two weeks after transduction mice were repeatedly (twice per week) injected intraperitoneally with different DMXAA doses (5 mg DMXAA/kg bodyweight, 10 mg DMXAA/kg body weight and 20 mg DMXAA/kg bodyweight). Three hours after injection blood was collected from each mouse and IFN-β protein levels in the serum were determined with VeriKine-HSTM (High Sensitivity) Mouse IFN-β Serum ELISA (pbl Assay Science, Piscataway NJ USA).

### Results and discussion

As shown in Fig. 2, administration of 20 mg DMXAA/kg bodyweight already caused an elevation of IFN-β levels in AAV8-GFP transduced control animals after a first injection while IFN-β levels increased even further after repeated injections. This was expected, as mice express murine STING (mSTINGwt) which is already sensitive towards DMXAA and shows IFN-β upregulation *in vitro* (s. example 1, experiment 1). Notably, IFN-β levels were more than 10-fold higher in mice transduced with AAV8-hSTINGmut at any given timepoint compared to AAV8-GFP controls. In both cases systemic STING stimulation caused high toxicity and mortality (as it can be seen by the drop in the number of remaining animals with successive DMXAA injections). However, doses of 5 or 10 mg DMXAA/kg bodyweight showed only very low levels of IFN-β in AAV8-hSTINGmut transduced mice and, moreover, did neither cause significant toxicity nor mortality. These data show that i) exogenic STING expression enhances the IFN response to STING agonists dose-dependently, ii) systemic STING activation is associated with unacceptable toxicity and iii) that the induction of IFN-β in the transduced tissue can be elicited on demand by injections of the STING agonist DMXAA.

### Example 3: STING-activitv dependent promoter

As DMXAA-induced STING-activity caused enhanced cytokine expression levels in cells upon transduction with either hSTINGmut or mSTINGwt, the objective of the following experiments was to develop a system where a target gene could be expressed dependent on STING-activity in the transduced cell. Therefore, the aim of the following 4 experiments was to construct vectors with a STING-activity responsive promoter.

### Experiment 1: IFN-β promoter

One of the main cytokines upregulated upon DMXAA-mediated STING-activation is IFN-β, making the IFN-β promoter a feasible target to examine its function as a STING-activity dependent promoter. Therefore, the objective of this experiment was to examine the specificity of different modified IFN-β promoters in their response to DMXAA-induced STING activity.

### Materials and methods

To this end, five different modifications of the IFN-β promoter (short IFN-β A according to SEQ ID NO: 18, short IFN-β B according to SEQ ID NO: 19, short IFN-β C according to SEQ ID NO: 20, short IFN-β D according to SEQ ID NO: 21, short IFN-β E according to SEQ ID NO: 22) were designed. Lentiviral vectors encoding luciferase under the control of these five different promoters were constructed. These vectors were used to lentivirally transduce MC38 cell lines stably expressing hSTINGmut as described above (example 1) while a control group did not receive further lentivirus transduction (none). 24 h after seeding, the cells were treated with DMXAA (50 µg/mL) or vehicle. After another 3h (Fig 3A) or 3 and 6 h (Figures 3B, 3C, 3D) or 6h (Figures 3E, 3F, 3G and 3H), cells were homogenized in 50 µL of Passive Lysis Buffer (Promega) and frozen in dry ice. After 3 cycles of thaw-vortex-freeze to ensure lysis, cells were spun at 12000 g for 2 min at 4 °C. 20 µL were added to a luminometer 96-well plate and luciferase activity was determined using the respective substrates D-Luciferin and Coelenterazine.

### Results and discussion

As shown in Fig. 3 A, each of the aforementioned promoters showed luciferase activity upon DMXAA administration, whereas cells that were transduced with only hSTINGmut (that is, cells lacking vectors encoding luciferase, indicated as "none" in Fig 3A) showed no luciferase activity irrespective of DMXAA administration. Notably, the short IFN-β. A promoter showed the highest luciferase activity upon DMXAA treatment compared to the other promoters. However, significant luciferase activity was observed even in the absence of DMXAA. This shows that although the short IFN-β A promoter correlates with the activation of hSTINGmut the specificity of this promoter is somewhat low as basal luciferase activity is observed even when hSTINGmut is not activated by DMXAA (leaky expression). Similarly, also the other promoters showed significant leaky expression. Moreover, the overall responsiveness of the other promoters towards DMXAA-activated STING was rather low.

### Experiment 2: Chemokine promoter

Next to IFN-β, also the chemokines CXCL9 and CXCL10 showed a distinct upregulation upon DMXAA-mediated STING activation, making the CXCL9- and CXCL10-promoters feasible targets to examine their function as STING-activity dependent promoters. Therefore, the objective of this experiment was to examine the specificity of these promoters in their response to DMXAA-induced STING activity.

### Materials and methods

The CXCL9-promoter (CXCL9 according to SEQ ID NO: 23) and the CXCL10-promoter (CXCL10 according to SEQ ID NO: 24) were used for the following experiment. To this end, lentiviral vectors encoding luciferase under the control of these two different promoters were constructed. These vectors were used to lentivirally transduce MC38 cell lines stably expressing hSTINGmut as described above (example 1) while a control group did not receive further lentivirus transduction (control). In addition, the lentiviral vectors encoding the short IFN-β A promoter, the short IFN-β B promoter, the short IFN-β C promoter described in experiment 1 were used as a comparison. 24h after seeding, the cells were treated with DMXAA (50 µg/mL) or vehicle. After another 3 or 6 h, cells were harvested and luciferase activity measured as described in experiment 1.

### Results and discussion

As shown in Fig. 3 B, each of the aforementioned promoters showed luciferase activity upon DMXAA administration, whereas cells that were transduced with hSTINGmut alone (Control) showed no luciferase activity irrespective of DMXAA administration. Notably, the CXCL9-promoter and the CXCL10-promoter showed significantly higher luciferase activities compared to the IFN-β promoters. However, significant luciferase activity was observed for both chemokine promoters even in the absence of DMXAA. This shows that although these promoters cause a high luciferase expression upon DMXAA administration the specificity of these promoters is somewhat low as basal luciferase activity is observed even when hSTINGmut is not activated by DMXAA (leaky expression).

### Experiment 3: Functionality of STING-activity dependent promoters

Experiments 1 and 2 prove that the IFN-β, the CXCL9- and the CXCL10-promoter, when operably linked to a luciferase transgene, cause its upregulation upon DMXAA-induced STING activation. The objective of the following experiment was to further prove the functionality of these promoters by operably linking them to the expression of the immunomodulatory single chain interleukin-12 (scIL-12).

### Materials and methods

The CXCL9-promoter and the CXCL10-promoter from experiment 2, and a full length IFN-β-promoter (IFN-β according to SEQ ID NO: 25) were used for the following experiment. To this end, lentiviral vectors encoding murine scIL-12 under the control of these three different promoters were constructed. These vectors were used to lentivirally transduce MC38 cell lines stably expressing hSTINGmut as described above (example 1) while a control group did not receive further lentivirus transduction (no vector). 24 h after seeding, the cells were treated with DMXAA (50 µg/mL) or vehicle. After the indicated time points, cells were harvested. The cell lysate was used to measure scIL-12 mRNA expression by qPCR as described before (example 1, experiment 1), whereas the supernatant was used to measure secreted scIL-12 protein in NUNC MaxiSorp flat-bottom 96-well plates using an ELISA assay kit (OptEIA, Mouse IL-12 (p70), BD Biosciences, San Diego, CA USA), following manufacturer's instructions.

### Results and discussion

As shown in Fig. 3 C - D, cells that were transduced with hSTINGmut alone showed low expression of scIL-12 mRNA in the cell and did not secrete scIL-12 in detectable levels, irrespective of DMXAA administration. The functionality of the IFN-β-promoter and CXCL9-promoter in terms of scIL-12 mRNA or protein expression was poor even upon DMXAA treatment. In contrast, scIL-12 expression was highly upregulated 6 h after DMXAA treatment and control of the CXCL10-promoter, and significant amounts of scIL-12 protein were secreted into the supernatant. However, in accordance with the previous experiments, the CXCL10-promoter showed significant leakage in that scIL-12 was also produced in the absence of DMXAA. While this shows that the general concept of STING-activity dependent promoters is feasible, the considerable leakage of the promoters used in these experiments deems it necessary to examine other promoters with high inducibility upon DMXAA administration together with high specificity towards STING activity.

### Experiment 4: Synthetic promoters

As described above, the specificity of the promoters used in the previous experiments was somewhat poor. Therefore, the aim of the following experiment was to find other promoters that combine high inducibility upon DMXAA administration together with high specificity towards STING activity. In order to achieve this, synthetic promoters have been generated.

### Materials and methods

Seven different promoters were generated, namely short STATx4 (4 STAT motifs separated by 6 nucleotides according to SEQ ID NO: 26), IRSF3x4 (4 IRSF3 motifs separated by 20 nucleotides according to SEQ ID NO: 27), short IRSF3x4 (4 IRSF3 motifs separated by 6 nucleotides according to SEQ ID NO: 28), ISREx4 (4 ISRE motifs separated by 20 nucleotides according to SEQ ID NO: 12), short ISREx4 (4 ISRE motifs separated by 6 nucleotides according to SEQ ID NO: 13), NFκBx4 (4 NFκB motifs separated by 20 nucleotides according to SEQ ID NO: 29) and short NFκBx4 (4 NFκB motifs separated by 6 nucleotides according to SEQ ID NO: 30). pGL3-basic plasmids containing the luciferase sequence under the control of the respective promoters were designed. In addition, a plasmid containing the luciferase sequence under the control of the murine IFN-β-promoter (mIFN-β) and a plasmid containing the luciferase sequence under the control of CXCL10-promoter were used as controls. Either MC38 cells or human HEK293T cells, both which stably expressed hSTINGmut as described above (example 1), were transfected with the indicated plasmids using Lipofectamine3000 according to the manufacturers protocol, whereas a negative control was mock-transfected (Basic). 24h after transfection, the cells were treated with DMXAA (50 µg/mL) or vehicle, and luciferase activity was measured after another 48 h as described in experiment 1.

### Results and discussion

As shown in Fig. 3 E - F, both ISRE-promoters as well as both NFκB-promoters caused increased luciferase activity in MC38 cells upon DMXAA-mediated STING activation. However, the NFκB-promoters also displayed considerable luciferase activity in the absence of DMXAA treatment. In contrast, the ISRE-promoters showed high inducibility upon DMXAA stimulation with low leakage. The short STAT-promoter and both IRSF-promoters showed poor inducibility. The validity of these assays are corroborated by the fact that no luciferase activity was observed in mock-transfected controls (Basic) irrespective of DMXAA treatment.

As shown in Fig. 3 G - H, both ISRE-promoters as well as both NFκB-promoters caused increased luciferase activity in HEK293T cells upon DMXAA-mediated STING activation. Moreover, compared to murine MC38 cells the leakage of either promoter was considerably reduced in HEK293T cells. However, while no detectable leakage could be observed for the ISRE-promoters, the NFκB-promoters still displayed some leakage in the absence of DMXAA treatment. The highest inducibility was observed for the CXCL10-promoter which, however, displayed considerable leakage. The other promoters showed a rather low inducibility but also limited leakage. Therefore, the ISREx4-promoter showed the highest inducibility while simultaneously displaying undetectable leakage.

Taken together, it should be noted that the ISREx4-promoter exhibits leakage in MC38 cells but not in 293T cells. The former is a tumor cell line while the latter is a non-malignant cell line of embryonic origin. Tumor cell lines frequently have chromosomal abnormalities and micronuclei in the cytoplasm which may sustain smoldering STING activation leading to low-level IFN-β production and low-grade activation of STING-responsive promoters.

### Example 4: STING activity-dependent IL-12 expression in vivo

The ISREx4-promoter showed the highest inducibility while maintaining very low leakage, thereby making it the most promising promoter for *in vivo* applications. Hence, the aim of the following experiments was to analyze the translatability of STING-activity mediated IL-12 expression *in vivo.*

### Experiment 1: IL-12 production in the absence of DMXAA-mediated STING-induction in vivo

The objective of the first experiment was to examine whether IL-12 is produced in transduced cells *in vivo* even in the absence of DMXAA-mediated STING activation.

### Materials and methods

An adeno-associated virus (AAV) of the serotype 8 (AAV8) was constructed that encodes scIL-12 under the control of the ISREx4-promoter (AAV8-ISREx4-IL12) as well as an AAV8 control vector encoding scIL-12 under the control of the murine IFN-β-promoter (AAV8-mIFN-β-IL12). To this end, three different doses, namely 1×10¹⁰ viral genomes, 5×10¹⁰ viral genomes and 1×10¹¹ viral genomes, of AAV8-ISREx4-IL12 or AAV8-pIFN-β-IL12 were injected intravenously in different groups of C57 wild type mice, aged 6 to 10 weeks (n=3 per group), and IL-12 serum levels were measured 7 days thereafter as described in example 2.

### Results and discussion

As shown in Fig. 4 A, the IL-12 expression was dose-dependent as higher viral doses caused increased IL-12 production for both AAVs. Notably, the expression was distinctly higher upon intravenous administration of AAV8-ISREx4-IL12 compared to AAV8-pIFN-β-IL12. Hence, although expression levels were higher for AAV8-ISREx4-IL12, both AAVs caused the production of IL-12 *in vivo* even when no DMXAA was administered. Therefore, measures must be taken to reduce leaky IL-12 production *in vivo.*

### Experiment 2: Restraint of IL-12 leakage by micro RNA binding sites in vivo

As shown in the experiment above, upon intravenous administration of AAV8-ISREx4-IL12, IL-12 is produced irrespective of DMXAA-presence which may cause adverse side effects. Many AAV serotypes, including AAV8, target mainly the liver when present in the blood circulation. Even in the event of intratumor injections, some AAV might enter the blood circulation as tumors are typically highly vascularized, thereby transducing hepatocytes causing them to express IL-12 under the control of an ISRE-promoter. The liver is exposed to multiple pathogen-derived molecules of intestinal origin, which are capable of inducing IFN type I production, thereby causing the production of IL-12. Since IL-12 is a potent immune-stimulatory but cytotoxic cytokine it is critical to limit IL-12 expression to the targeted tissue and to preclude IL-12 production in the liver. One way to limit expression of a certain protein is to intervene on a post-transcriptional level, for example by miRNA binding and subsequent mRNA degradation. Thus, to control IL-12 expression a STING-responsive promoter with little leakage was selected and in an attempt to restrain hepatic IL-12 production the IL-12 sequence was operably linked to binding sites for the micro RNA 122 (miR122) which is highly abundant in liver cells.

### Materials and methods

A nucleotide sequence was constructed where 5 repetitions of the target sequence of miR122 was placed at the 3'end of the IL-12 sequence. An AAV vector comprising this construct under the control of the ISREx4-promoter was constructed and the respective viral particles produced (AAV8-ISREx4-IL12miR122T). Two groups of C57 wild type mice, aged 6 to 10 weeks (n=4 each), were injected intravenously with either AAV8-ISREx4-IL12 or AAV8-ISREx4-IL12miR122T (each 0.5×10¹¹ viral genomes) and IL-12 serum levels were measured at day 7 after vector injection as described above.

### Results and discussion

As shown in Figure 4 B, the animals treated with the vector containing the miR122 binding sites (AAV8-ISREx4-IL12miR122T) had undetectable serum levels of IL-12, while values of more than10 ng/ml were found in those mice which received AAV8-ISREx4-IL12. Thus, undesired basal liver production of IL-12 can be avoided by controlling its expression via miR122 binding sites.

### Experiment 3: Restraint of IL-12 leakage by micro RNA binding sites in the presence of DMXAA in vivo

Since undesired basal liver production of IL-12 can be avoided by controlling its expression via miR122 binding sites, the objective of the following experiment was to elucidate whether undesired liver production of IL-12 can be achieved even in the presence of the STING agonist DMXAA.

### Materials and methods

To this end, C57 wild type mice, aged 6 to 10 weeks , were injected intravenously with an AAV8 vector encoding hSTINGmut under the control of a constitutive CAG promoter (AAV8-CAG-hSTINGmut, 1×10¹¹ viral genomes) plus the aforementioned AAV8 vector containing the ISRE-promoter and the single chain IL-12 with (AAV8-ISREx4-IL12miR122T, 0.5×10¹¹ viral genomes) or without 5 miR122 binding sites at the 3'end (AAV8-ISREx4-IL12, 0.5×10¹¹ viral genomes) (n= 4 animals each group). One week later these animals were challenged intravenously with DMXAA (20 mg DMXAA/kg bodyweight), a vehicle or not at all (basal). 4 h thereafter 150 µL blood was collected, and IL-12 serum levels quantified by ELISA as described above.

### Results and discussion

As shown in Figure 4 C, while mice injected with the IL-12 vector lacking miR122 binding sites (AAV8-ISREx4-IL12) showed increased basal serum levels of IL-12 which further increased upon DMXAA administration, those animals injected with the IL-12 vector containing miR122 binding sites (AAV8-ISREx4-IL12miR122T) exhibited almost undetectable IL-12 serum levels in basal situation and extremely low levels after DMXAA challenge. Hence, AAV8-ISREx4-IL12miR122T shows almost no leakage even in the presence of DMXAA.

### Example 5: Therapeutic efficacy upon intratumor co-injection of AAV8-hSTINGmut plus AAV8-ISREx4-IL12miR122T followed by repeated DMXAA administration

The objective of the following experiment was to analyze whether co-injecting AAV8-hSTINGmut plus AAV8-ISREx4-IL12miR122T into the tumor followed by repeated DMXAA administration shows a therapeutic effect as measured by tumor size reduction.

### Materials and methods

To this end, a murine model of colon cancer generated by subcutaneous injection of MC38 cells in syngeneic STING deficient C57 mice (C57BL/6J-Tmem173gt/J, STING KO) was used. In these animals DMXAA will not activate STING systemically but only in those tissues that have been transduced with the vector encoding hSTINGmut thus mimicking the human situation. In this experiment MC38 subcutaneous tumors were generated in STING KO mice by subcutaneous injection of 500,000 MC38 cells. When the tumor nodules reached a size of about 70 mm³ they were injected with AAV8-hSTINGmut (1×10¹¹ viral genomes) plus AAV8-ISREx4-IL12miR122T (0.5×10¹¹ viral genomes) and 48 h later the animals were treated with DMXAA (20 mg/kg bodyweight) (n=6 animals) or vehicle two times per week (n=5 animals). In another mouse control group, the tumors were co-injected with AAV8-hSTINGmut (1×10¹¹ viral genomes) plus AAV8-GFP (0.5×10¹¹ viral genomes) and then received vehicle two times per week (n=3 animals).

### Results and discussion

As shown in Figure 5, a clear antitumor effect was observed in the two groups treated with AAV8-hSTINGmut plus AAV8-ISREx4-IL12miR122T, whereas the antitumor effect was significantly higher in mice treated additionally with DMXAA. Notably, complete tumor elimination was observed in 1 out 6 mice treated with DMXAA but in none given vehicle. In contrast, the tumor volume of mice that received AAV8-GFP plus vehicle treatment continued to grow throughout the time course of the experiment. The antitumor effect in mice treated with AAV8-hSTINGmut plus AAV8-ISREx4-IL12miR122T given vehicle is attributable to low-level intra-tumor production of IL-12 resulting from low-grade basal activation of the ISREx4-promoter in MC38 cells, as mentioned above. Importantly, therapy was well tolerated without symptoms of toxicity in any animal. The strategy here described was designed to exert therapeutic effects without toxicity. In order to avoid toxicity, the AAV vector expressing IL-12 was constructed in such a way that IL-12 will be synthesized within the tumor mass but not in the liver, thus eliminating the risk of sustained basal hepatic production of IL-12 by those AAV particles that gain access to blood and liver after its intra-tumor injection. On the other hand, an agonist is employed, that interacts with the transgenic STING and not with the endogenous human STING. In this way, DMXAA given by intravenous or oral route will activate STING in the tumor but not systemically making the therapy tolerable. The marginal activation of transgenic STING expressed by the small fraction of AAV vector which could reach the liver after intra-tumor injection causes only a slight elevation of type I IFN and IL-12 under 0.2 pg/mL in serum (data not shown) which is very well tolerated by the experimental animals.

### Example 6: Intra-tumor transduction efficiency upon radiation therapy

It is known that different DNA damaging agents including topoisomerase inhibitors and radiation have been shown to enhance AAV transduction likely due to the induction of DNA repair synthesis (Russell DW, et al. PNAS 1995; 92, 5719-5723; Alexander IE et al J Virol 1994; 68, 8282-8287). AAVs are single-stranded DNA vectors which need to be converted into double-stranded DNA to form transcription templates. The DNA damage response elicited by radiotherapy helps to convert single stranded DNA into double stranded DNA (Peng D. et al. J Hepatol. 2000 Jun;32(6):975-85). The use of radiotherapy to enhance tumor transduction by AAV vectors is an attractive option as radiation is frequently given to treat solid tumors. Published data relating AAV transduction and radiation refer to *in vitro* studies with different radiation doses and to *in vivo* studies with high radiation doses. However high radiation doses cause vessel damage and marked alteration of tissue architecture DNA. The objective of the following experiment was to examine whether also low dose radiation enables enhances intra-tumor transduction efficiency. A lower radiation dose preserves tumor vessels, thus, enabling lymphocyte trafficking to the tumor.

### Materials and methods

MC38 subcutaneous tumors were generated in C57 mice, aged 6 to 10 weeks by subcutaneous injection of 500,000 MC38 cells. When the tumor nodules reached a size of about 70 mm³ the tumor was injected with an AAV8 encoding GFP under a constitutively active CAG promoter (AAV8-GFP, 1×10¹¹ viral genomes). In a group of mice (N=3) the tumors received focal moderate/low-dose radiation (8 Gy) using a SARRP (Small Animal Radiation Research Platform, Xstrahl Inc. Suwanee, GA, USA) immediately before intratumoral AAV-injection whereas in the control group (N=6) the tumors were not irradiated. Two days after injection, immunohistochemical sections of the tumor were prepared. Sections were incubated overnight at 4 °C with anti-GFP (1:2000, Abcam, ab6556). After rinsing in TBS-T, the sections were incubated with goat anti-rabbit labeled polymer EnVisionTM+ System (Dako, K400311-2; Glostrup, Denmark) for 30 min at room temperature and peroxidase activity was revealed using DAB+ (Dako, K346811-2). Finally, sections were lightly counterstained with Harris hematoxylin, dehydrated, and coverslipped with Eukitt (Labolan, 28500). Sections were examined by a pathologist to obtain a subjective appraisal of tissue transduction and also they were scanned using a CS2 Aperio scanner and the percentage of pixels with intense GFP staining was quantified with Aperio software.

### Results and discussion

As shown in Figure 6 A tumors that did not receive any radiation prior to intra-tumoral AAV injection showed transduction efficiencies of about 10-15%. However, tumors that received 8 Gy radiotherapy directly before AAV injection showed transduction efficiencies of about 25-40 % of the tumor area. As shown in Figure 6 B, when tumor sections were analyzed using the Aperio software, a statistically significant increase in the amount of intense GFP-signals in relation to the total area in tumors that received 8 Gy was observed as compared with non-irradiated tumors.

These data show that tumor pre-treatment with a low dose of radiation resulted in marked enhancement of tumor transduction by AAV vectors

### Example 7: Radiotherapy induced STING activation in vitro

It has been shown that radiation increases IFN-β production by tumor cells and that this effect is mediated by STING. The objective of this experiment was to investigate whether IFN-β production is furthermore increased by exogenic STING.

### Materials and methods

To this end, lentiviral vectors encoding hSTINGmut were used to generate MC38 cells stably expressing hSTINGmut as described above (example 1) while in a control group the cells had not been transduced. Cells were trypsinized, counted and diluted to 500.000 cells/mL in complete growth media. Cell suspension was divided in equal volumes that were non-irradiated or irradiated in a Gammacell Elan 3000 (Nordion, Canada) for 90 s (8 Gy) immediately before seeding at a density of 750 cells/mm². At indicated time points after radiation, the supernatant was collected, and IFN-β protein levels quantified by ELISA as described above.

### Results and discussion

As shown in Figure 7 IFN-β production remained at low levels throughout the time course of the experiment for cells that neither received radiation nor expressed exogenic hSTINGmut. The same was true for cells that expressed exogenic hSTINGmut but did not receive radiation. However, radiation alone caused an upregulation in IFN-β expression beginning at 24 h after radiation which was furthermore elevated when hSTINGmut was exogenically expressed. Therefore, the effect of radiation on IFN-β production by tumor cells is enhanced by increasing STING cell abundance.

### Example 8: Local irradiation followed by intratumor injection of vectors encoding the expression system induces tumor regression and abscopal effects

It has been shown that radiotherapy induces STING-mediated IFN-β production in tumor cells (Deng L. et al Immunity, 2014; 41, 843-852). The previous example shows that enrichment in STING increases the production of IFN-β by irradiated cells. By connecting IFN-β expression with IL-12 production, it was analyzed in the following experiments whether a robust anti-tumor effect could be achieved by radiating and transducing the tumor with the expression system. A

### Experiment 1: Anti-tumor activity of combined treatment without DMXAA

The objective of the first experiment was to investigate whether the synergistic effects observed *in vitro* are also applicable to the *in vivo* situation and whether the combined treatment of radiotherapy plus immuno-gene-therapy has an anti-tumor effect. Therefore, MC38 tumors were implanted in wild-type mice that were then treated with radiotherapy alone, a combination of immune-gene-therapy and radiotherapy or as a control no treatment at all.

### Material and methods

Group 1 (n = 4) had MC38 tumors that were neither irradiated nor transduced with any vector; group 2 (n = 5) were mice in which the tumor was irradiated with 8 Gy as described above and that immediately afterwards received an intra-tumor injection with AAV8-hSTINGmut (1.0×10¹¹ viral genomes) plus AAV8-ISREx4-IL12miR122T (0.5×10¹¹ viral genomes), and group 3 (n = 4) corresponded to mice with MC38 tumors that were irradiated with 8 Gy as described before and then immediately afterwards received an intra-tumor injection with AAV8-GFP (1.5×10¹¹ viral genomes). Tumor sizes were measured twice weekly using an electronic caliper and the volume was calculated ((length × width²)/2). In addition, in a second part of the experiment blood was collected 17 d after transduction and IL-12 serum levels were quantified by ELISA as described above (example 4). Mice were euthanized when tumors reached 17 mm on the longest axis.

### Results and discussion

As shown in Figure 8 A, in the control groups (group 1 and group 3) the tumor continued to grow throughout the time course of the experiment. However, due to the radiation treatment tumor growth in group 3 progressed in a slower rhythm than in group 1. In contrast, animals from group 2 eliminated the tumor very efficiently. As shown in Figure 8 B, serum IL-12 remained undetectable at the time of tumor elimination in 4 of the 5 mice of group 2. Of note, the IL-12 serum level in this one mouse was distinctly lower than in the previous experiment where no miRNA122 target site was used (example 4, experiment 1). Serum levels in group 1 and 3 were undetectable in all mice. These findings point to the concept that radiotherapy-induced DNA damage activates STING which in turn activates the promoter driving IL-12 expression resulting in increased intra-tumor levels of IFN type I and IL-12 leading to tumor rejection. Furthermore, IL-12 was contained in the tumor thereby preventing unwanted toxicity. Taken together, a synergistic effect is also observed *in vivo* by combining radiotherapy with the immunogene therapy according to the invention.

### Experiment 2: Abscopal effect of combined treatment without DMXAA

As described above, combined therapy comprising radiotherapy and immune-gene-therapy caused tumor- elimination in all mice. Not wishing to be bound by therapy, it is thought that immunomodulatory molecules such as IL-12 stimulate the immune system to target the tumor cells which normally manage to evade the immune system (tumor escape). Therefore, this treatment even when it is applied intratumorally could stimulate the immune system to also target tumors outside of the injection site. This is known as the abscopal effect where local treatments also affects untreated tumors (e.g. metastases).

### Materials and methods

In this study, MC38 tumors were implanted on the right flank by subcutaneous injection of the tumor cells in C57BL/6 wild-type mice, aged 6 to 10 weeks, as described above (example 6). In addition, 2×10⁵ MC38 tumor cells were inoculated in the left flank concurrently. When tumors on the right flank reached a volume of 70-100 mm³, mice were randomized into different treatment groups (n = 6 mice per group). All groups received radiation of the right flank with 8 Gy and subsequent intratumor injection of the respective AAVs 11 d after tumor inoculation as described above whereas the tumors in the left flank remained untreated. Group 1 received intratumor injection of AAV8-GFP (1.5×10¹¹ viral genomes), group 2 received intratumor injection of AAV8-hSTINGmut (1.0×10¹¹ viral genomes) plus AAV8-GFP (0.5×10¹¹ viral genomes), group 3 received intratumor injection of AAV8-hSTINGmut (1.0×10¹¹ viral genomes) plus AAV8-ISREx4-IL12miR122T (0.5×10¹¹ viral genomes), and group 4 received intratumor injection of AAV8-GFP (1.0×10¹¹ viral genomes) plus AAV8-ISREx4-IL12miR122T (0.5×10¹¹ viral genomes). Tumor sizes were measured twice weekly by using an electronic caliper and the volume was calculated ((length × width²)/2). Mice were euthanized when tumors reached 17 mm on the longest axis.

### Results and discussion

As shown in Figure 8 C, the tumor progression of the treated tumors (right flank) for groups 1 and 3 were similar to that of the previous experiment. Notably, intratumor injection with AAV8-hSTINGmut but without AAV8-ISREx4-IL12miR122T (group 2) did not affect tumor progression significantly compared to the AAV8-GFP control group. However, intratumor injection with AAV8-ISREx4-IL12miR122T but without AAV8-hSTINGmut (group 4) caused the elimination of the tumor in a similar course as the combined treatment (group 3). Of note, the course of tumor progression of the untreated tumors (respective left flank) was similar, although less pronounced, than as for the treated tumors as shown in Figure 8 D. This study confirms a strong antitumor effect of the therapy on the treated tumor leading to complete tumor rejection in those animals given low-dose radiotherapy together with intratumor injection of AAV8-hSTINGmut plus AAV8-ISREx4-IL12miR122T. And, in addition, it shows that the combined therapy is also accompanied by a significant abscopal effect.

### Example 9: Synergism between canonical STING pathway activated by DMXAA and non-canonical STING pathway activated by radiotherapy in vitro

As indicated before, it is known that radiotherapy-induced DNA damage activates STING. This activation, however, is limited due to low expression of cGAS/STING in many tumors (Dunphy et al., 2018, Molecular Cell 71, 745-760). Recently, it was shown that DNA-damaging agents activate STING by a non-canonical cGAMP-independent route favoring NFκB pathway rather than IFN type I production. Since DMXAA is a canonical activator of STING, the objective of the following experiments was to investigate whether there is a synergism between canonical and non-canonical pathways. It should be mentioned that at the high MOI (10⁵) used in the following experiments there are no significant differences in the cell transduction rate between irradiated and non-irradiated cells and hence strong differences in the effect of DMXAA on irradiated and non-irradiated cells in terms of cytokine induction could not be ascribed to differences in cell transduction rate.

### Experiment 1: Synergism proof of principle I

The objective of the first experiment was to proof that there is a general synergism between the canonical and non-canonical STING pathways. Therefore, STING was activated in MC38 cells either by radiotherapy (non-canonical) and/or by DMXAA (canonical).

### Materials and methods

To this end, MC38 cells received 8 Gy or 0 Gy as described above (example 7). Cells were seeded thereafter at 750 cells/mm² density and were then either mock-transduced or transduced with AAV8-hSTINGmut (1×10⁵ viral genomes/cell). For cell transduction 8.5×10⁹ viral genomes of AAV8-hSTINGmut from known concentration stock solutions, were resuspended in 50 µl RPMI 2% FBS. The resuspended viral particles were then added to the wells where the cells were seeded. 48 h later, cells were treated with vehicle or DMXAA (50 µg/mL) and the cells were collected after additional 6 h. The IFN-β mRNA was determined by qPCR as described above (example 1) and expression levels were quantified in reference to non-transduced, non-irradiated cells treated with vehicle.

### Results and discussion

As shown in Figure 9 A, irradiation combined with DMXAA treatment already caused an increase in IFN-β expression even without exogenic hSTINGmut expression. This is most likely because murine MC38 cells also express DMXAA-sensitive mSTINGwt. However, upon exogenic expression of hSTINGmut IFN-β expression is further increased showing synergistic effects of canonical and non-canonical STING pathways. Notably, the other groups only showed minor IFN-β expression except hSTINGmut-transduced MC38 cells that received DMXAA but no irradiation which showed an intermediate expression.

### Experiment 2: Synergism proof of principle II

In addition to experiment 1, the objective of the second experiment was to proof that the observed synergism between the canonical and non-canonical STING pathways also causes elevated IL-12 levels when MC38 cells are additionally transduced with AAV8-ISREx4-IL12miR122T. Therefore, MC38 cells were additionally transduced with AAV8-ISREx4-IL12miR122T, and STING was activated in MC38 cells either by radiotherapy (non-canonical) and/or by DMXAA (canonical).

### Materials and methods

To this end, MC38 cells received 8 Gy or 0 Gy as described above and then were co-transduced with AAV8-hSTINGmut (1×10⁵ viral genomes/cell) and AAV8-ISREx4-IL12miR122T (0.5×10⁵ viral genomes/cell) as described above. For cell transduction, 8.5×10⁹ viral genomes of AAV8-hSTINGmut and 4.25×10⁹ viral genomes of AAV8-scIL12-miR122T, from known concentration stock solutions, were resuspended, each one, in 50 µl RPMI 2% FBS. The resuspended viral particles were then added to the wells where the cells were seeded. 48 h later, cells were treated with vehicle or DMXAA and the cells were collected after additional 6 h. The IL-12 mRNA was determined by qPCR as described above (example 3) and expression levels were quantified in reference to non-transduced, non-irradiated cells treated with vehicle.

### Results and discussion

As shown in Figure 9 B, those cells that had been transduced with AAV8-hSTINGmut plus AAV8-ISREx4-IL12miR122T, the administration of DMXAA caused a slight elevation in IL-12 mRNA levels in non-irradiated cells but elicited a strong elevation of this transcript in those cells that had been irradiated with 8 Gy. These findings, in agreement with the previous experiment, confirm that DMXAA and radiotherapy activate STING synergistically.

### Experiment 3: Synergism proof of principle III

In addition to experiments 1 and 2, the objective of the third experiment was to further investigate the observed synergism between the canonical and non-canonical STING. Therefore, cells of human origin (HEK293) cells were transduced with vectors encoding hSTINGmut, ISREx4-IL12-miR122T and/or GFP as a control, and then treated with radiation (non-canonical) and/or DMXAA (canonical).

### Materials and methods

To this end, HEK293 cells received 8 Gy or 0 Gy as described above for MC38 cells and then were co-transduced with AAV8-hSTINGmut (1×10⁵ viral genomes), AAV8-ISREx4-IL12miR122T (1×10⁵ viral genomes) and/or AAV-GFP (1×10⁵ viral genomes) as indicated in the figure. 48 h later, cells were treated with vehicle or DMXAA (50 µg/mL), and the cells were collected 6 h thereafter. The IFN-β and the IL-12 mRNA expression levels were determined by qPCR as described above (example 1) and expression levels were quantified in reference to the indicated controls.

### Results and discussion

As shown in Figure 9 C, IFN-β expression levels were upregulated for hSTINGmut expressing cells receiving DMXAA agonist treatment. However, expression levels were significantly increased upon additional irradiation of these cells. Cells that did not express hSTINGmut or only received vehicle showed low expression levels. As shown in Figure 9 D, IL12 expression levels were upregulated for AAV-ISREx4-IL12miR122T transduced cells irrespective of other treatments, whereas non-transduced controls or cells that only received AAV8-hSTINGmut showed nearly no expression of IL-12. However, additional radiation already increased IL-12 expression in cells expressing ISREx4-IL12miR122T. Moreover, further DMXAA treatment caused a significant increase in IL-12 expression levels. Importantly, compared to murine MC38 cells the response of HEK293 cells was much more specific towards irradiation in that the ISREx4-promoter exhibits leakage in MC38 cells but not in 293T cells. The former is a tumor cell line while the latter is a non-malignant cell line of embryonic origin. Tumor cell lines frequently have chromosomal abnormalities and micronuclei in the cytoplasm which may sustain smoldering STING activation leading to low-level IFN-β production and low-grade activation of STING-responsive promoters. In agreement with the previous experiments, these findings confirm that DMXAA and radiation activate STING synergistically.

### Experiment 4: Synergism proof of principle IV

In addition to the previous experiments, the objective of the third experiment was to further investigate the observed synergism between the canonical and non-canonical STING. Therefore, MC38 cells were treated with different doses of radiation (non-canonical) and different doses of DMXAA (canonical).

### Materials and methods

To this end, native non-transduced MC38 cells received 0 Gy, 2 Gy or 8 Gy as described above. 24 h later, cells were treated with vehicle or different doses of DMXAA (10 µg/mL, 20 µg/mL or 50 µg/mL), and the cells were collected at the indicated time points. The IFN-β protein levels were determined by ELISA as described above. In another part of the experiment MC38 cells received 0 Gy or 8 Gy, received 50 µg/mL DMXAA or vehicle 24 h thereafter and were collected after another 24 h. The supernatants were used to quantify IFN-β levels as described above.

### Results and discussion

As shown in Figure 9 E, IFN-β production at 24 h or 48 h after cell treatment with 8 Gy radiation plus DMXAA stimulation (at different doses) induced IFN-β production at levels exceeding the sum of those obtained with radiation alone and DMXAA alone, indicating a synergistic effect. These findings suggest that radiation activates STING and sensitizes this molecule to STING agonists like DMXAA. As shown in Figure 9 F already a single dose of radiation results in IFN-β production for more than 48h and this effect is enhanced by DMXAA. In agreement with the previous experiments, these findings confirm that DMXAA and radiation activate STING synergistically.

### Example 10: Synergism between canonical STING pathway activated by DMXAA and non-canonical STING pathway activated by radiotherapy in a humanized mouse model in vivo

The previous example showed the synergistic effect achieved by radiotherapy in combination with immunogene therapy according to the invention in wild-type mice. The following experiment investigates whether the synergistic effect is also observable in a humanized mouse model where STING is knocked out. This reflects the human situation where DMXAA administration only causes STING activation in the tumor which has been transduced with the expression system whereas systemic STING activation is prevented.

### Materials and methods.

In this study, MC38 tumors were implanted on the right flank by subcutaneous injection of the tumor cells in syngeneic STING deficient C57 mice (C57BL/6J-Tmem173gt/J, STING KO), aged 6 to 10 weeks, as described above (example 6). Mice were then randomly divided into 5 groups and treatment started when the size of the tumor nodules reached 100 mm³. Tumor nodules received local radiation (8 Gy) followed by an intratumor injection of AAV8-hSTINGmut (1.0×10¹¹ viral genomes) plus AAV8-ISREx4-IL12miR122T (0.5×10¹¹ viral genomes) (G2 and G4), or intratumor injection of AAV8-GFP (1.5×10¹¹ viral genomes) (G1), or AAV8-GFP (1.0×10¹¹ viral genomes) plus AAV8-ISREx4-IL12miR122T (0.5×10¹¹ viral genomes) (G3). As a control tumor nodules received mock-radiation (0 Gy) followed by an intratumor injection of AAV8-hSTINGmut (1.0×10¹¹ viral genomes) plus AAV8-ISREx4-IL12miR122T (0.5×10¹¹ viral genomes) (G5). 48 h thereafter, mice were treated i.p. with DMXAA (20 mg/kg) or vehicle and were sacrificed 3 h later (or 30 h in G4). Serum levels of A) IFN-β B) IL-12 and C) IFN-γ were determined by ELISA as described above. D) In a separated group of mice the tumors were collected at 6h after administration of DMXAA (20mg/kg) or vehicle and then the tumor tissue was homogenized in RIPA buffer and, after sonication and centrifugation, IL-12 was measured by ELISA in supernatant

### Results and discussion

As shown in Figure 10 A - C, while details differ in between experiments a general trend is observable. The groups G1 and G3 showed no significant induction of either cytokine upon DMXAA-administration. In contrast, throughout the experiments the highest cytokine expression was observed in the groups receiving radiotherapy with intratumor injections of AAV8-hSTINGmut plus AAV8-ISREx4-IL12miR122T (G2 and G4). Notably, the expression was highest at an earlier time point (3 h, G2) compared to a later time point (30 h, G4). However, also the mock-radiated group with intratumor injections of AAV8-hSTINGmut plus AAV8-ISREx4-IL12miR122T (G5) showed response to DMXAA treatment although to a lesser degree than G2 which received both vectors plus radiation. In addition, throughout the experiments, administration of vehicle did not induce significant levels of either investigated cytokine corroborating the high specificity of the system. Notably, as shown in Figure 10 D, in the group of mice in which we have determined the intratumor levels of IL-12 we found that the values were low in those treated with vehicle and increased significantly only in those that were irradiated and treated with DMXAA. Thus, also in a mouse model that resembles the human situation in that STING is specifically activated within the tumor, synergistic effects of radiotherapy combined with the immunogene therapy according to the invention are observable. Taken together, as the experiments showed clear evidence of the feasibility of the therapy according to the invention, further investigations have to examine the effect of this therapy for the treatment of cancer in humans.

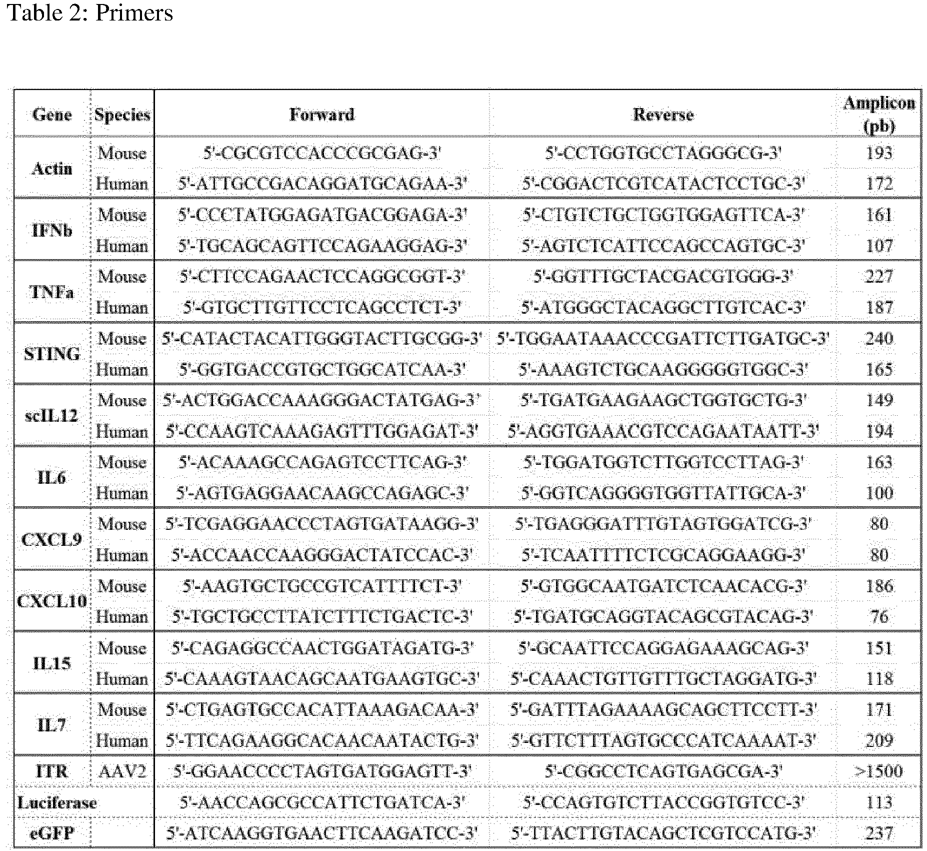

## Claims

1. A recombinant expression system for use in a combined gene and radiation therapy of a disease in a recipient mammal, wherein
a. the recombinant expression system comprises a first nucleic acid construct encoding for a target STING, that is responsive to a STING agonist recognizing said target STING but not the human STING and natural variants thereof, said sequence encoding the target STING is operatively linked to elements allowing expression and/or translation of said target STING; and/or a second nucleic acid construct comprising a therapeutic gene sequence which is operatively linked to a STING responsive promoter; and
b. the recombinant expression system is administered to the recipient mammal after irradiation of said recipient mammal with low dose radiation of the tissue or organ to be transfected or transduced with said recombinant expression system (target tissue or organ); and/or
c. after administration of said recombinant expression system in step b) the target tissue or organ of the recipient mammal is subjected to low dose radiation and/or a STING agonist is administered to the recipient mammal.

2. The recombinant expression system according to claim 1, wherein the disease is selected from the group consisting of cancer, autoimmune disease, inflammatory disease and infectious disease, preferably a solid cancer selected from the group consisting of bladder cancer, bone cancer, brain cancer, breast cancer, cancer of the thymus, cervical cancer, colon cancer, esophageal cancer, gastric cancer, glioblastoma, head & neck cancers, Hodgkin's lymphoma, liver cancer, lung cancer, melanoma, mesothelioma, multiple myeloma, Merkel cell carcinoma, non-Hodgkin's lymphoma, ovarian cancer, pancreatic cancer, prostate cancer, rectal cancer, renal cancer, sarcoma, skin cancer, testicular cancer, thyroid cancer, uterine cancer, plasma cell tumors, neuroendocrine tumors, cholangiocarcinoma, or carcinoid tumors.

3. The recombinant expression system according to claim 1 or 2, wherein the therapeutic gene sequence of the second nucleic acid construct encodes for a protein or gene selected from the group consisting of cytokines such as chemokines, interferons, interleukins, lymphokines, and tumor necrosis factors; hormones; growth factors; cell surface receptors; enzymes; transcription factors; tumor suppressor proteins; nanobodies; monoclonal antibodies; single chain variable fragments (scFv); peptides blocking the activity of cytokines, nuclear factors or hormones; or is a gene such as shRNA against cytokines, nuclear factors or hormones.

4. The recombinant expression system according to claim 3, wherein the protein encoded by the therapeutic gene of second nucleic acid construct is selected from the group consisting of interleukin-1 (IL-1), interleukin-2 (IL-2), interleukin-4 (IL-4), interleukin-5 (IL-5), interleukin-7 (IL-7), interleukin-10 (IL-10), interleukin-12 (IL-12), interleukin-15 (IL-15), interleukin-21 (IL-21), interleukin-23 (IL-23), granulocyte-macrophage colony-stimulating factor (GM-CSF), type I interferons (IFN-α and IFNβ), IFN-γ, TNF-α, FLT3-ligand; immunostimulatory monoclonal antibody or scFV or nanobody neutralizing PD1, PDL1, CTLA4, CD137, TIM3, LAG3; blocking peptides or shRNA against immunosuppressive factors such as TGF-β or IL-10 or FoxP3.

5. The recombinant expression system according to any of the above claims, wherein the STING-responsive promoter second nucleic acid construct is selected from the group consisting of IEN-β-responsive promoter, CXCL9-responsive promoter, CXCL10-responsive promoter, STAT-motif promoter, IRSF3-motif promoter, ISRE-motif promoter, NFκB-motif promoter, STAT6-motif promoter, preferably wherein the STING responsive promoter comprises at least one interferon-stimulated response element (ISRE).

6. The recombinant expression system according to any of the above claims, wherein the STING-responsive promoter of the second nucleic acid construct is selected from the list consisting of:
a. a nucleotide sequence comprising one to four copies of the ISRE as depicted in SEQ ID NO: 11, comprising preferably four copies of said ISRE;
b. a nucleotide sequence according to SEQ ID NO: 12;
c. a nucleotide sequence according to SEQ ID NO: 13.

7. The recombinant expression system according to any of the above claims, wherein the second nucleic acid construct of the expression system comprising a therapeutic gene further comprises at least one sequence motif that inhibits transgenic expression of the therapeutic gene sequence in the liver, wherein the sequence motif is preferably a target sequence of a microRNA which is highly abundant in the liver.

8. The recombinant expression system of claim 7, wherein the microRNA highly abundant in the liver is selected from the group consisting of miR-122, miR-192, miR-199a, miR-101, miR-99a, let7a, let7b, let7c, let7f, preferably wherein the microRNA abundant in the liver is miR-122.

9. The recombinant expression system according to any of the above claims, wherein the low dose radiation is based on X-rays, gamma-rays or particle rays.

10. The recombinant expression system according to any of the above claims, wherein the low dose radiation has a total dose of radiation of 40 Gy or less and preferably of 8 Gy and less.

11. The recombinant expression system according to any of the above claims, wherein the low dose radiation is administered using stereotactic radiosurgery, preferably performed by linear accelerator (LINAC) machines, gamma Knife machines or proton beam therapy.

12. The recombinant expression system according to any of the above claims, wherein the time interval between the low dose irradiation of b. and the subsequent administration of the expression system is between 1 minute and 10 days, preferably between 10 minutes and 2 days and more preferably between 30 minutes and 24 hours.

13. The recombinant expression system according to any of the above claims, wherein the subsequent low dose irradiation of c. given after administration of the recombinant expression system are repeated at intervals between 1 days and 30 days preferably between 5 days and 20 days and more preferably between 7 days and 15 days.

14. The recombinant expression system according to any of the above claims, wherein after administration of the recombinant expression system comprising the first nucleic acid construct, the recipient mammal is administered with a human-non-reacting STING agonist, preferably selected from the group consisting of 5,6-dimethylxanthenone-4-acetic acid (DMXAA), 7-bromo-DMXAA, 7-iodo-DMXAA, 7-hydroxy-DMXAA, 7-formyl-DMXAA, 7-hydroxymethyl-DMXAA, 7-(2-hydroxyethyl)-DMXAA, flavone-8-acetic acid (FAA), 2,7-bis(2-dimethylamino ethoxy)fluoren-9-one (Tilorone) and 10-carboxymethyl-9-acridanone (CMA), and more preferably wherein the STING agonist is DMXAA.

15. A recombinant expression system comprising a nucleic acid construct comprising a therapeutic gene sequence which is operatively linked to a STING responsive promoter, wherein the nucleic acid construct is preferably the second nucleic acid construct according to any of claims 3 to 8.
